# EUROPEAN PATENT APPLICATION

(11) **EP 4 737 571 A1**
(43) Date of publication of application: **06.05.2026**
(21) Application number: 24830988.2
(22) Date of filing: 28.06.2024
(51) Int. Cl.: C12N 15/38, C12N 15/63, A61K 39/25, C07K 14/04, A61P 31/22

(54) **VARICELLA ZOSTER VIRUS (VZV) VACCINE**

(30) Priority: 30.06.2023 WO PCT/CN2023/104921
(71) Applicant: Shenzhen Shenxin Biotechnology Co., Ltd., Shenzhen, Guangdong 518000 (CN)
(72) Inventor: LI, Linxian, Shenzhen, Guangdong 518000 (CN)
(74) Representative: Dentons Patent Solutions Rechtsanwaltsgesellschaft mbH
(86) International application number: PCT/CN2024/102410
(87) International publication number: WO 2025/002359

(57) **Abstract**

The present invention relates to a non-natural nucleic acid, a genetic engineering vector, a host cell, a delivery carrier, a pharmaceutical composition and use thereof, and a varicella zoster virus (VZV) vaccine, wherein the non-natural nucleic acid comprises a polynucleotide encoding a VZV gE protein or a fragment thereof.

## Description

### Technical Field

The present invention belongs to the field of biotechnology, and relates to a varicella-zoster virus (VSV) vaccine.

### Background

Herpes zoster (HZ) is caused by the reactivation of previously infected varicella-zoster virus (VZV). The varicella-zoster virus is highly infectious, and the person infected by the virus is the main infectious source. The ways of infection mainly include direct contact and droplet transmission. The virus would be excreted through the lacrimal gland or saliva two days before the onset of skin rashes in patients. Viruses in blister fluid from varicella-zoster patients are infectious and are infected by contact with skin lesions or inhalation.

The onset of herpes zoster is usually manifested as a skin rash that occurs on one side of the body and is accompanied by pain and itching, which can last two to four weeks, and papulovesicle and blisters may occur sysmatically, accompanied by severe pain. A common complication for herpes zoster is post-herpes zoster neuralgia, which can last months to years and seriously affect the patients' normal work and life. With the increase of age, the immune function of the human body decreases, and the incidence of herpes zoster increases sharply. The global incidence of people over 60-year-old can reach 8-12/1000 per year, while at least half or more of the elderly people over 85-year-old have suffered from herpes zoster at least once.

### Summary

The present disclosure provides a non-natural nucleic acid that can induce a significant immune response in the body.

In addition, the present disclosure further provides a polypeptide or protein encoded by the non-natural nucleic acid, a genetic engineering vector comprising the non-natural nucleic acid, a delivery carrier, a pharmaceutical composition and a vaccine comprising the non-natural nucleic acid, the polypeptide or protein encoded by the non-natural nucleic acid, or the genetic engineering vector, and use thereof.

A non-natural nucleic acid comprising a polynucleotide encoding a VZV gE protein or a fragment thereof.

In some embodiments, the amino acid sequence of the VZV gE protein comprises or is an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 1.

In some embodiments, the VZV gE protein is a full-length gE protein.

In some embodiments, the fragment of the VZVgE protein is one of the follows:
(1) a truncated polypeptide of positions 23 to 623, 31 to 623, 1 to 561, 1 to 573, 1 to 543, 31 to 573 or 31 to 543 of the VZVgE protein; and
(2) a polypeptide having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence of the truncated polypeptide of (1).

In one embodiment, the VZV gE protein or fragment thereof further has a mutation; preferably, the mutation is one or both of mutations Y569A and Y582G.

In one embodiment, the VZV gE protein or fragment thereof comprises or is one of the following polypeptides or proteins:
(1) a polypeptide or protein having an amino acid sequence as set forth in one of SEQ ID NOs: 1-8; and
(2) a polypeptide or protein comprising an amino acid sequence having at least 90%, 91%, 92%, 93%, 94%, 95%, 96%, 97%, 98% or 99% identity to one of the sequences SEQ ID NOs: 1-8.

In some embodiments, the VZV gE protein or fragment thereof is further linked to a heterologous signal peptide and/or a ferritin.

In some embodiments, the heterologous signal peptide comprises one or more of an IgE signal peptide and an IgGκ signal peptide.

In some embodiments, the amino acid sequence of the IgGκ signal peptide is set forth in SEQ ID NO: 9.

In some embodiments, the amino acid sequence of the ferritin is set forth in SEQ ID NO: 10.

In some embodiments, the nucleic acid is RNA.

In some embodiments, the polynucleotide encoding the VZV gE protein or fragment thereof is codon optimized.

In some embodiments, the polynucleotide encoding the VZV gE protein or fragment thereof comprises or is one of the follows:
(1) an RNA corresponding to a polynucleotide having a nucleotide sequence set forth in one of SEQ ID NOs: 11-18; and
(2) an RNA corresponding to a polynucleotide comprising a nucleotide sequence having at least 50%, 55%, 60%, 65%, 70%, 75%, 80%, 85%, 90%, 91%, 92%, 93%, 94%, 95%, 96%, 97%, 98% or 99% identity to the nucleotide sequence set forth in one of SEQ ID NOs: 11-18.

In some embodiments, the nucleic acid is RNA, and the nucleic acid comprises or is one of the following RNAs:
(1) an RNA corresponding to a polynucleotide having a nucleotide sequence set forth in one of SEQ ID NOs: 19-24; and
(2) an RNA corresponding to a polynucleotide comprising a nucleotide sequence having at least 50%, 55%, 60%, 65%, 70%, 75%, 80%, 85%, 90%, 91%, 92%, 93%, 94%, 95%, 96%, 97%, 98% or 99% identity to the nucleotide sequence set forth in one of SEQ ID NOs: 19-24.

In some embodiments, the nucleic acid is mRNA.

In some embodiments, the RNA is mRNA; preferably, the mRNA comprises at least one of a 5' -cap structure, 5'-UTR, 3'-UTR, and a poly(A) tail.

In some embodiments, the DNA sequence corresponding to the 5'-UTR is set forth in SEQ ID NO: 25.

In some embodiments, the DNA sequence corresponding to the 3'-UTR is set forth in SEQ ID NO: 26.

In some embodiments, the nucleotides constituting the poly(A) tail comprise at least 20, at least 40, at least 80, at least 100, or at least 120 A nucleotides. In some embodiments, the nucleotides constituting the poly(A) tail comprise at least 20, at least 40, at least 80, at least 100, or at least 120 continuous A nucleotides. In some embodiments, the nucleotides constituting the poly(A) tail comprise one or more nucleotides other than the A nucleotide. In some embodiments, the DNA sequence corresponding to the poly(A) tail is set forth in SEQ ID NO: 27.

In some embodiments, the nucleic acid is DNA. In some embodiments, the DNA can be transcribed into RNA.

In some embodiments, the polynucleotide encoding the VZV gE protein or fragment thereof is codon optimized.

In some embodiments, the polynucleotide encoding the VZV gE protein or fragment thereof comprises or is one of the follows:
(1) a polynucleotide having a nucleotide sequence set forth in one of SEQ ID NOs: 11-18; and
(2) a polynucleotide comprising a nucleotide sequence having at least 50%, 55%, 60%, 65%, 70%, 75%, 80%, 85%, 90%, 91%, 92%, 93%, 94%, 95%, 96%, 97%, 98% or 99% identity to the nucleotide sequence set forth in one of SEQ ID NOs: 11-18.

In some embodiments, the nucleic acid comprises or is one of the following polynucleotides:
(1) a polynucleotide having a nucleotide sequence set forth in one of SEQ ID NOs: 19-24; and
(2) a polynucleotide comprising a nucleotide sequence having at least 50%, 55%, 60%, 65%, 70%, 75%, 80%, 85%, 90%, 91%, 92%, 93%, 94%, 95%, 96%, 97%, 98% or 99% identity to the nucleotide sequence set forth in one of SEQ ID NOs: 19-24.

In some embodiments, the nucleic acid contains a modified nucleotide.

In some embodiments, the nucleic acid contains a modified nucleoside.

In some embodiments, the modified nucleoside comprises at least one of a modified uridine, a modified cytidine, a modified adenosine, and a modified guanosine.

A genetic engineering vector, comprising the above nucleic acid, or comprising a polynucleotide capable of being transcribed into the above nucleic acid.

A host cell comprising the above genetic engineering vector.

A protein or polypeptide encoded by the above nucleic acid.

A delivery carrier comprising the above nucleic acid, the above genetic engineering vector.

In some embodiments, the delivery carrier is a lipid nanoparticle (LNP), a cationic liposome, a cationic protein, or a lipopolyplex (LPP).

In some embodiments, the delivery carrier is a lipid nanoparticle comprising a cationic lipid comprising the following compound (IV), or a pharmaceutically acceptable salt or stereoisomer thereof: 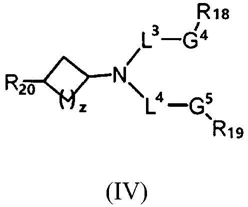 where:
L³ and L⁴ are the same or different, and are each independently C1-C12 alkylene, C2-C12 alkenylene or C2-C12 alkynylene; preferably L³ and L⁴ are the same or different, and are each independently C3-C10 alkylene, C3-C10 alkenylene or C3-C10 alkynylene; further preferably, L³ and L⁴ are the same or different, and are each independently C3-C10 alkylene; most preferably, L³ and L⁴ are the same or different, and are each independently C5-C8 alkylene;
G⁴ and G⁵ are the same or different, and are each independently -O-(C=O)-, -(C=O)-O-, - C(=O)-, -O-, -C(=O)-S- or -S-C(=O)-; preferably, G⁴ and G⁵ are the same or different, and are each independently -O-(C=O)-, -(C=O)-O-, -C(=O)- or -O-; most preferably, G⁴ and G⁵ are the same or different, and are each independently selected from -O-(C=O)- or -(C=O)-O-;
R₁₈ and R are the same or different, and are each independently C5-C27 alkyl or C5-C27 alkenyl containing one or more double bonds; preferably, R₁₈ and R are the same or different, and are each independently C8-C20 alkyl or C8-C20 alkenyl containing one or more double bonds; further preferably, R₁₈ and R are the same or different, and are each independently C9-C17 alkyl or C9-C18 alkenyl containing one or two double bonds; most preferably, R₁₈ and R are the same or different, and are each independently 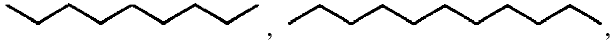 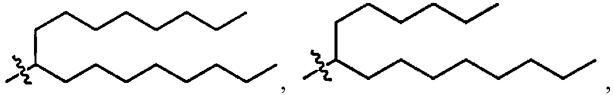 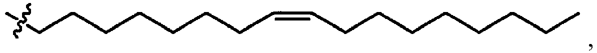 or 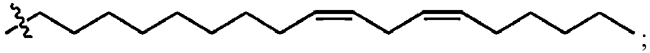
R₂₀ is halogen, hydroxyl, cyano, C1-C6 alkyl, nitro, C1-C6 alkoxy, C1-C6 alkylcarbonyloxy, C1-C6 alkoxycarbonyl, C1-C6 alkylaminocarbonyl or C1-C6 alkylcarbonylamino; preferably, R₂₀ is halogen, hydroxyl, cyano, C1-C6 alkoxy, C1-C6 alkylcarbonyloxy, C1-C6 alkoxycarbonyl, C1-C6 alkylaminocarbonyl or C1-C6 alkylcarbonylamino; further preferably, R₂₀ is halogen, hydroxyl, cyano, C1-C4 alkoxy, C1-C4 alkylcarbonyloxy, C1-C4 alkoxycarbonyl, C1-C4 alkylaminocarbonyl or C1-C4 alkylcarbonylamino; most preferably, R₂₀ is fluorine, hydroxyl, cyano, methoxy, acetoxy, methoxycarbonyl, butylaminocarbonyl or acetamido;
z is 1, 2 or 3.

In some embodiments, the cationic lipid is the following compound (IV-1), or a pharmaceutically acceptable salt or stereoisomer thereof: 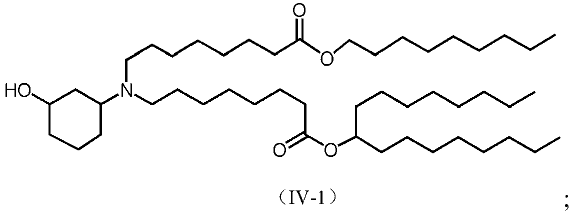 or the cationic lipid is the following compound, or a pharmaceutically acceptable salt thereof: 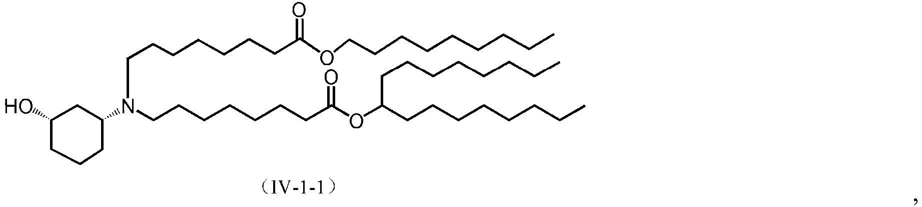 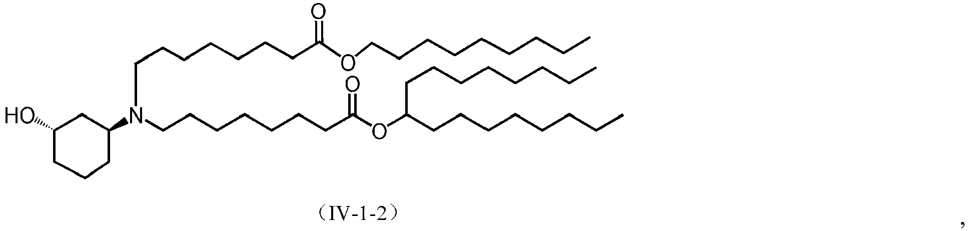 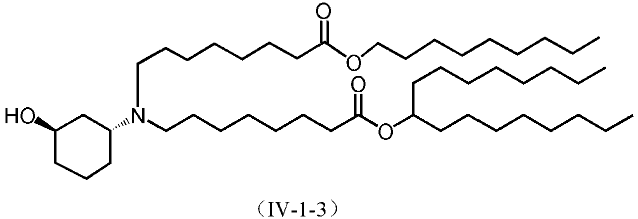 or

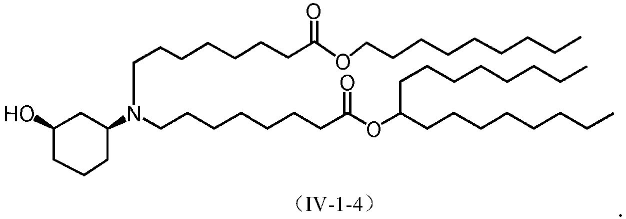

A pharmaceutical composition comprising the above nucleic acid, the above genetic engineering vector, the above host cell, the above protein or polypeptide, or the above delivery carrier, and a pharmaceutically acceptable carrier.

A vaccine, comprising the above nucleic acid, the above genetic engineering vector, the above protein or polypeptide, or the above delivery carrier.

An mRNA vaccine, comprising the above nucleic acid or the above delivery carrier.

In some embodiments, the above delivery carrier is a lipid nanoparticle (LNP).

Use of the above nucleic acid, the above genetic engineering vector, the above host cell, the above protein or polypeptide, or the above delivery carrier or the above pharmaceutical composition in the preparation of a medicament.

In some embodiments, the medicament is used to prevent or treat VZV infection or diseases caused by VZV infection.

In some embodiments, the medicament is used for preventing, treating or ameliorating neuralgia caused by VZV infection.

In some embodiments, the medicament is used for preventing, treating or ameliorating post-herpes zoster neuralgia.

In some embodiments, the medicament is a vaccine; preferably, the medicament is an mRNA vaccine.

In some embodiments, the medicament is used to prevent or treat varicella or herpes zoster.

### Brief Description of Drawings

FIGS. 1A to 1C show the cellular immunoassays on day 6 after single immunization of mice with different mRNA vaccines. FIG. 1A is the Elispot detection of IFN-γ, FIG. 1B is the Elispot detection of IL-4, and FIG. 1C is IFN-γ/IL-4, that is, the comparison of Th1 and Th2 typing for different mRNAs (Th1 bias: IFNγ/IL-4 > 1); 1087 refers to the mRNA vaccine encapsulating the mRNA numbered as 1087, and the same applies to others.
FIGS. 2A-2F are comparisons of the induced cellullar immune levels on day 14 and day 81 after double immunization of mice with mRNA vaccine (1087) and Shingrix. FIG. 2A is the Elispot detection of IFN-γ on day 14, FIG. 2B is the Elispot detection of IL-4 on day 14, FIG. 2C is the comparison of IFN-γ/IL-4 on day 14, that is, Th1 and Th2 typing of different mRNAs (Th1 bias: IFNγ/IL-4 > 1), FIG. 2D is the Elispot detection of IFN-γ on day 81, FIG. 2E is the Elispot detection of IL-4 on day 81, and FIG. 2F is the IFN-γ/IL-4 on day 81.
FIG. 3 is a comparison of the induced IgG antibody titers after immunization of mice with mRNA vaccine (1087) and Shingrix.
FIGS. 4A-4B show IgG antibody titers induced after three immunizations of rats with different doses of mRNA vaccine (1087), among which FIG. 4A shows IgG antibody titers of male rats, and FIG. 4B shows IgG antibody titers of female rats.
FIG. 5 shows the Elispot detection of IFN-γ on day 58 after immunization of rats with different doses of mRNA vaccine (1087).
FIG. 6 is the detection of cellular immune response (ELISpot) of mRNA vaccine in pre-immunized mice.
FIG. 7 is the detection of cellular immuno response (ICS) of mRNA vaccine in pre-immunized mice.
FIG. 8 is the detection of humoral immune response (gE-specific IgG antibody) of mRNA vaccine in serum after pre-immunization of mice.

### Detailed description of the invention

### I. Definition

All patents, patent applications, scientific publications, manufacturers' instructions and guidelines, etc., cited herein, whether supra or infra, are incorporated herein by reference in their entirety. Any content herein should not be understood as an admission that the present disclosure is not entitled to antedate such disclosure.

Unless otherwise indicated, scientific and technical terms used herein have meanings commonly understood by those skilled in the art. Moreover, the terms related to protein and nucleic acid chemistry, molecular biology, cell and tissue culture and microbiology used herein are all terms widely used in the corresponding fields (see, for example, Molecular Cloning: A Laboratory Manual, 2nd Edition, J. Sambrook et al. eds., Cold Spring Harbor Laboratory Press, Cold Spring Harbor 1989). In addition, to understand the present application better, the following definitions and explanations of related terms are provided.

As used herein, the expressions "comprising," "including," "containing," and "having" are openended, meaning including the recited elements, steps, or components but not excluding other element, step, or component that is not recited. The expression "consisting of" excludes any element, step, or component that is not specified. The expression "consisting essentially of" means that the scope is limited to the specified elements, steps, or components, plus optional elements, steps, or components that do not materially affect the basic and novel nature of the claimed subject matter. It should be understood that the expressions "consisting essentially of" and "consisting of" are encompassed within the meaning of the expression "comprising".

As used herein, the singular expressions "a" and "an" and "the" and similar referents used in the context describing the application (especially in the context of the claims) should be understood to cover both the singular and the plural, unless specified in the context otherwise. The term "one or more" or "at least one" encompasses 1, 2, 3, 4, 5, 6, 7, 8, 9 or more. The term "at least one" or "one or more" encompasses 1, 2, 3, 4, 5, 6, 7, 8, 9 or more.

Numerical ranges recited herein should be understood to encompass any and all sub-ranges comprised therein. For example, a range of "1 to 10" should be understood to include not only the explicitly recited values of 1 and 10, but also any single value (e.g., 2, 3, 4, 5, 6, 7, 8 and 9) and sub-range within the range of 1 to 10 (e.g., 1 to 2, 1.5 to 2.5, 1 to 3, 1.5 to 3.5, 2.5 to 4, 3 to 4.5, etc.). This principle also applies to ranges using only one numerical value as the minimum or maximum value.

As used herein, the terms "and/or", "any combination thereof," and grammatical equivalents thereof are used interchangeably. These terms may express, specifically refer to any combination. For example, the following phrases "A, B, and/or C" or "A, B, C, or any combination thereof" may mean "A alone; B alone; C alone; A and B; B and C; A and C; and A, B, and C."

All methods described herein can be performed in any suitable order unless otherwise indicated.

As used herein, the term "naturally occurring" or "naturally occurred" refers to the fact that a substance can be found in nature. For example, peptides, amino acids, proteins, or nucleic acids, which are present in organisms, including viruses, and can be isolated from natural sources and are not artificially modified in experiments, are naturally occurring.

As used herein, the term "non-naturally occurring" as used herein in describing a nucleic acid is intended to mean that the nucleic acid is not found in nature. For example, a non-naturally occurring nucleic acid encoding a viral peptide or protein has at least one genetic change or chemical modification which is generally not found in the wild-type strain of the mentioned virus. Such genetic alterations include, for example, the introduction of expressible nucleic acid sequences encoding peptide fragments or polypeptides heterologous to the mentioned virus, other nucleic acid additions, nucleic acid deletions, nucleic acid substitutions, and/or other functional disruptions to the genetic material of the virus. Chemical modifications include, for example, one or more functional nucleotide analogs as described herein.

As used herein, the term "wild-type" means that the sequence is naturally occurring and not artificially modified, including naturally occurring mutants. The term "fragment" or "fragment of a nucleic acid" in nucleic acids refers to a portion of a nucleic acid. For example, nucleic acids shortened at the 5' and/or 3' ends. A fragment of a nucleic acid comprises at least 50%, 60%, 70%, or 80% from the nucleic acid. Preferably, a fragment of a nucleic acid comprises at least 70% or 80% from the nucleic acid. Nucleotide residues of at least 90%, 95%, 96%, 97%, 98% or 99% are preferred. It can generally be a shorter portion of the full length of the nucleic acid.

The term "variant" in reference to a nucleic acid refers to a nucleic acid variant having at least one nucleotide different from a reference nucleic acid (or "parent"). As compared to the reference nucleic acid, variant nucleic acids include the deletion, addition, mutation, and/or insertion of single or multiple nucleotides, where: deletion includes removing one or more nucleotides from the reference nucleic acid; addition includes fusing one or more nucleotides (e.g., 1, 2, 3, 5, 10, 20, 30, 50 or more nucleotides) to the 5' and/or 3' end of the reference nucleic acid; mutation can include, but is not limited to, substitution (e.g., at least one nucleotide is removed and another nucleotide is inserted at its position (e.g., transversion and conversion)); insertion includes adding at least one nucleotide. As used herein, the term "nucleic acid variant" includes naturally occurring variants and engineered variants. Thus, a "nucleic acid variant" as defined herein can be derived from, isolated from, related to, based on, or homogeneous to a reference nucleic acid sequence. A "nucleic acid variant" may optionally have at least 5%, 10%, 20%, 30%, 40%, 50%, 60%, 70%, 80%, 85%, 86%, 87%, 88%, 89%, 90%, 91%, 92%, 93%, 94%, 95%, 96%, 97%, 98% or 99%; preferably at least 70%, more preferably at least 80%, even more preferably at least 85%, even more preferably at least 90%, most preferably at least 95% or even 97% sequence identity to the corresponding naturally occurring (wild-type) nucleic acid or homolog, fragment or derivative thereof. It may be understood that with respect to nucleic acid molecules, the term "variant" includes degenerate nucleic acid sequences, wherein a degenerate nucleic acid sequence according to the invention is a nucleic acid that differs from a reference nucleic acid in a codon sequence due to the degeneracy of the genetic code.

The term "variant" in reference to a protein refers to a protein variant having at least one amino acid different from a reference protein (or "parent"). As compared to the reference protein, protein variants include the deletion, addition, mutation, and/or insertion of single or multiple amino acids, where: deletion includes removing one or more amino acids from the reference protein; addition includes fusing one or more amino acids (e.g., 1, 2, 3, 5, 10, 20, 30, 50 or more nucleotides) to the N-terminus and/or C-terminus of the reference protein; mutation may includes, but is not limited to, substitution (e.g., at least one amino acid is removed and another amino acid is inserted at its position); insertion includes adding at least one amino acid. Generally, the protein variant has the same or similar activity as the reference protein; alternatively, the protein variant has an altered activity (e.g., increased or decreased) relative to the reference protein. In some embodiments, a "protein variant" may optionally have at least 5%, 10%, 20%, 30%, 40%, 50%, 60%, 70%, 80%, 85%, 86%, 87%, 88%, 89%, 90%, 91%, 92%, 93%, 94%, 95%, 96%, 97%, 98% or 99%; preferably at least 70%, more preferably at least 80%, even more preferably at least 85%, even more preferably at least 90%, most preferably at least 95% or even 97% sequence identity to the corresponding naturally occurring (wild-type) protein or homolog, fragment or derivative thereof.

As used herein with respect to the term "% identical" or "% identity" refers to the percentage of nucleotides or amino acids that are identical in the optimal alignment between the sequences to be compared, the difference between the two sequences may be distributed over a local region (segment) or the entire length of the sequences to be compared. The identity between two sequences is typically determined after the optimal alignment to a segment or "comparison window." Optimal alignment may be performed manually or by means of algorithms known in the art. Algorithms known in the art include, but are not limited to, the local homology algorithms described by Smith and Waterman, 1981, Ads App. Math. 2, 482 and Neddleman and Wunsch, 1970, J. Mol. Biol. 48, 443, the similarity search methods described by Pearson and Lipman, 1988, Proc. Natl Acad. Sci. USA 88, 2444, or using computer programs such as Wisconsin Genetics Software Package, Genetics Computer Group, 575 Science Drive, Madison, GAP in the Wis., BESTFIT, FASTA, BLAST P, BLAST N and TFASTA. For example, the percentage identity of the two sequences may be determined using publicly available algorithms BLASTN or BLASTP on the website of National Center for Biotechnology Information (NCBI).

"% identical" or "% identity" can be obtained by determining the number of identical positions corresponding to the sequences to be compared, dividing this number by the number of compared positions (e.g., the number of positions in the reference sequence), and multiplying this result by 100 to obtain % identity. In some embodiments, a degree of identity is given to a region of at least about 50%, at least about 55%, at least about 60%, at least about 65%, at least about 70%, at least about 75%, at least about 80%, at least about 85%, at least about 90%, at least about 95%, or about 100%. In some embodiments, the degree of identity is given for the entire length of the reference sequence. Alignment for determining sequence identity can be performed with tools known in the art, preferably using optimal sequence alignment, for example, using Align with standard settings, preferably EMBOSS::needle, Matrix: Blosum62, Gap Open 10.0, Gap Extend 0.5.

Preferably, a fragment or variant of a particular nucleic acid, or a nucleic acid having a particular degree of identity to a particular nucleic acid preferably has at least one functional property of the particular nucleic acid, and preferably is functionally equivalent to the particular nucleic acid, e.g., a nucleic acid exhibiting properties that are the same or similar to those of the particular nucleic acid.

As used herein, "nucleotide" includes deoxyribonucleotides, ribonucleotides, deoxyribonucleotide derivatives, and ribonucleotide derivatives. As used herein, "ribonucleotide" is a substance constituting ribonucleic acid (RNA), which consists of a molecule of base, a molecule of pentose and a molecule of phosphate, which refers to a nucleotide having a hydroxyl group at the 2' position of the β-D-ribofuranosyl group, and "deoxyribonucleotide" is a substance constituting deoxyribonucleic acid (DNA), which also consists of a molecule of base, a molecule of pentose and a molecule of phosphate, which refers to a nucleotide having the hydroxyl group at the 2' position of the β-D-ribofuranosyl group substituted by hydrogen, and is the main chemical component of a chromosome.

"Nucleotide" is generally referred to a a single letter representing the base therein, "A" or "A nucleotide" refers to an adenine deoxyribonucleotide or an adenine ribonucleotide containing adenine, "C" or "C nucleotide" refers to a cytosine deoxyribonucleotide or a cytosine ribonucleotide containing cytosine, "G" or "G nucleotide" refers to a guanine deoxyribonucleotide or a guanine ribonucleotide containing guanine, "U" or "U nucleotide" refers to a uracil ribonucleotide containing uracil, and "T" or "T nucleotide" refers to a thymine deoxyribonucleotide containing thymine.

As used herein, the term "nucleic acid" generally refers to any compound comprising a polymer of deoxyribonucleotides (deoxyribonucleic acid, abbreviated as DNA) or a polymer of ribonucleotides (ribonucleic acid, abbreviated as RNA), or a combination thereof. In addition, nucleic acids herein also include derivatives of nucleic acids. The term "derivative of a nucleic acid" includes chemical derivatization of a nucleic acid on the base , the saccharide, or the phosphate of a nucleotide, as well as nucleic acids containing non-natural nucleotides and nucleotide analogs. In addition, nucleic acids herein can be single or double stranded in the form of linear or covalent closed circular molecules.

"Polynucleotide sequence", "nucleic acid sequence" and "nucleotide sequence" can be used interchangeably to indicate the ordering of nucleotides in a polynucleotide. It should be understood by those skilled in the art that the coding strand (sense strand) of DNA and the RNA encoded thereby can be regarded as having the same nucleotide sequence, and the deoxythymidylate in the sequence of the coding strand of DNA corresponds to the uridylate in RNA sequence encoded thereby. RNA corresponding to DNA refers to a polynucleotide with T in DNA is completely replaced with U.

A polynucleotide can comprise one or more segments (nucleic acid fragments) (e.g., 1, 2, 3, 4, 5, 6, 7, 8 segments). For example, a polynucleotide may comprise a segment encoding a polypeptide of interest, such as the polypeptides and polypeptide antigens described herein. In particular embodiments, a polynucleotide may comprise a segment encoding a polypeptide of interest as well as regulatory segments including, but not limited to, segments for the regulation of transcription and the regulation of translation. In one embodiment, the regulatory segment comprises a polynucleotide corresponding to one or more of the following regulatory elements: a promoter, a 5' untranslated region (5'-UTR), a 3' untranslated region (3'-UTR), and a poly (A) tail.

As used herein, the term "promoter" refers to a polynucleotide located upstream of the 5' end of the coding region of a gene, which contains conserved sequences specific binded by RNA polymerase and required for the initiation of transcription, and can activate RNA polymerase, so that RNA polymerase can accurately bind to template DNA and has the specificity for the initiation of transcription. The promoter may be derived from viruses, bacteria, fungi, plants, insects, and animals. Representative examples of promoters include the phage T7 promoter, the phage T3 promoter, the SP6 promoter, the lac operon-promoter, the tac promoter, the SV40 late promoter, the SV40 early promoter, the RSV-LTR promoter, the CMV IE promoter, the SV40 early promoter, or the SV40 late promoter and the CMV IE promoter.

As used herein, the term "5' untranslated region" or "5'-UTR" may be an RNA sequence in mRNA that is upstream of the coding sequence and is not translated into a protein. The 5' -UTR in a gene typically starts at the transcription initiation site and ends at the nucleotide upstream of the translation initiation codon of the coding sequence. The 5' -UTR may comprise elements that control gene expression, such as a ribosome binding site, a 5'-terminal oligo pyrimidine bundle, and a translation initiation signal such as a Kozak sequence. mRNA can be post-transcriptional modified by adding a 5' cap. Thus, 5' -UTR in mature mRNA can also refer to the RNA sequence between the 5' cap and the start codon.

As used herein, the term "3' untranslated region" or "3'-UTR" may be an RNA sequence in mRNA that is downstream of the coding sequence and is not translated into a protein. The 3' -UTR in the mRNA is located between the stop codon of the coding sequence and the poly (A) sequence, e.g., starting at the nucleotide downstream of the stop codon and ending at the nucleotide upstream of the poly (A) sequence.

As used herein, the terms "polyadenylic acid," "poly (A) sequence," and "poly (A) tail" are used interchangeably, and the naturally occurring poly (A) sequences typically consists of adenine ribonucleotides. According to the present invention, the term "modified poly(A) sequence" refers to a poly(A) sequence comprising a nucleotide or a segment of nucleotides other than adenine ribonucleotide. The poly (A) sequence is typically located at the 3' end of mRNA, e.g., the 3' end (downstream) of 3' -UTR . As used herein, the term "5'-cap structure": 5'-cap structure is typically located at the 5' end of a mature mRNA. In some embodiments, the 5'-cap structure is linked to the 5'-end of the mRNA by a 5'-5'-triphosphate linkage. The 5'-cap structure is typically formed from modified (e.g., methylated) ribonucleotides, especially from guanine nucleotide derivatives. For example, m7GpppN (cap 0 or "cap0", which is a cap structure formed by the reaction of the 5' phosphate group of hnRNA with the 5'-phosphate group of m7GTP under the action of guanylyltransferase to form a 5',5'-phosphodiester bond), wherein N is the 5'-end nucleotide of the nucleic acid carrying the 5'-cap structure. In some embodiments, the 5'-cap structure includes, but is not limited to, cap 0, cap 1 (a cap structure formed by further methylation at 2'-OH of glycosyl in the nucleotide at position 1 of hnRNA on the basis of cap 0, or referred to as "cap1"), cap 2 (a cap structure formed by further methylation at 2' -OH of glycosyl in the nucleotide at position 2 of hnRNA on the basis of cap 1, or referred to as "cap2"), cap 4, cap 0 analog, cap 1 analog, cap 2 analog, or cap 4 analog.

As used herein, the term "expression" includes transcription and/or translation of a nucleotide sequence. Thus, expression may involve the production of transcripts and/or polypeptides. The term "transcription" refers to the process of transcribing the genetic codons in a DNA sequence into an RNA (transcript). The term "in vitro transcription" refers to the in vitro synthesis of RNA, particularly mRNA, in a cell-free system (e.g., in an appropriate cell extract) (see, e.g., Pardi N., Muramatsu H., Weissman D., Karikó K. (2013). In: Rabinovich P. (eds) Synthetic Messenger RNA and Cell Metabolism Modulation. Methods in Molecular Biology (Methods and Protocols), vol 969. Humana Press, Totowa, NJ.). A vector that can be used to produce a transcript are also referred to as "transcription vector" which contains regulatory sequences required for transcription. The term "transcription" encompasses "in vitro transcription."

As used herein, the term "host cell" refers to a cell for receiving, holding, replicating, expressing a polynucleotide or vector. The term "host cell" includes prokaryotic cells (e.g., E. coli) or eukaryotic cells (e.g., yeast cells and insect cells). For example, cells from human, mouse, hamster, pig, goat, primates. Cells can be derived from a variety types of tissues and include primary cells and cell lines. Some specific examples include keratinocytes, peripheral blood leukocytes, bone marrow stem cells, and embryonic stem cells. In other embodiments, the host cell is an antigen presenting cell, particularly a dendritic cell, a monocyte, or a macrophage. Nucleic acids can be present in a host cell as a single copy or as several copies. In some embodiments, the host cell may be a cell in which the polypeptide of the present invention is expressed.

In the context of the present invention, the term "plasmid" generally refers to a circular DNA molecule, but the term may also encompass linearized DNA molecules. Specifically, the term "plasmid" also encompasses molecules obtained by linearizing a circular plasmid by, for example, digesting the circular plasmid with a restriction enzyme, thereby converting the circular plasmid molecule into a linear molecule. Plasmids can be replicated, i.e., amplified in cells independently from the genetic information stored as chromosomal DNA, and can be used for cloning, i.e., for amplifying genetic information in bacterial cells. Preferably, the DNA plasmid is a medium-copy or high-copy plasmid, more preferably a high-copy plasmid. Examples of such high-copy plasmids include, for example, pUC and pTZ plasmids or any other plasmid (e.g., pMB1, p CoIE1) that includes an origin of replication that supports the high copy of the plasmid.

The term "vaccine" is typically understood to be a prophylactic or therapeutic material that provides at least one antigen or has an antigenic function, which can stimulate the adaptive immune system in the body to provide an adaptive immune response.

The term "treating" or the like is used herein to generally refer to obtaining a desired pharmacological and/or physiological effect. Thus, the treatment in the present application may involve the treatment of the condition of a certain disease, but may also involve prophylactic treatment in terms of the complete or partial prevention of the disease or the symptoms thereof. Preferably, in some embodiments, the term "treating" should be understood to be therapeutic in partially or completely curing a disease and/or adverse effects and/or symptoms attributed to the disease. The treatment may also be a prophylactic or preventive treatment, i.e., a measure conducted to prevent a disease, e.g., to prevent an infection and/or the onset of a disease.

As used herein, the terms "subject," "subject," and "patient" may be used interchangeably. In certain embodiments, the subject is a mammal, such as human, non-human primates (e.g., simian, chimpanzee, monkey, and chimpanzee), domestic animals (including dog and cat and livestocks (e.g., horse, cow, pig, sheep, and goat)), or other mammals. Other mammals include, but are not limited to, mouse, rat, guinea pig, rabbit, hamster, etc. In particular embodiments, the subject is human. In one embodiment, the subject is a mammal (e.g., human) suffering from an infectious disease or a neoplastic disease . In another embodiment, the subject is a mammal (e.g., human) at risk of developing an infectious or neoplastic disease.

As used herein, the term "administering" refers to providing or administering an agent to a subject by any effective route. Exemplary routes of administration include, but are not limited to, one or more of the follows: injection (e.g., subcutaneous, intramuscular, intradermal, intraperitoneal, intrathecal, intracerebroventricular, or intravenous), oral, intraductal, sublingual, rectal, transdermal, intranasal, vaginal, and inhalation. When used to treat a disease, disorder, condition, or symptom thereof, the administration of the substance is typically performed after the onset of the disease, disorder, condition, or symptom thereof. When used to prevent a disease, disorder, condition or symptom, the administration of the substance is typically performed prior to the onset of the disease, disorder, condition or symptom. Herein, some elements of the present invention will be described. These elements are listed together with specific embodiments, however it should be understood that they can be combined in any manner and in any number to generate additional embodiments. The different described examples and preferred embodiments should not be construed as limiting the invention to the explicitly described embodiments only. This specification should be understood to support and include embodiments that combine the explicitly described embodiments with any number of the disclosed and/or preferred elements. For example, in one embodiment, the fragment of the VZV gE protein is further linked to a heterologous signal peptide, and in another embodiment, the heterologous signal peptide is an IgGκ signal peptide, then the following solution is also an embodiment claimed in the present application: the fragment of the VZV gE protein is further linked to a heterologous signal peptide, and the heterologous signal peptide is an IgGκ signal peptide. In addition, any arrangement and combination of all described elements in the present invention should be construed as disclosed in the description of the present application unless indicated otherwise in the context.

### II. Nucleic Acid

One embodiment of the present disclosure provides a nucleic acid comprising a polynucleotide encoding a VZV gE protein or a fragment thereof. As used herein, "VZV gE protein or a fragment thereof" is short for a VZV gE protein or fragment of a VZV gE protein.

As used herein, a fragment of a VZV gE protein is an immunogenic derivative that retains the ability to induce an immune response to VZV after administration to a human (e.g., an immunogenic fragment of a VZV gE protein).

In some embodiments, the amino acid sequence of the VZV gE protein comprises or is an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 1.

In some embodiments, the VZV gE protein is a full-length gE protein. In some embodiments, the amino acid sequence of the VZV gE protein is set forth in SEQ ID NO: 10.

In some embodiments, the fragment of the VZV gE protein is one of the follows: (1) a truncated polypeptide of positions 1 to 623, positions 23 to 623, positions 31 to 623, positions 1 to 561, positions 1 to 573, positions 1 to 543, positions 31 to 573, or positions 31 to 543 of the VZV gE protein; and (2) a polypeptide having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequence of the truncated polypeptide in (1). It should be noted that the positions of amino acids in positions 1 to 623, positions 23 to 623, positions 31 to 623 and the like herein are numbered based on the corresponding positions of amino acids in the polypeptide shown in SEQ ID NO: 1. It should be noted, and one skilled in the art will understand, that different VZV gE proteins may have different numbering systems, e.g., adding or removing additional amino acid residues as compared to SEQ ID NO: 1. Thus, it should be understood that when a particular amino acid residue is referred to by a number, the description is not limited to the amino acid located exactly at that numbered position when counted from a given amino acid sequence, but also refers to the equivalent/corresponding amino acid residue in any and all VZV gE protein sequences, even if the residue is not at the same exact numbered position, e.g., if the VZV gE protein is shorter or longer than SEQ ID NO: 1, or has an insertion or deletion compared to SEQ ID NO: 1. It can be understood that in other genotypes of the VZV virus, the polypeptide identical to the truncated polypeptide corresponding to the above position number description is also a fragment of the VZV gE protein of the present disclosure. In some embodiments, the VZV gE protein or a fragment thereof is derived from the Oka strain.

In some embodiments, the VZV gE protein or a fragment thereof further has a mutation. In some embodiments, the VZV gE protein or a fragment of the VZV gE protein has at least one mutation in at least one motif of the VZV gE protein, which is associated with ER retention, endocytosis, and/or localization in the Golgi body or trans-Golgi network (TGN). In some embodiments, the VZV gE protein or a fragment of the VZV gE protein has a mutation associated with localization in the Golgi body or trans-Golgi network that is capable of causing a reduction in the VZV gE polypeptide targeted or localized in the Golgi body or trans-Golgi network. In some embodiments, the VZV gE protein or a fragment of the VZV gE protein has a mutation associated with endocytosis that can result in a decrease in endocytosis of the VZV gE polypeptide. In some embodiments, the VZV gE protein or fragment of the VZV gE protein has at least one mutation in at least one phosphorylation acidic motif. In some embodiments, the VZV gE protein or the fragment package of the VZV gE protein has one or both of the following mutations: Y569A mutation and Y582G mutation.

In some embodiments, the VZV gE protein or a fragment thereof comprises or is one of the following polypeptides or proteins:
(1) a polypeptide or protein having an amino acid sequence as set forth in one of SEQ ID NOs: 1-8; and
(2) a polypeptide or protein comprising an amino acid sequence having at least 90%, 91%, 92%, 93%, 94%, 95%, 96%, 97%, 98% or 99% identity to one of the sequences SEQ ID NOs: 1-8.

In some embodiments, the VZV gE protein or a fragment thereof is further linked to ferritin and/or a heterologous signal peptide.

In some embodiments, the VZV gE protein or a fragment thereof is further linked to a heterologous signal peptide.

In some embodiments, the VZV gE protein or a fragment thereof is linked to a heterologous signal peptide; the heterologous signal peptide is located at the N-terminus or C-terminus of the VZV gE protein or a fragment thereof.

In some embodiments, the heterologous signal peptide comprises one or more of an IgE signal peptide and an IgGκ signal peptide. In some embodiments, the heterologous signal peptide is an IgE HC (Ig heavy chain ε-1) signal peptide. In some embodiments, the heterologous signal peptide has the sequence MDWTWILFLVAAATRVHS. In some embodiments, the heterologous signal peptide has the sequence METPAQLLFLLLLWLPDTTG. In some embodiments, the heterologous signal peptide is selected from one or more of the follows: Japanese encephalitis PRM signal sequence (MLGSNSGQRVVFTILLLLVAPAYS), VSV g protein signal sequence (MKCLLYLAFLFIGVNCA), and Japanese encephalitis JEV signal sequence (MWLVSLAIVTACAGA).

In some embodiments, the IgGκ signal peptide comprises or is one of: (1) a polypeptide having an amino acid sequence as set forth in SEQ ID NO: 9; and (2) a polypeptide having at least 90%, 91%, 92%, 93%, 94%, 95%, 96%, 97%, 98%, or 99% identity to the amino acid sequence as set forth in SEQ ID NO: 9.

In some embodiments, one of the following VZV gE proteins or fragments thereof is further linked to a heterologous signal peptide: a truncated polypeptide of positions 1 to 623, positions 23 to 623, positions 31 to 623, positions 1 to 561, positions 1 to 573, positions 1 to 543, positions 31 to 573, or positions 31 to 543 of the VZV gE protein. In some embodiments, the VZV gE protein or a fragment thereof comprises or is a polypeptide or protein having an amino acid sequence as set forth in SEQ ID NO: 1, 4 or 5, and the VZV gE protein or a fragment thereof is not linked to a heterologous signal peptide. In other embodiments, the ZV gE protein or fragment thereof comprises or is a polypeptide or protein having an amino acid sequence as set forth in SEQ ID NO: 1, 4 or 5, and the VZV gE protein or a fragment thereof is linked to a heterologous signal peptide.

In some embodiments, the VZV gE protein or a fragment thereof comprises or is a polypeptide or protein having an amino acid sequence as set forth in SEQ ID NO: 1, 2, 3, 6, 7 or 8, and the VZV gE protein or a fragment thereof is linked to a heterologous signal peptide.

In some embodiments, the VZV gE protein or a fragment thereof is further linked to ferritin.

In some embodiments, ferritin comprises or is one of: (1) a polypeptide or protein having an amino acid sequence as set forth in SEQ ID NO: 10; and a polypeptide or protein having at least 90%, 91%, 92%, 93%, 94%, 95%, 96%, 97%, 98%, or 99% identity to the amino acid sequence as set forth in SEQ ID NO: 10.

In some embodiments, the polypeptide or protein encoded by the nucleic acid comprises or is one of the follows:
(1) a polypeptide or protein having an amino acid sequence as set forth in one of SEQ ID NOs: 1, 4, 5 and 28-33; and
(2) a polypeptide or protein comprising an amino acid sequence having at least 90%, 91%, 92%, 93%, 94%, 95%, 96%, 97%, 98% or 99% identity to one of the sequences SEQ ID NOs: 1, 4, 5 and 28-33.

In some embodiments, the above nucleic acid further comprises a polynucleotide encoding a protein or polypeptide other than VZV gE protein or a fragment thereof. It can be understood that the protein or polypeptide other than the VZV gE protein or a fragment thereof refers to a protein or polypeptide with biological meaning (e.g., immunogenicity), for example, other proteins (e.g., envelope protein) of VZV, a fragment, a variant or a variant of the fragment thereof, other glycoproteins of VZV (e.g., one or more of gI, gB, gH, gK, gL, gC, gN and gM of VZV), a fragment, a variant, or a variant of the fragment thereof, a protein or polypeptide of other viruse (e.g., HA protein of influenza virus or S protein of SARS-CoV-2), fragment, variant or fragment thereof, immunoglobulin Fc region, a fragment, a variant, or a variant of the fragment thereof, ferritin, a fragment, a variant, or a variant of the fragment thereof, or flagellin, a fragment, a variant, or a variant of the fragment thereof. It should be noted that "A, a fragment, a variant, or a variant of the fragment" herein is short for A, a fragment of A, a variant of A, or a variant of a fragment of A.

In some embodiments, the nucleic acid encodes one or more proteins or polypeptides other than the VZV gE protein or a fragment thereof. For example, the protein or polypeptide other than the VZV gE protein or a fragment thereof encoded by the nucleic acid is one or more of the following (1) to (5): (1) other protein of VZV (e.g., envelope protein), a fragment, a variant, or a variant of the fragment thereof, (2) other glycoprotein of VZV (e.g., one or more of gI, gB, gH, gK, gL, gC, gN and gM of VZV), a fragment, a variant, or a variant of the fragment thereof, (3) a protein or polypeptide of other virus (e.g., HA protein of influenza virus or S protein of ARS- CoV-2), a fragment, a variant, or a variant of the fragment thereof, (4) Fc region of immunoglobulin (e.g., human IgG, IgA, IgD, gE or IgM.), a fragment, a variant, or a variant of the fragment thereof, and (5) ferritin, a fragment, a variant, or a variant of the fragment thereof.

In some embodiments, the polynucleotide encoding the VZV gE protein or a fragment thereof is codon optimized. Codon optimization methods are known in the art and may be used as provided herein. In some embodiments, the codon optimization can be used to: match the codon frequencies of the target and host organisms to ensure proper folding; bias GC content to increase mRNA stability or reduce secondary structure; minimize tandem repeat codons or base extension that can impair gene construction or expression; customize transcription and translation control regions; insert or remove protein transport sequences; remove/add post-translation modification sites in the encoded protein (e.g., glycosylation site); add, remove, or shuffle protein domains; insert or delete restriction sites; modify ribosome binding sites and mRNA degradation sites; modulate translation rates so that various domains of the protein can be folded properly; or reduce or eliminate problematic secondary structures within the polynucleotide. Codon optimization tools, algorithms, and services are known in the art, non-limiting examples include services from GeneArt (Life Technologies), DNA2.0(Menlo Park CA), and/or proprietary methods. In some embodiments, the open reading frame (ORF) sequence is optimized using an optimization algorithm. In some embodiments, the codon-optimized nucleic acid is at least 50%, 55%, 60%, 65%, 70%, 75%, 80%, 85%, 90%, 95%, or 99% identical to the nucleic acid prior to the codon-optimization.

In some embodiments, the polynucleotide encoding the VZV gE protein or a fragment thereof comprises or is one of the follows:
(1) an RNA corresponding to a polynucleotide having a nucleotide sequence set forth in one of SEQ ID NOs: 11-18; and
(2) an RNA corresponding to a polynucleotide comprising a nucleotide sequence having at least 50%, 55%, 60%, 65%, 70%, 75%, 80%, 85%, 90%, 91%, 92%, 93%, 94%, 95%, 96%, 97%, 98% or 99% identity to the nucleotide sequence set forth in one of SEQ ID NOs: 11-18.

In some embodiments, the polynucleotide encoding the heterologous signal peptide is codon optimized.

In some embodiments, the polynucleotide encoding the heterologous signal peptide comprises or is one of: (1) a polynucleotide having a nucleotide sequence as set forth in SEQ ID NO: 34 or a corresponding RNA thereof; and (2) a polynucleotide having at least 50%, 55%, 60%, 65%, 70%, 75%, 80%, 85%, 90%, 91%, 92%, 93%, 94%, 95%, 96%, 97%, 98%, or 99% identity to the nucleotide sequence as set forth in SEQ ID NO: 34 or a corresponding RNA thereof.

In some embodiments, the polynucleotide encoding ferritin is codon optimized.

In some embodiments, the polynucleotide encoding ferritin comprises one of the following polynucleotides: (1) a polynucleotide having a nucleotide sequence as set forth in SEQ ID NO: 35 or a corresponding RNA thereof; and (2) a polynucleotide having at least 50%, 55%, 60%, 65%, 70%, 75%, 80%, 85%, 90%, 91%, 92%, 93%, 94%, 95%, 96%, 97%, 98% or 99% identity to the nucleotide sequence as set forth in SEQ ID NO: 35 or a corresponding RNA thereof.

In some embodiments, the nucleic acid comprises or is one of the following polynucleotides:
(1) a polynucleotide having a nucleotide sequence as set forth in one of SEQ ID NOs: 11, 14, 15 and 19-24 or a corresponding RNA thereof; and
(2) a polynucleotide comprising a nucleotide sequence having at least 50%, 55%, 60%, 65%, 70%, 75%, 80%, 85%, 90%, 91%, 92%, 93%, 94%, 95%, 96%, 97%, 98% or 99% identity to the nucleotide sequence set forth in one of SEQ ID NOs: 11, 14, 15 and 19-24 or a corresponding RNA thereof.

In some embodiments, the nucleic acid is mRNA.

It can be understood that in the above nucleic acids, whether the polynucleotide encoding the VZV gE protein or the fragment thereof or the polynucleotide encoding the protein or polypeptide other than the VZV gE protein or the fragment thereof may have a plurality of (e.g., two or three) repeat units on the same strand. For example, for a polynucleotide encoding a VZV gE protein, the polynucleotide encoding the VZV gE protein is simply referred to as an "A fragment", and the same nucleic acid above may contain a plurality of A fragments. For another example, for a polynucleotide encoding a VZV gE protein and other glycoprotein of VZV (e.g., VZV gl), the polynucleotide encoding the VZV gE protein is referred to as "A fragment" for short, and the polynucleotide encoding the other glycoprotein of VZV is referred to as "B fragment" for short, then the same nucleic acid above may contain a plurality of A fragments and a plurality of B fragments, may contain one A fragment and a plurality of B fragments, or may contain a plurality of A fragments and one B fragment. Polynucleotides encoding multiple proteins located on the same nucleic acid strand are linked directly or indirectly through a linker element (e.g., a polynucleotide encoding a linker peptide such as a 2A peptide, G, GG, GGG, GS, or SAIG, etc.). The GS linker peptide is composed of Gly and Ser residues, for example (Gly-Gly-Gly-Gly-Ser)n, and the length of the GS linker peptide can be changed by adjusting the value of n (the number of repeats), so that the two linked proteins can be optimally separated or the interaction with each other is enabled.

In some embodiments, the nucleic acid comprises a plurality of polynucleotides encoding a VZV gE protein or a fragment thereof. For example, a polynucleotide encoding the full length of a VZV gE protein is referred to as an "A1 fragment" for short, and a polynucleotide encoding a fragment of a VZV gE protein is referred to as an "A2 fragment" for short, then the same nucleic acid may contain one or more A1 fragments and one or more A2 fragments. In some embodiments, the above nucleic acid is an isolated nucleic acid.

In some embodiments, the above nucleic acid is a non-naturally occurring nucleic acid. In some embodiments, the above nucleic acid is RNA.

In some embodiments, the above nucleic acid is RNA comprising an open reading frame (ORF).

In some embodiments, the nucleic acid is RNA, and the RNA is mRNA.

In some embodiments, the above nucleic acid is a non-self-replicating mRNA.

In some embodiments, the above nucleic acid is mRNA which comprises at least one of a 5'-cap structure, a 5'-UTR, a 3'-UTR, and a poly(A) tail. In an optional specific example, the above nucleic acid further comprises a 5'-cap structure, a 5'-UTR, a 3'-UTR and a poly(A) tail.

In some embodiments, the 5' -cap structure is selected from at least one of m⁷GpppG, m₂^{7,3'}-^{O}GpppG, m⁷G ppp (5')N1 and m⁷Gppp(m^{2'}- ^{O})N 1; wherein "m⁷G" represents a 7-methyl guanosine cap nucleoside, "ppp" represents a triphosphate bond between the 5' carbon of the cap nucleoside and the first nucleotide of the primary RNA transcript, N1 is the 5'-most nucleotide, "G" represents a guanosine, "7" represents a methyl group at the 7-position of guanosine, and "m^{2'}- ^{O}" represents a methyl group at the 2' -O position of nucleotide. In some embodiments, the 5' -cap structure is m⁷G ppp (5')N1 or m⁷Gppp(m^{2'}- ^{O})N1. It can be understood that in other embodiments, the 5'-cap structure is not limited to the above.

In some embodiments, the DNA sequence corresponding to the 5'-UTR is set forth in SEQ ID NO: 25.

In some embodiments, the DNA sequence corresponding to the 3'-UTR is set forth in SEQ ID NO: 26. It should be noted that the "DNA sequence corresponding to 5'-UTR" refers to the nucleotide sequence of 5'-UTR in DNA form, and the same applies to the "DNA sequence corresponding to 3'-UTR" and the "DNA sequence corresponding to poly(A) tail" below. It may be understood that, in other embodiments, the 5'-UTR and the 3'-UTR are not limited to the above, and may alternatively be 5'-UTR and 3'-UTR described in other patents such as CN108291230A, CN104321432A, and CN107849574A.

In some embodiments, the nucleotides constituting the poly(A) tail comprise at least 20, at least 40, at least 80, at least 100, or at least 120 A nucleotides. In some embodiments, the nucleotides constituting the poly(A) tail comprise at least 20, at least 40, at least 80, at least 100, or at least 120 A continuous nucleotides. In some embodiments, the nucleotides constituting the poly(A) tail comprise one or more nucleotides other than the A nucleotide. In an optional specific example, the nucleotide DNA sequence corresponding to the poly(A) tail is set forth in SEQ ID NO: 27. It can be understood that in other embodiments, the poly(A) tail is not limited to the above, and may also be other poly(A) tails, such as the poly(A) tail described in patents such as US20170166905A1 and WO2020074642A1.

In some embodiments, the above nucleic acid does not contain a modified nucleotide.

In some embodiments, the above nucleic acid contains a modified nucleotide.

In some embodiments, the above nucleic acid contains a modified nucleoside. In some embodiments, the modified nucleoside comprises at least one of a modified uridine, a modified cytidine, a modified adenosine, and a modified guanosine.

In some embodiments, the modified nucleoside in the above nucleic acid is a modified uridine. In some embodiments, 0.1%~100% of the uridines in the above nucleic acid are modified. In some embodiments, 80%~100% of the uridines are modified. In some embodiments, 100% of the uridines are modified. Exemplary modified uridine comprises and is not limited to one or more of the follows: pseudouridine (ψ), N1-methylpseudouridine, pyridin-4-one ribonucleoside, 5-aza-uridine, 6-aza-uridine, 2-thio-5-aza-uridine, 2-thio-uridine (s2U), 4-thio-uridine (s4U), 4-thio-uridine, 2-thio-pseudouridine, 5-hydroxy-uridine (ho5U), 5-aminoallyl-uridine, 5-halo-uridine (e.g., 5-iodo-uridine or 5-bromo-uridine), 3-methyl-uridine (m3U), 5-methoxy-uridine (mo5U), uridine-5-oxyacetic acid (cmo5U), uridine-5-oxyacetate methyl ester (mcmo5U), 5-carboxymethyl-uridine (cm5U), 1-carboxymethyl-pseudouridine, 5-carboxyhydroxymethyl-uridine (chm5U), 5-carboxyhydroxymethyl-uridine methyl ester (mchm5U), 5-methoxycarbonylmethyl-uridine (mem5U), 5-methoxycarbonylmethyl-2-thio-uridine (mcm5s2U), 5-aminomethyl-2-thio-uridine (nm5s2U), 5-methylaminomethyl-uridine (mnm5U), 5-methylaminomethyl-2-thio-uridine (mnm5s2U), 5-methylaminomethyl-2-seleno-uridine (mnm5se2U), 5-carbamoylmethyl-uridine (ncm5U), 5-carboxymethylaminomethyl-uridine (cmnm5U), 5-carboxymethylaminomethyl-2-thio-uridine (cmnm5s2U), 5-propynyl-uridine, 1-propynyl-pseudouridine, 5-tauromethyl-uridine (τm5U), 1-Taurine methyl-pseudouridine, 5-Taurine methyl-2-thio-uridine (τm5s2U), 1-Taurine methyl-4-thio-uridine, 5-methyl-uridine (m5U, i.e., deoxythymine with nucleobase), 1-methyl-pseudouridine (m1ψ), 5-methyl-2-thio-uridine (m5s2U), 1-Methyl-4-thio-pseudouridine (m1s4ψ), 4-thio-1-methyl-pseudouridine, 3-methyl-pseudouridine (m3ψ), 2-thio-1-methyl-pseudouridine, 1-methyl-1-denitride-pseudouridine, 2-thio-1-methyl-1-denitride-pseudouridine, dihydrouridine (D), dihydropseudouridine, 5,6-dihydrouridine, 5-methyl-dihydrouridine (m5D), 2-thio-dihydrouridine, 2-thio-dihydrouridine, 2-methoxy-uridine, 2-methoxy-4-thio-uridine, 4-methoxy-uridine, 4-methoxy-2-thio-uridine, N1-methyl-uridine, 3-(3-amino-3-carboxypropyl) uridine (acp3U), 1-methyl-3-(3-amino-3-carboxypropyl) pseudouridine (acp3ψ), 5- (isopentenylaminomethyl) uridine (inm5U), 5- (isopentenylaminomethyl)-2-thio-uridine (inm5s2U), α-thio-uridine, 2'-O-methyl-uridine (Um), 5,2'-O- dimethyl-uridine (m5U m), 2'-O-methyl-pseudouridine (ψm), 2-thio-2'-O-methyl-uridine (s2Um), 5-methoxycarbonylmethyl-2'-O-methyl-uridine (mcm5Um), 5-carbamoylmethyl-2'-O-methyl-uridine (ncm5Um), 5-carboxymethylaminomethyl-2'-O-methyl-uridine (cmnm5Um), 3,2'-O-dimethyl-uridine (m3Um), 5-(isopentenylaminomethyl)-2'-O- methyl-uridine (inm5Um), 1-thio-uridine, deoxythymidine, 2'-F-ara-uridine, 2'-F-uridine, 2'-OH-ara-uridine, 5-(2-carbomethoxyvinyl) uridine, and 5-[3-(1-E-propenylamino) uridine.

In some embodiments, the modified nucleoside in the above nucleic acid is a modified cytidine. In some embodiments, 0.1%~100% of the cytidines in the above nucleic acid are modified. In some embodiments, 80%~100% of the cytidine are modified. In some embodiments, 100% of the cytidine are modified. Exemplary modified cytidine comprises but is not limited to one or more of the follows: 5-aza-cytidine, 6-aza-cytidine, pseudoisocytidine, 3-methyl-cytidine (m3C), N4-acetyl-cytidine (ac4C), 5-formyl-cytidine (f5C), N4-methyl-cytidine (m4C), 5-methyl-cytidine (m5C), 5-halo-cytidine (e.g., 5-iodo-cytidine), 5-hydroxymethyl-cytidine (hm5C), 1-methyl-pseudoisocytidine, pyrrolo-cytidine, pyrrolo-pseudoisocytidine, 2-thio-cytidine (s2C), 2-thio-5-methyl-cytidine, 4-thio-pseudoisocytidine, 4-thio-1-methyl-pseudoisocytidine, 4-thio-1-methyl-1-deaza-pseudoisocytidine, 1-methyl-1-deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, 4-methoxy-pseudoisocytidine, 4-ethoxy-1-methyl-pseudoisocytidine, Lysidine (K2C), α-thio-cytidine, 2'-O-methyl-cytidine (Cm), 5,2'-O-dimethyl-cytidine (m5Cm), N4-Acetyl-2'-O-methyl-cytidine (ac4Cm), N4,2'-O-dimethyl-cytidine (m4Cm), 5-formyl-2'-O-methyl-cytidine (f5Cm), N4,N4,2'-O-trimethyl-cytidine (m42Cm), 1-thio-cytidine, 2'-F-arabino-cytidine, 2'-F-cytidine, and 2'-OH-arabino-cytidine.

In some embodiments, the modified nucleoside in the above nucleic acid is a modified adenosine. In some embodiments, 0.1%~100% of the adenosines in above the nucleic acid are modified. In some embodiments, 80%~100% of the adenosines are modified. In some embodiments, 100% of the adenosines are modified. Exemplary modified adenosine is selected from but is not limited to one or more of the follows: 2-amino-purine, 2,6-diaminopurine, 2-amino-6-halo-purine (e.g., 2-amino-6-chloro-purine), 6-halo-purine (e.g., 6-chloro-purine), 2-amino-6-methyl-purine, 8-azido-adenosine, 7-deaza-adenine, 7-deaza-8-aza-adenine, 7-deaza-2-amino-purine, 7-deaza-8-aza-2-amino-purine, 7-deaza-2,6-diaminopurine, 7-deaza-8-aza-2,6-diaminopurine, 1-methyl-adenosine (m1A), 2-methyl-adenine (m2A), N6-methyl-adenosine (m6A), 2-methylthio-N6-methyl-adenosine (ms2m6A), N6-isopentenyl-adenosine (i6A), 2-methylthio-N6-isopentenyl-adenosine (ms2i6A), N6- (cis-hydroxyisopentenyl) adenosine (io6A), 2-methylthio -N6- (cis-hydroxyisopentenyl) adenosine (ms2io6A), N6-glycinylcarbamoyl-adenosine (g6A), N6-threcarbamoyl-adenosine (t6A), N6-methyl -N6- threcarbamoyl-adenosine (m6t6A), 2-methylthio -N6-threyl carbamoyl-adenosine (ms2g6A), N6,N6-dimethyl-adenosine (m62A), N6-hydroxyvalylcarbamoyl-adenosine (hn6A), 2-methylthio-N6-hydroxyvalylcarbamoyl-adenosine (ms2hn6A), N6-acetyl-adenosine (ac6A), 7-methyl-adenine, 2-methylthio-adenine, 2-methoxy-adenine, α-thio-adenosine, 2'-O-methyl-adenosine (Am), N6,2'-O-dimethyl-adenosine (m6Am), N6,N6,2'-O- trimethyl-adenosine (m62Am), 1,2'-O-dimethyl-adenosine (m1Am), 2'-O-ribosyl-adenosine (phosphate) (Ar(p)), 2-amino-N6-methyl-purine, 1-thio-adenosine, 8-azido-adenosine, 2'-F-arabine-adenosine, 2'-F-adenosine, 2'-OH-arabine-adenosine, and N6-(19-amino-pentaoxa-nonadecyl)-adenosine.

In some embodiments, the modified nucleoside in the above nucleic acid is a modified guanosine. In some embodiments, 0.1%~100% of the guanosines in the above nucleic acid are modified. In some embodiments, 80%~100% of the guanosines are modified. In some embodiments, 100% of the guanosines are modified. Exemplary modified guanosine is selected from but is not limited to one or more of the follows: inosine (I), 1-methyl-inosine (m1I), wyosine (imG), methyl wyosine (mimG), 4-demethyl-wyosine (imG-14), isowyosine (imG2), wybutosine (yW), peroxywybutosine (o2yW), hydroxywybutosine (OHyW), undermodified hydroxywybutosine (OHyW *), 7-deaza-guanosine, queuosine (Q), epoxy-queuosine (oQ), galactosyl-queuosine (galQ), mannosyl-queuosine (manQ), 7-cyano-7-deaza-guanosine (preQ0), 7-aminomethyl-7-deaza-guanosine (preQ1), archaeosine (G+), 7-deaza-8-aza-guanosine, 6-thio-guanosine, 6-thio-7-deaza-guanosine, 6-thio-7-deaza--8-aza-guanosine, 7-methyl-guanosine (m7G), 6-thio-7-methyl-guanosine, 7-methyl-inosine, 6-methoxy-guanosine, 1-methyl-guanosine (m1G), N2-methyl-guanosine (m2G), N2,N2-dimethyl-guanosine (m22G), N2,7-dimethyl-guanosine (m2,7G), N2,N2,7-trimethyl-guanosine (m2,2,7G), 8-oxo-guanosine, 7-methyl-8-oxo-guanosine, 1-methyl-6-thio-guanosine, N2-methyl-6-thio-guanosine, N2,N2-dimethyl-6-thioguanosine, α-thio-guanosine, 2'-O-methyl-guanosine (Gm), N2-methyl-2'-O-methylguanosine (m2Gm), N2,N2-dimethyl-2'-O-methyl-guanosine (m22Gm), 1-methyl-2'-O-methyl-guanosine (m1Gm), N2,7-dimethyl-2'-O-methyl-guanosine (M2,7Gm), 2'-O-methyl-inosine (Im), 1,2'-O-dimethyl-inosine (m1Im), 2'-O-ribosyl-guanosine (phosphate) (Gr(p)), 1-thio-guanosine, O6-methyl-guanosine, 2'-F-arabino-guanosine, and 2'-F-guanosine.

In some embodiments, the modified nucleotide in the above nucleic acid comprises a nucleotide containing an isotope.

In some embodiments, the above nucleic acid comprises a nucleotide containing an isotope of hydrogen. The isotope of hydrogen is not limited to deuterium, tritium. In addition, in some embodiments, the above nucleic acid further comprises or is a nucleotide containing an isotope of other elements than hydrogen, wherein the other elements include, but are not limited to, carbon, oxygen, nitrogen and phosphorus.

In some embodiments, all of the uridine in the nucleic acid is replaced with N1-methylpseudouridine, and comprises or is one of the following RNAs: (1) an RNA corresponding to a polynucleotide having a nucleotide sequence as set forth in one of SEQ ID NOs: 11, 14, 15 and 19-24; and (2) an RNA corresponding to a polynucleotide comprising a nucleotide sequence having at least 50%, 55%, 60%, 65%, 70%, 75%, 80%, 85%, 90%, 91%, 92%, 93%, 94%, 95%, 96%, 97%, 98% or 99% identity to the nucleotide sequence as set forth in one of SEQ ID NOs: 11, 14, 15 and 19-24.

In some embodiments, the nucleic acid is RNA, and the nucleic acid has a length of at least 1000 nt, 1200 nt, 1500 nt, 1550 nt, 1600 nt, 1650 nt, 1700 nt, 1700 nt, 1850 nt, 1900 nt, 1950 nt, 2000 nt, 2050 nt, 2100 nt, 2150 nt, 2200 nt, 2250 nt, 2300 nt, 2350 nt, 2400 nt, 2450 nt, 2500 nt, 2550 nt, 3000 nt, 3100 nt, 3200 nt, 3300 nt, 3400 nt, 3500 nt, 3600 nt, 3700 nt, 3800 nt, 3900 nt, 4000 nt, 4100 nt, 4200 nt, 4300 nt, 4400 nt, 4500 nt, 4600 nt, 4700 nt, 4800 nt, 4900 nt, 5000 nt, 5100 nt, 5200 nt, 5300 nt, 5400 nt, or 5500 nt.

In some embodiments, the nucleic acid is RNA, and the nucleic acid has a length of 1000 nt-8000 nt, 1500 nt- 7000 nt, 1500 nt- 6500 nt, 1500-6000 nt, 1500-5500 nt, 1500-5000 nt, 1500-4500 nt, 1500-4000 nt, 1500-3500 nt, 1500-3000 nt, 1600-2500 nt, 1700 nt- 2400 nt, 1800-2400 nt, or 1900-2400 nt.

In some embodiments, the nucleic acid is DNA.

In some embodiments, the nucleic acid is DNA, and the DNA can be transcribed into RNA.

In some embodiments, the nucleic acid is DNA comprising ORF.

In some embodiments, the nucleic acid comprises or is one of the following nucleotides: (1) a polynucleotide having a nucleotide sequence as set forth in one of SEQ ID NOs: 11, 14, 15 and 19-24; and (2) a polynucleotide comprising a nucleotide sequence having at least 50%, 55%, 60%, 65%, 70%, 75%, 80%, 85%, 90%, 91%, 92%, 93%, 94%, 95%, 96%, 97%, 98%, or 99% identity to the nucleotide sequence as set forth in one of SEQ ID NOs: 11, 14, 15 and 19-24. In some embodiments, the nucleic acid does not comprise a start codon and/or a stop codon, which may be added when used.

In some embodiments, the nucleic acid comprises a stop codon. It can be understood that the stop codon in the nucleic acid can be replaced with other stop codons, and of course, other stop codons can be further added.

In some embodiments, the stop codon is one or more of TAG, TAA, TGA, UAA, UAG, and UGA.

In some embodiments, the stop codon in the nucleic acid may be one or more, for example, two or three.

In some embodiments, the above nucleic acid is the mRNA in Table 1. An mRNA in Table 1 is an mRNA encoding a VZV gE protein or a fragment thereof, which comprises a Cap1 type cap structure, a 5'-UTR corresponding to the sequence shown in SEQ ID NO: 25, a 3'-UTR corresponding to the sequence shown in SEQ ID NO: 26, a poly(A) tail corresponding to the sequence shown in SEQ ID NO: 27, and a protein coding region of a gE protein or a fragment thereof corresponding to one sequence of SEQ ID NOs: 11-18, wherein the coding region of the VZV gE protein or the fragment thereof is RNA, and the uridines in all the mRNAs in Table 1 are replaced with N1-methylpseudouridine. For example, the mRNA shown in No. 1087 comprises a Cap1 cap structure, a 5'-UTR corresponding to the sequence shown in SEQ ID NO: 25, a 3'-UTR corresponding to the sequence shown in SEQ ID NO: 26, a poly(A) tail corresponding to the sequence shown in SEQ ID NO: 27, and an gE protein coding region corresponding to the sequence shown in SEQ ID NO: 13, and all uridines in the mRNA shown in No. 1087 are replaced with N1-methylpseudouridine. In addition, in Table 1, the mRNA numbered as 466, 1086, 1087, 1116 and 1117 further contains a polynucleotide encoding the IgGκ signal peptide as set forth in SEQ ID NO: 9 (i.e., SEQ ID NO: 34) at the N-terminus (5' terminus) of the coding region of the gE protein or fragment thereof. For example, the DNA sequence corresponding to the coding region of the mRNA shown in No. 466 is set forth in SEQ ID NO: 19, which comprises a polynucleotide (SEQ ID NO: 34) encoding the IgGk signal peptide further added at the 5' end of the coding region of the gE protein or fragment thereof; the mRNA shown in No. 1107 and 1117 further comprises a polynucleotide (i.e., SEQ ID NO: 35) encoding the ferritin shown in SEQ ID NO: 10 at the C-terminus (3' end) of the coding region of the gE protein fragment thereof, and the polynucleotide encoding the ferritin shown in SEQ ID NO: 10 is linked to the polynucleotide encoding the gE protein fragment via a linker (AGCGCCATCGGC). For example, the DNA sequence corresponding to the coding region of the mRNA shown in No. 1107 is set forth in SEQ ID NO: 22, which comprises a polynucleotide encoding ferritin (SEQ ID NO: 35) further added at the 3' end of the coding region of the gE protein or fragment thereof.

**Table 1**

| mRNA NO. | Amino acid sequence of VZV gE protein or the fragment thereof | DNA sequence corresponding to coding region for VZV gE protein or the fragment thereof | DNA sequence corresponding to the coding region in mRNA |
|---|---|---|---|
| 466 | SEQ ID NO: 1 | SEQ ID NO: 11 | SEQ ID NO: 19 |
| 468 | SEQ ID NO: 1 | SEQ ID NO: 11 | SEQ ID NO: 11 |
| 1086 | SEQ ID NO: 2 | SEQ ID NO: 12 | SEQ ID NO: 20 |
| 1087 | SEQ ID NO: 3 | SEQ ID NO: 13 | SEQ ID NO: 21 |
| 1104 | SEQ ID NO: 4 | SEQ ID NO: 14 | SEQ ID NO: 14 |
| 1106 | SEQ ID NO: 5 | SEQ ID NO: 15 | SEQ ID NO: 15 |
| 1107 | SEQ ID NO: 6 | SEQ ID NO: 16 | SEQ ID NO: 22 |
| 1116 | SEQ ID NO: 7 | SEQ ID NO: 17 | SEQ ID NO: 23 |
| 1117 | SEQ ID NO: 8 | SEQ ID NO: 18 | SEQ ID NO: 24 |

The nucleic acid of the present disclosure can induce the body to produce a significant immune response, cause the body to produce a high level of IgG antibody against VZV gE protein, and induce a significant IFN-γ-positive cellular immune response.

In some embodiments, the VZV gE protein or a fragment thereof produced by the above nucleic acid can induce the body to produce an IFN-γ-positive cellular immune response. In some embodiments, the VZV gE protein or a fragment thereof produced by the nucleic acid can induce the body to produce an IFN-γ-positive cellular immune response, which is Th1-bias.

### III. Genetic Engineering Vector, Method of Preparing Nucleic Acid, Host Cell, Protein or Polypeptide

The present disclosure also provides a genetic engineering vector comprising the nucleic acid of any of the above embodiments, or a polynucleotide capable of being transcribed into the nucleic acid of any of the above embodiments.

In some embodiments, the above genetic engineering vector is a plasmid, cosmid, virus, phage, or another vector conventionally used in genetic engineering. In an optional specific example, the above genetic engineering vector is a plasmid. In some embodiments, the above genetic engineering vector further comprises at least one or more of the follows: origin of replication (ORI), a marker gene or fragment thereof, a reporter gene or fragment thereof, and a restriction site allowing the insertion of a DNA element. In an alternative specific example, the restriction site allowing the insertion of a DNA element is a multiple cloning site (MCS).

In some embodiments, the above genetic engineering vector is an expression vector.

In some embodiments, the genetic engineering vector comprises a promoter, a 5'-UTR, the polynucleotide encoding the signal peptide of any of the above embodiments, the polynucleotide encoding the VZV gE protein or a fragment thereof of any of the above embodiments, a 3'-UTR and a poly(A) tail, wherein the poly(A) tail, the promoter, the 5'-UTR, the polynucleotide encoding the signal peptide, the polynucleotide encoding the VZV gE protein or a fragment thereof and the 3'-UTR are operably linked to each other.

In other embodiments, the above genetic engineering vector is a cloning vector.

In some embodiments, the above genetic engineering vector comprises the nucleic acid without a 5'-cap structure.

The present disclosure also provides a method of preparing the nucleic acid of any one of the above embodiments, which comprises a step of introducing (e.g., introducing in the form of a plasmid) the genetic engineering vector of any one of the above embodiments into a host cell (e.g., E. coli) and then culturing the host cell containing the nucleic acid.

In addition, the present disclosure also provides another preparation method of the above nucleic acid, which includes a step of preparing the nucleic acid according to the nucleotide sequence of the nucleic acid of any one of the above embodiments by chemical synthesis. It can be understood that the chemical synthesis method may be a method known in the art, such as a solid phase phosphoramidite method.

It can be understood that the preparation method of the nucleic acid of any of the above embodiments is not limited to the above, and may also be other methods.

In addition, the present disclosure also provides a host cell comprising the nucleic acid of any of the above embodiments or the genetic engineering vector of any of the above embodiments.

In some embodiments, the above host cell is an isolated cell.

In some embodiments, a host cell is used to store and/or amplify the nucleic acid of any of the above embodiments.

In some embodiments, the host cell is a bacterial cell. Bacterial host cells include E. coli cells which is well known to those skilled in the art.

The host cell of the present disclosure can be prepared by transforming the genetic engineering vector of any of the above embodiments into a competent host cell. Competent host cells are cells that have the ability to uptake free extracellular genetic material (e.g., DNA plasmid) independent of sequence. A variety of bacterial cells known to those skilled in the art are naturally capable of uptaking exogenous DNA from the environment and thus can serve as bacterial host cells according to the present disclosure. In addition, it is known to those skilled in the art that competent bacterial host cells can be obtained from natural non-competent bacterial cells using, for example, electroporation or chemicals (e.g., treatment with calcium ions with high temperature exposure). Upon uptake, the exogenous DNA is preferably neither degraded nor integrated in the genome of the bacterial host cell.

In addition, the present disclosure also provides a protein or polypeptide encoded by the nucleic acid of any of the above embodiments.

In some embodiments, the protein or polypeptide encoded by the above nucleic acid comprises or is a VZV gE protein or a fragment thereof.

In addition, the present disclosure also provides a VZV immunogen comprising a VZV gE protein or a fragment thereof encoded by the nucleic acid of any of the above embodiments.

In addition, the present disclosure also provides a method for preparing a protein or polypeptide, comprising: transcribing a polynucleotide encoding the protein or polypeptide (e.g., a nucleic acid in the form of DNA of any of the above embodiments) into mRNA; and translating the mRNA obtained by the transcription into the polypeptide or protein.

In some embodiments, the method of preparing the above protein or polypeptide is performed completely or partially in vitro.

In some embodiments, the above protein or polypeptide comprises or is a VZV gE protein or a fragment thereof.

### IV. RNA and Method for Preparing RNA

The present disclosure also provides a method of preparing RNA, comprising a step of transcription using the genetic engineering vector of any of the above embodiments of the present disclosure.

In some embodiments, the above method of preparing RNA is an in vitro method. In some embodiments, the above method of preparing RNA comprises contacting the genetic engineering vector (e.g., plasmid) of any of the above embodiments with an RNA polymerase. In some embodiments, the above method of preparing RNA further comprises the step of linearizing the genetic engineering vector (e.g., plasmid). In some embodiments, prior to the linearization, the superhelical rate of the genetic engineering vector (e.g., plasmid) is at least about 85% (e.g., 85%, 86%, 87%, 88%, 89%, 90%, 91%, 92%, 93%, etc.). In some embodiments, the method of preparing RNA further comprises a step of purifying the linearized genetic engineering vector. In some embodiments, the above method of preparing RNA further comprises a step of purifying RNA.

The present disclosure further provides another method of preparing RNA, comprising a step of preparing the RNA according to the nucleotide sequence of the RNA or DNA corresponding to any of the above embodiments by chemical synthesis. It can be understood that the chemical synthesis method may be a method known in the art, such as a solid phase phosphoramidite method.

In some embodiments, the RNA is mRNA.

In some embodiments, any of the above methods of preparing RNA further comprises a step of capping and optionally purifying the capping product. In some embodiments, the cap is a Cap1 cap. The Cap1 cap structure is as follows:
cap G¹G² = m⁷G-5'-ppp-5'-Gm2'-3'-p-[m7 = 7-CH₃; m2' = 2'-O-CH₃; -ppp- = -PO₂H-O-PO₂H-O-PO₂H)-; -p- = -PO₂H-].

The capping reaction is as follows:
pppN1(p)Nx-OH(3') → ppN1(pN)x-OH(3') + Pi
ppN1(pN)x-OH(3') + GTP → G(5')ppp(5')N1(pN)x-OH(3') + PPi
G(5')ppp(5')N1(pN)x-OH(3') + AdoMet → m7G(5')ppp(5')N1(pN)x-OH(3') + AdoHyc
m7GpppN1(pN)x-OH(3') + AdoMet → m7Gppp[m2'-O]N1(pN)x-OH(3') + AdoHyc.

In other embodiments, the above method of preparing RNA is partially an in vitro method. The method of preparing RNA, at this moment, comprises the following steps: preparing the genetic engineering vector of any of the above embodiments in vitro; and introducing the genetic engineering vector into an organism (e.g., in the form of a plasmid). In some embodiments, the genetic engineering vector is encapsulated in a delivery carrier. At this moment, the genetic engineering vector can be delivered into an organism by a delivery carrier.

In some embodiments, the RNA prepared in the method of preparing RNA according to any one of the above embodiments comprises a modified nucleoside or a modified nucleotide. Correspondingly, the raw material for preparing the RNA comprises one or more modified nucleosides or nucleotides. It can be understood that the amount and type of modified nucleosides or nucleotides correspond to the RNA to be prepared.

In addition, the present disclosure further provides an RNA prepared by the method of preparing an RNA according to any one of the above embodiments. In some embodiments, the RNA is mRNA.

### V. Nucleic Acid Composition

The present disclosure also provides a nucleic acid composition comprising a first nucleic acid or a first genetic engineering vector, the first nucleic acid is the nucleic acid of any of the above embodiments, and the first genetic engineering vector is the genetic engineering vector of any of the above embodiments.

In some embodiments, the first nucleic acid is RNA.

In some embodiments, the first nucleic acid is mRNA.

In some embodiments, the nucleic acid composition comprises a first nucleic acid or a first genetic engineering vector, the first nucleic acid is the nucleic acid comprising the polynucleotide encoding the VZV gE protein or the fragment thereof of any of the above embodiments and the signal peptide, and the first genetic engineering vector is the genetic engineering vector encoding the polynucleotide encoding the VZV gE protein or a fragment thereof of any of the above embodiments and the signal peptide.

In some embodiments, the above nucleic acid composition is not limited to include the first nucleic acid or first genetic engineering vector, but also includes other nucleic acids (such as nucleic acids encoding other immunogens) and/or other substances (such as buffers or lyoprotectants).

In some embodiments, the above nucleic acid composition further comprises a second nucleic acid or a second vector, the second nucleic acid comprises a polynucleotide encoding a protein or polypeptide other than the VZV gE protein or the fragment thereof, and the second vector comprises a polynucleotide encoding a protein or polypeptide other than the VZV gE protein or the fragment thereof. The proteins or polypeptides other than the VZV gE protein or the fragment thereof are as described above, and will not be repeated here.

In some embodiments, the second nucleic acid is RNA.

In some embodiments, the second nucleic acid is mRNA.

It can be understood that the second nucleic acid contained in the above nucleic acid composition is not limited to one but also be more. For example, the nucleic acid composition can further comprise a second nucleic acid comprising a polynucleotide encoding a protein or polypeptide other than, or a fragment thereof. The above-mentioned nucleic acid composition may also comprise a plurality of second nucleic acids, which are independent of each other and are a plurality of nucleic acids comprising polynucleotides encoding proteins or polypeptides other than the VZV gE protein or the fragment thereof, for example, the above nucleic acid composition further comprises three second nucleic acids, which are independent of each other: a nucleic acid comprising a polynucleotide encoding VZV gI, a fragment, a variant, or a variant of the fragment thereof, a nucleic acid comprising a polynucleotide encoding HA of influenza virus, a fragment, a variant, or a variant of the fragment thereof, and a nucleic acid comprising a polynucleotide encoding S protein of SARS- Cov-2, a fragment, a variant, or a variant of the fragment thereof. It should be noted that "independent of each other" herein means that a plurality of second nucleic acids are not linked by a linker element, and in this case, polynucleotides encoding a plurality of other proteins or polypeptides are not present on the same nucleic acid chain. The second genetic engineering vector is also not limited to one or more, and the plurality of second genetic engineering vectors are also independent of each other.

### VI. Delivery carrier, pharmaceutical composition and use thereof

The present disclosure also provides a delivery carrier comprising the nucleic acid of any of the above embodiments, the genetic engineering vector of any of the above embodiments, the host cell of any of the above embodiments, the protein or polypeptide encoded by the nucleic acid of any of the above embodiments, the immunogen of any of the above embodiments, the RNA of any of the above embodiments, or the nucleic acid composition of any of the above embodiments.

In some embodiments, the nucleic acid in the delivery carrier is RNA. InIn some embodiments, the nucleic acid in the delivery carrier is mRNA. In

In some embodiments, the delivery carrier is selected from one or a complex of several of the follows: lipid nanoparticles (LNPs), liposomes, cationic proteins, vesicles, microparticles, polymers, and micelles. In some embodiments, the delivery carrier is selected from one of lipid nanoparticles, liposomes, cationic proteins, vesicles, microparticles, polymers, and micelles.

In some embodiments, the delivery carrier is lipid nanoparticles (LNPs) comprising the nucleic acid of any of the above embodiments, the genetic engineering vector of any of the above embodiments, the host cell of any of the above embodiments, the protein or polypeptide encoded by the nucleic acid of any of the above embodiments, the immunogen of any of the above embodiments, the RNA of any of the above embodiments, or the nucleic acid composition of any of the above embodiments.

In some embodiments, lipid nanoparticles refer to particles at the order of nanometers (e.g., 1nm-1000nm) that include one or more lipids.

In some embodiments, the lipid nanoparticles have an average diameter of 20nm~800nm, 20nm~500nm, 20nm~400nm, 20nm~300nm, 20nm~200nm, 20nm~100nm, 30nm~700nm, 30nm~500nm, 30nm~300nm, 30nm~200nm, 30nm~100nm, 40nm~800nm, 40nm~600nm, 40nm~500nm, 40nm~300nm, 40nm~200nm, 40nm~100nm, 50nm~800nm, 50nm~600nm, 50nm~500nm, 50nm~500nm, 50nm~400nm, 50nm~500nm, 50nm~400nm, 50nm~300nm, 50nm~200nm, 50nm~100nm, 60nm~800nm, 60nm~600nm, 60nm~500nm, 60nm~400nm, 60nm~300nm, 60nm~200nm or 60nm~100nm. In some alternative specific examples, the lipid nanoparticles have an average diameter of 26nm, 31nm, 36nm, 41nm, 46nm, 51nm, 56nm, 61nm, 66nm, 71nm, 76nm, 81nm, 86nm, 91nm, 96nm, 101nm, 106nm, 111nm, 116nm, 121nm, 126nm, 131nm, 136nm, 141nm, 146nm, 151nm, 156nm, 161nm, 166nm, 171nm, 176nm, 181nm, 186nm, 191nm, 196nm, 201nm, 206nm, 211nm, 216nm, 221nm, 226nm, 231nm, 236nm, 241nm, 246nm or 249nm. Herein, the average diameter of the lipid nanoparticles may be expressed as a z-average determined by dynamic light scattering.

In some embodiments, the lipid nanoparticles comprise one of the follows: cationic lipid nanoparticles, solid lipid nanoparticles (SLN), nanostructured lipid carriers (NLC), and non-lamellar lipid nanoparticles. In an optional specific example, the lipid nanoparticles are cationic lipid nanoparticles.

In some embodiments, the lipid nanoparticles contain one or more of the following substances: cationic lipids, helper lipids, structural lipids, and polymer-lipids.

The term "cationic lipid" refers to a lipid that becomes positively charged by lowering the pH below the pKa of the ionizable group of the lipid, but becomes gradually neutral at higher pH values, where the positively charged lipid is able to bind to negatively charged nucleic acids. In certain embodiments, the cationic lipid comprises a zwitterionic lipid.

In some embodiments, the cationic lipid comprises the following compound (I), N-oxide thereof, salt thereof or isomer thereof: 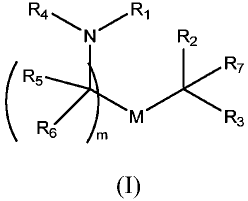 where:
R₁ is selected from the group consisting of C5-C30 alkyl, C5-C20 alkenyl, -R*YR", -YR", and -R"M'R';
R₂ and R₃ are independently selected from the group consisting of H, C1-C14 alkyl, C2-C14 alkenyl, -R*YR", -Y", and -R*OR", or R₂ and R₃ together with the atom to which they are attached form a heterocyclic or carbocyclic ring;
R₄ is selected from the group consisting of hydrogen, C₃-C₆ carbocycle, -(CH₂)ₙQ, - (CH₂)ₙCHQR, -(CH₂)ₒC(R₁₀)₂(CH₂)ₙ₋ₒQ, -CHQR, -CQ(R)₂, and unsubstituted C₁-C₆ alkyl, wherein Q is selected from carbocycle, heterocycle, -OR, -O(CH₂)ₙN(R)₂, -C(O)OR, -OC(O)R, -CX₃, - CX₂H, -CXH₂, -CN, -N(R)₂, -C(O)N(R)₂, -N(R)C(O)R, -N(R)S(O)₂R, -N(R)C(O)N(R)₂, - N(R)C(S)N(R)₂, N(R)R₈, -N(R)S(O)₂R₈, -O(CH₂)ₙOR, -N(R)C( = NR₉)N(R)₂, -N(R)C( = CHR₉)N(R)₂, -OC(O)N(R)₂, -N(R)C(O)OR, -N(OR)C(O)R, -N(OR)S(O)₂R, -N(OR)C(O)OR, - N(OR)C(O)N(R)₂, -N(OR)C(S)N(R)₂, -N(OR)C(=NR₉)N(R)₂, -N(OR)C(=CHR₉)N(R)₂, -C(= NR₉)N(R)₂, -C( = NR₉)R, -C(O)N(R)OR and -C(R)N(R)₂C(O)OR, each o is independently selected from 1, 2, 3 and 4, and each n is independently selected from 1, 2, 3, 4 and 5;
each R₅ is independently selected from the group consisting of OH, C1-C3 alkyl, C2-C3 alkenyl, and H;
each R₆ is independently selected from the group consisting of OH, C1-C3 alkyl, C2-C3 alkenyl, and H;
M and M' are independently selected from the group consisting of -C(O)O-, -OC(O)-, -OC(O)-M"-C(O)O-, -C(O)N(R')-, -N(R')C(O)-, -C(O)-, -C(S)-, -C(S)S-, -SC(S)-, -CH(OH)-, - P(O)(OR')O-, -S(O)₂-, -S-S-, aryl groups, and heteroaryl groups, wherein M" is a bond, C₁-C₁₃ alkyl, or C₂₋-C₁₃ alkenyl;
R₇ is selected from the group consisting of C1-C3 alkyl, C2-C3 alkenyl, and H;
R₈ is selected from the group consisting of C3-C6 carbocycle and heterocycle;
R₉ is selected from the group consisting of H, CN, NO₂, C₁-C₆ alkyl, -OR, -S(O)₂R, - S(O)₂N(R)₂, C2-C6 alkenyl, C3-C6 carbocycle, and heterocycle;
R₁₀ is selected from the group consisting of H, C1-C3 alkyl, and C2-C3 alkenyl;
each R is independently selected from the group consisting of C1-C3 alkyl, C2-C3 alkenyl, (CH₂)_{q}OR*, and H,
and each q is independently selected from 1, 2 and 3;
each R' is independently selected from the group consisting of C1-C18 alkyl, C2-C18 alkenyl, -R*YR", -YR", H and 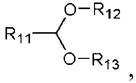 and R₁₁, R₁₂ and R₁₃ are each independently selected from the group consisting of C1-C12 alkyl and C2-C12 alkenyl;
each R" is independently selected from the group consisting of C3-C15 alkyl and C3-C15 alkenyl;
each R* is independently selected from the group consisting of absent, C1-C12 alkyl, and C2-C12 alkenyl;
each Y is independently a C3-C6 carbocycle;
each X is independently selected from the group consisting of F, Cl, Br, and I; and
m is selected from 5, 6, 7, 8, 9, 10, 11, 12 and 13; and wherein when R₄ is -(CH₂)ₙQ, - (CH₂)ₙCHQR, -CHQR, or -CQ(R)₂, then (i) when n is 1, 2, 3, 4 or 5, Q is not -N(R)₂; or (ii) when n is 1 or 2, Q is not 5, 6 or 7-membered heterocycloalkyl.

In an optional specific example, the cationic lipid is the following compound (I), N-oxide thereof, salt thereof or isomer thereof: 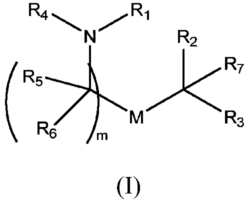 R₁-R₇, M and m are as defined above.

In some embodiments, the cationic lipid comprises the following compound (II), N-oxide thereof, salt thereof or isomer thereof: 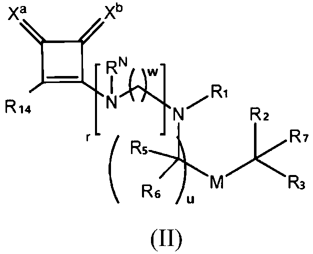 where:
R₁, R₂, R₃, R₅, R₆, M and R₇ are as described above,
R^{N} is H or C1-C3 alkyl;
X^{a} and X^{b} are each independently O or S;
R₁₄ is selected from the group consisting of H, halogen,-OH, R^{b}, -N(R^{b})₂, -CN, -N₃, -C(O)OH, -C(O)OR^{b}, -OC(O)R^{b}, -OR^{b}, -SR^{b}, -S(O)R^{b}, -S(O)OR^{b}, -S(O)₂OR^{b}, -NO₂, -S(O)₂N(R^{b})₂, - N(R^{d})S(O)₂R^{b}, -NH(CH₂)ₜ₁N(R^{b})₂, -NH(CH₂)ₚ₁O(CH₂)_{q1}N(R^{b})₂, -NH(CH₂)ₛ₁OR^{b}, -N((CH₂)_{S}OR^{b})₂, -N(R^{b})-carbocycle, -N(R^{b})-heterocycle, N(R^{b})-aryl, -N(R^{b})-heteroaryl, -N(R^{b})(CH₂)ₜ-carbocycle, - N(R^{b})(CH₂)ₜ₁-heterocycle, -N(R^{b})(CH₂)ₜ₁-aryl, -N(R^{b})(CH₂)ₜ₁-heteroaryl, carbocycle, heterocycle, aryl and heteroaryl;
each R^{b} is independently selected from the group consisting of C1-C3 alkyl, C2-C3 alkenyl, and H;
u is 5, 6, 7, 8, 9, 10, 11, 12 or 13;
w is 1, 2, 3, 4, 5, 6, 7, 8, 9 or 10;
r is 0 or 1;
t¹ is 1, 2, 3, 4 or 5;
p¹ is 1, 2, 5, 4 or 5;
q¹ is 1, 2, 5, 4 or 5; and
s¹ is 1, 2, 3, 4 or 5.

In an optional specific example, the cationic lipid is the following compound (II), its N-oxide, its salt or its isomer: 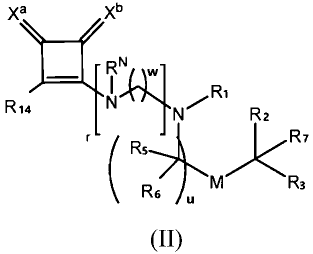 where R₁-R₃, R₅-R₇, R₁₄, X^{a}, X^{b}, R^{N}, M, u, w and r are as defined above.

In some embodiments, the cationic lipid comprises the following compound (III), N-oxide thereof, salt thereof or isomer thereof: 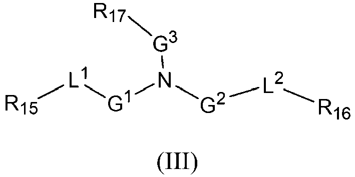 where:
one of L¹ or L² is -O(C=O)-, -(C=O)O-, -C(=O)-, -O-, -S(O)ₓ-, -S-S-, -C(=O)S-, -SC(= O)-, -NR^{a}C(=O)-, -C(=O)NR^{a}-, NR^{a}C(=O)NR^{a}-, -OC(=O)NR^{a}- or -NR^{a}C(=O)O-, and the other of L¹ or L² is -O(C=O)-, -(C=O)O-, -C(=O)-, -O-, -S(O)ₓ-, -S-S-, -C(=O)S-, SC(=O)-, - NR^{a}C(=O)-, -C(=O)NR^{a}-, NR^{a}C(=O)NR^{a}-, -OC(=O)NR^{a}- or -NR^{a}C(=O)O-or a bond;
G¹ and G² are each independently unsubstituted C1-C12 alkylene or C1-C12 alkenylene;
G³ is C1-C24 alkylene, C1-C24 alkenylene, C3-C8 cycloalkylene, C3-C8 cycloalkenylene;
R^{a} is H or C1-C12 hydrocarbyl;
R₁₅ and R₁₆ are each independently C6-C24 alkyl or C6-C24 alkenyl;
R₁₇ is H, OR₁₈, CN, -C(=O)OR₁₉, -OC(=O)R₁₉ or -NR₁₈C(=O)R₁₉;
R₁₉ is C1-C12 hydrocarbyl;
R₁₈ is H or C1-C6 hydrocarbyl; and
x is 0, 1 or 2.

In an optional specific example, the cationic lipid is the following compound (III), its N-oxide, its salt or its isomer: 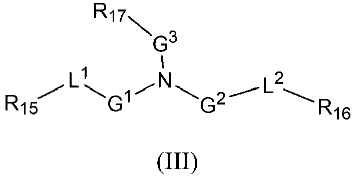 R₁₅-R₁₇, G¹-G³, L¹-L² are as defined above.

In some embodiments, the cationic lipid comprises the following compound (IV), or a pharmaceutically acceptable salt or stereoisomer thereof: 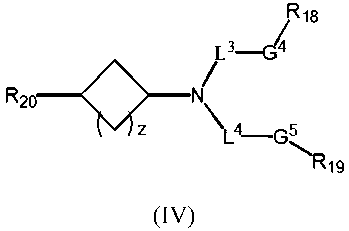 where:
L³ and L⁴ are the same or different, and are each independently C1-C12 alkylene, C2-C12 alkenylene or C2-C12 alkynylene; in some embodiments, L³ and L⁴ are the same or different, and are each independently C3-C10 alkylene, C3-C10 alkenylene or C3-C10 alkynylene; in some embodiments, L³ and L⁴ are the same or different, and are each independently C3-C10 alkylene; in some embodiments, L³ and L⁴ are the same or different, and are each independently C5-C8 alkylene;
G⁴ and G⁵ are the same or different, and are each independently -O-(C=O)-, -(C=O)-O-, - C(=O)-, -O-, -C(=O)-S-or-S-C(=O)-; in some embodiments, G⁴ and G⁵ are the same or different, and are each independently -O-(C=O)-, -(C=O)-O-, -C(=O)- or -O-; in some embodiments, G⁴ and G⁵ are the same or different, and are each independently selected from -O- (C=O)- or - (C=O)-O-;
R₁₈ and R₁₉ are the same or different, and are each independently C5-C27 alkyl or C5-C27 alkenyl containing one or more double bonds; in some embodiments, R₁₈ and R₁₉ are the same or different, and are each independently C8-C20 alkyl or C8-C20 alkenyl containing one or more double bonds; in some embodiments, R₁₈ and R₁₉ are the same or different, and are each independently C9-C17 alkyl or C9-C18 alkenyl containing one or two double bonds; in some embodiments, R₁₈ and R₁₉ are the same or different, and are each independently C9-C17 alkyl; 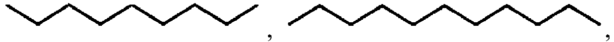 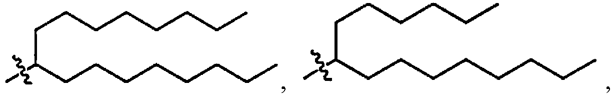 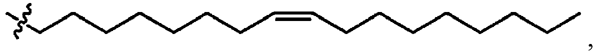 or 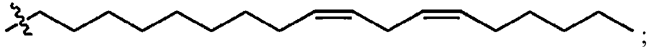
R₂₀ is halogen, hydroxyl, cyano, C1-C6 alkyl, nitro, C1-C6 alkoxy, C1-C6 alkylcarbonyloxy, C1-C6 alkoxycarbonyl, C1-C6 alkylaminocarbonyl or C1-C6 alkylcarbonylamino; in some embodiments, R₂₀ is halogen, hydroxyl, cyano, C1-C6 alkoxy, C1-C6 alkylcarbonyloxy, C1-C6 alkoxycarbonyl, C1-C6 alkylaminocarbonyl or C1-C6 alkylcarbonylamino; in some embodiments, R₂₀ is halogen, hydroxyl, cyano, C1-C4 alkoxy, C1-C4 alkylcarbonyloxy, C1-C4 alkoxycarbonyl, C1-C4 alkylaminocarbonyl or C1-C4 alkylcarbonylamino; in some embodiments, R₂₀ is fluorine, hydroxyl, cyano, methoxy, acetoxy, methoxycarbonyl, butylaminocarbonyl or acetamido;
z is 1, 2 or 3.

In some embodiments, the cationic lipid is the following compound (IV), or a pharmaceutically acceptable salt or stereoisomer thereof:

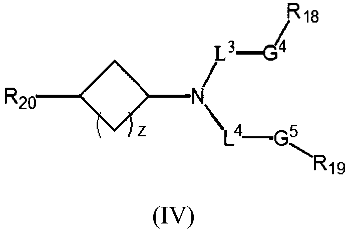

R₁₈-R₂₀, G⁴-G⁵, L³-L⁴ and z are as defined above.

In some embodiments, the cationic lipid comprises the following compound (IV-1), or a pharmaceutically acceptable salt or stereoisomer thereof:

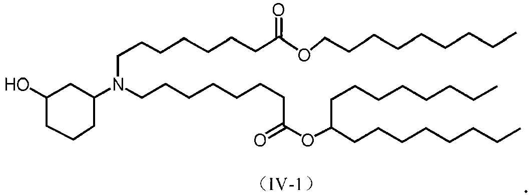

In some embodiments, the cationic lipid comprises or is the following compounds, or pharmaceutically acceptable salts thereof: 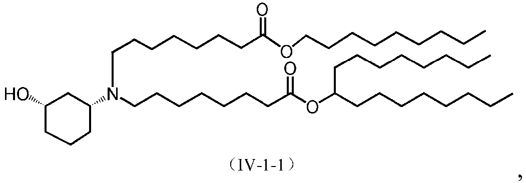 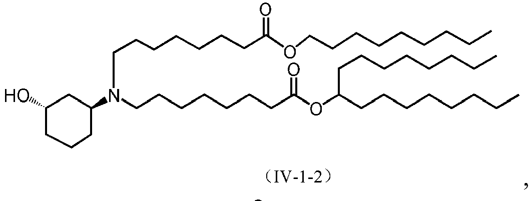 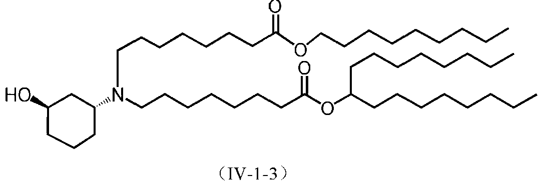 or

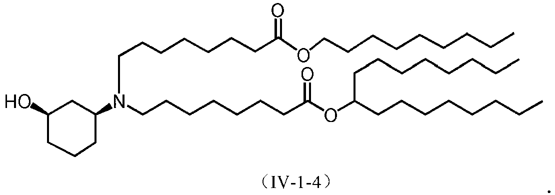

In some embodiments, the cationic lipid comprises one or more of ALC-0315 (CAS No. 2036272-55-4), SM-102 (CAS No. 2089251-47-6), and

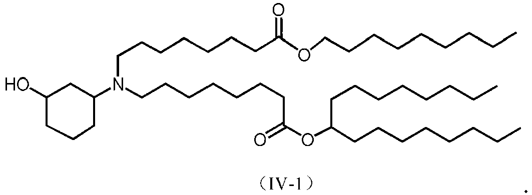

In some embodiments, the auxiliary lipid of the lipid nanoparticle comprises a phospholipid. A phospholipids is generally semi-synthetic and may be naturally derived or chemically modified. In an optional specific example, the auxiliary lipid of the lipid nanoparticle is a phospholipid substance. In some embodiments, the phospholipids of the lipid nanoparticles include one or more of the following: DSPC (distearoyl phosphatidylcholine), DOPE (dioleoyl phosphatidylethanolamine), DOPC (dioleoyl phosphatidylcholine), DOPS (dioleoyl phosphatidylserine), DSPG (1,2-dioctadecanoyl-sn-glycero-3-phospho-(1'-rac-glycerol)), DPPG (dipalmitoyl phosphatidylglycerol), DPPC (dipalmitoyl phosphatidylcholine), DGTS (1,2-dipalmitoyl-sn-glycero-3-O-4'-(N,N,N-trimethyl) homoserine), and lysophospholipids. In some embodiments, the auxiliary lipid of the lipid nanoparticle is one or more selected from the group consisting of DSPC, DOPE, DOPC, and DOPS. In some embodiments, the auxiliary lipid of the lipid nanoparticle is DSPC and/or DOPE.

In some embodiments, the structural lipid of the lipid nanoparticle includes a sterol. In an optional specific example, the structural lipid of the lipid nanoparticle is a sterol. In some embodiments, the sterol of the lipid nanoparticle includes one or more of the follows: 20α-hydroxycholesterol, cholesterol, cholesterol esters, sterols hormones, sterols vitamins, bile acids, cholesterol, ergosterol, β-sitosterol, and oxidized cholesterol derivatives. In some embodiments, the structural lipid of the lipid nanoparticle comprises at least one of cholesterol, cholesterol ester, sterol hormone, sterol vitamin, and bile acid. In some embodiments, the structural lipid of the lipid nanoparticles is cholesterol. In an alternative specific example, the structural lipid of the lipid nanoparticles is high purity cholesterol, in particular injection-grade high purity cholesterol, such as CHO-HP (produced by AVT). In other embodiments, the structural lipid is 20α-hydroxycholesterol.

A polymer-lipid refers to a conjugate comprising a polymer and a lipid coupled to the polymer. The polymer-lipid (e.g., polyethylene glycol-lipid) in the lipid nanoparticles can improve the stability of the lipid nanoparticles in vivo.

In some embodiments, the lipids of the polymer-lipid used to form the lipid nanoparticles include one or more of 1,2-dimyristoyl-sn-glycerol (DMG), distearoyl-phosphatidyl-ethanolamine (DSPE), diacylglycerol (DAG), dialkyloxypropyl (DAA), phospholipids, ceramide (Cer), 1,2-distearoyl-rac-glycerol (DSG), and 1,2-dipalmitoyl-rac-glycero (DPG).

In some embodiments, the polymer-lipid polymer used to form the lipid nanoparticles includes one or both of a hydrophilic polymer and a zwitterionic polymer.

In some embodiments, the polymer-lipid polymer used to form the lipid nanoparticles is a hydrophilic polymer. In other embodiments, the polymer-lipid polymer used to form the lipid nanoparticles is a zwitterionic polymer.

In some embodiments, the hydrophilic polymer includes one or more of the following: polyethylene glycol (PEG), poly (oxazolines) (POX), poly (glycerols) (PGs), poly (hydroxypropyl methacrylate) (PHPMA), poly (2-hydroxy ethyl methacrylate) (PHEMA), poly (N- (2-hydroxy propyl) methacrylamide) (HPMA), poly (vinylpyrrolidone) (PVP), poly(N,N-dimethyl acrylamide) (PDMA), poly(N-acryloyl morpholine) (PAcM), polyaminoacids, glycosaminoglycans (GAGs), heparin, hyaluronic acid (HA), polysialic acid (PSA), elastin-like polypeptides (ELPs), and serum albumin and CD47.

Correspondingly, polymer-lipids include one or more of the follows: polyethylene glycol-lipids (PEG-lipids), polyoxazoline-lipids, polyglycerol-lipids, polyhydroxypropyl methacrylate-lipids, poly(2-hydroxyethyl methacrylate)-lipids, poly(N-(2-hydroxypropyl) methacrylamide)-lipids, polyvinylpyrrolidone-lipids, poly(N,N-dimethylacrylamide)-lipids, poly(N-acryloylmorpholine)-lipids, glycosaminoglycan-lipids, heparin-lipids, hyaluronic acid-lipids, polysialyl-lipids, elastidin-like lipids, serum albumin-lipids, and CD47-lipids. It should be noted that "PEG-lipid" is a conjugate of polyethylene glycol and lipid, "polyoxazoline-lipid" refers to a conjugate formed by coupling polyoxazoline with lipid, "polyglycerol-lipid" refers to a conjugate formed by coupling polyglycerol with lipid, and the same applies to other polymer-lipids. In an optional specific example, the hydrophilic polymer comprises polyethylene glycol.

In some embodiments, the polymer-lipid comprises a PEG-lipid. In an alternative specific example, the polymer-lipid is a PEG-lipid. In some embodiments, PEG-lipids include one or more of PEG-myristoyl diglyceride (PEG-DMG or DMG-PEG), PEG-distearoyl phosphatidylethanolamine (PEG-DSPE), PEG-diacylglycerol (PEG-DAG), PEG-dialkyloxypropyl (PEG-DAA), PEG-phospholipids, PEG-ceramide (PEG-Cer), PEG-1,2-distearoyl-rac-glycerol (PEG-DSG), and PEG-1,2-dipalmitoyl-rac-glycerol (PEG-DPG). The PEG-lipid is preferably PEG-DMG, PEG-DSG, or PEG-DPG. PEG-DMG is a polyethylene glycol derivative of glycerol 1,2-dimyristate. In some embodiments, the average molecular weight of the PEG in the PEG-lipid is about 2000-5000. In an alternative specific example, the average molecular weight of PEG in PEG-lipid is about 2000. In some embodiments, the zwitterionic polymer includes one or more of poly(carboxybetaine) (pCB), poly(sulfobetaine) (pSB), phosphobetaine-base polymers, and phosphorylcholine polymers. In some embodiments, the zwitterionic polymer includes one or more of the following: polycarboxybetaine acrylamide , (pCBAA), poly(carboxybetaine methacrylate), poly(sulfobetaine methacrylate), poly(methacryloyloxyethyl phosphorylcholine), poly(vinyl-pyridinio propanesulfonate), poly(carboxybetaine) based on vinylimidazole, poly(sulfobetaine) based on vinylimidazole, poly(sulfobetaine) based on vinylpyridine.

Correspondingly, the polymer-lipid comprises one or more of the following: polyhydroxybetaine-lipid, polysulfobetaine-lipid, phosphobetaine-based polymer-lipid, and phosphocholine polymer-lipid. In some embodiments, the polymer-lipid comprises one or more of poly (carboxybetaine acrylamide)-lipid, poly (carboxybetaine methacrylate)-lipid, poly (sulfobetaine methacrylate)-lipid, poly (methacryloxyethyl phosphorylcholine)-lipid, poly (vinyl pyridyl propanesulfonate)-lipid, polyvinylimidazolyl betaine-lipid, polyvinylimidazolyl sulfobetaine-lipid, polyvinypyridyl sulfobetaine-lipid.

In addition, in some embodiments, the polymers applied to nanoparticles in Hoang Thi, Thai Thanh, et al., "The Importance of Poly (ethylene glycol) Alternatives for Overcoming PEG Immunogenicity in Drug Delivery and Bioconjugate." Polymers vol. 12,2298, are also incorporated herein.

In some embodiments, the lipid nanoparticle contains a cationic lipid, a helper lipid, a structural lipid, and a polymer-lipid. In some embodiments, the lipid nanoparticles comprise about 25.0%~75.0%, such as about 25.0%~28.0%, 28.0%~32.0%, 32.0%~35.0%, 35.0%~40.0%, 4.0% to 42.0%, 42.0% to 45.0%, 45.0% to 46.3%, 46.3% to 48.0%, 48.0% to 49.5%, 49.5% to 50.0%, 50.0% to 55.0%, 55.0% to 60.0%, 60.0% to 65.0%, or 65.0% to 75.0%, based on the total amount of cationic lipid, auxiliary lipid, structural lipid, and polymer-lipid.

In some embodiments, the lipid nanoparticle comprises a cationic lipid, a helper lipid, a structural lipid, and a polymer-lipid, the cationic lipid accounts for 25 mol% to 75 mol% of the total lipids present in the lipid nanoparticle, the helper lipid accounts for 0 mol% to 45 mol% of the total lipids present in the lipid nanoparticle, the structural lipid accounts for 0 mol% to 60 mol% of the total lipids present in the lipid nanoparticle, and the polymer-lipid accounts for 0.5 mol% to 5 mol% of the total lipids present in the lipid nanoparticle.

In some embodiments, the cationic lipid in the lipid nanoparticle accounts for 25 mol% to 75 mol% of the total lipid present in the lipid nanoparticle. For example, 25mol%, 28mol%, 30mol%, 31mol%, 32mol%, 33mol%, 34mol%, 35mol%, 36mol%, 37mol%, 38mol%, 39mol%, 40mol%, 41mol%, 42mol%, 43mol%, 44mol%, 45mol%, 45.5mol%, 46mol%, 46.5mol%, 47mol%, 47.5mol%, 48mol%, 48.5mol%, 49mol%, 49.5mol%, 50mol%, 50.5mol%, 51mol%, 52.5mol%, 53mol%, 53.5mol%, 54mol%, 54.5mol%, 55mol%, 55.5mol%, 56mol%, 56.5mol%, 57mol%, 57.5mol%, 58mol%, 58.5mol%, 59mol%, 59.5mol%, 60mol%, 60.5mol%, 61mol%, 61.5mol%, 62mol%, 62.5mol%, 63mol%, 63.5mol%, 64mol%, 64.5mol%, 65mol%, 68mol%, 70mol% or 75mol%. In some embodiments, the cationic lipid in the above lipid nanoparticle accounts for 30 mol% to 65 mol%, 30 mol% to 60 mol%, 35 mol% to 60 mol%, 40 mol% to 60 mol%, 45 mol% to 55 mol%, or 50 mol% to 55 mol% of the total lipid present in the lipid nanoparticle.

In some embodiments, the auxiliary lipid (e.g., DSPC) in the above lipid nanoparticle accounts for 0 mol% to 45 mol% of the total lipid present in the lipid nanoparticles. For example, 0.5 mol%, 1 mol%, 3 mol%, 5 mol%, 7.5 mol%, 8 mol%, 8.5 mol%, 9 mol%, 9.5 mol%, 10 mol%, 10.5 mol%, 11 mol%, 11.5 mol%, 12 mol%, 12.5 mol%, 13 mol%, 13.5 mol%, 14 mol%, 14.5 mol%, 15 mol%, 15.5 mol%, 16 mol%, 16.5 mol%, 17 mol%, 17.5 mol%, 18 mol%, 18.5 mol%, 19 mol%, 19.5 mol%, 20 mol%, 20.5 mol%, 21 mol%, 21.5 mol%, 22 mol%, 22.5 mol%, 23 mol%, 23.5 mol%, 24 mol%, 24.5 mol%, 25 mol%, 25.5 mol%, 26 mol%, 26.5 mol%, 27 mol%, 27.5 mol%, 28 mol%, 28.5 mol%, 29 mol%, 29.5 mol%, 30 mol%, 34 mol%, 35 mol%, 36 mol%, 38 mol%, 40 mol%, 42 mol%, 44 mol% or 45 mol%. In some embodiments, the auxiliary lipid (e.g., DSPC) in the above lipid nanoparticle accounts for 1 mol% to 40 mol%, 5 mol% to 40 mol%, 5 mol% to 35 mol%, 5 mol% to 30 mol%, or 5 mol% to 25 mol% of the total lipid present in the lipid nanoparticle.

In some embodiments, the structural lipid (e.g., cholesterol) in the above lipid nanoparticle accounts for 0 mol% to 60 mol% of the total lipid present in the lipid nanoparticle. For example, 0mol%, 1mol%, 5mol%, 8mol%, 10mol%, 12mol%, 14mol%, 15mol%, 16mol%, 18mol%, 20mol%, 22mol%, 24mol%, 25mol%, 27mol%, 27.5mol%, 28mol%, 28.5mol%, 29mol%, 29.5mol%, 30mol%, 30.5mol%, 31mol%, 31.5mol%, 32mol%, 32.5mol%, 33mol%, 33.5mol%, 34mol%, 34.5mol%, 35mol%, 35.5mol%, 36mol%, 36.5mol%, 37mol%, 37.5mol%, 38mol%, 38.5mol%, 39mol%, 39.5mol%, 40mol%, 40.5mol%, 41mol%, 41.5mol%, 42mol%, 42.5mol%, 43mol%, 44mol%, 45mol%, 46mol%, 47mol%, 48mol%, 49mol%, 50mol%, 51mol%, 52mol%, 53mol%, 54mol%, 55mol%, 56mol%, 57mol%, 58mol%, 59mol% or 60mol%. In some embodiments, the structural lipid (e.g., cholesterol) in the above lipid nanoparticle accounts for 1 mol% to 60 mol%, 1 mol% to 55 mol%, 5 mol% to 55 mol%, 10 mol% to 50 mol%, 15 mol% to 50 mol%, 15 mol% to 45 mol%, 20 mol% to 45 mol%, and 25 mol% to 40 mol% of the total lipid present in the lipid nanoparticle.

In some embodiments, the polymer-lipid (e.g., PEG lipid) in the above lipid nanoparticle accounts for 0.5 mol% to 5 mol% of the total lipid present in the lipid nanoparticle. For example, 0.5 mol%, 1 mol%, 1.5 mol%, 2 mol%, 2.5 mol%, 3 mol%, 3.5 mol%, 4 mol%, 4.5 mol% or 5 mol%. In some embodiments, the polymer-lipid (e.g., PEG lipid) in the above lipid nanoparticle accounts for 0.5 mol% to 4.5 mol%, 1 mol% to 4.5 mol%, 1 mol% to 4 mol%, 1.5 mol% to 4 mol%, 1.5 mol% to 3.5 mol%, or 1.5 mol% to 3 mol% of the total lipid present in the lipid nanoparticle.

In some embodiments, the non-lamellar lipid nanoparticles are selected from one of: alcoholic liposomes and echogenic liposomes.

In some embodiments, the delivery carrier is a liposome comprising the nucleic acid of any of the above embodiments, the genetic engineering vector of any of the above embodiments, the host cell of any of the above embodiments, the protein or polypeptide encoded by the nucleic acid of any of the above embodiments, the immunogen of any of the above embodiments, the RNA of any of the above embodiments, or the nucleic acid composition of any of the above embodiments. The liposome encapsulates the nucleic acid, the genetic engineering vector, the host cell, the protein or polypeptide encoded by the nucleic acid, the immunogen, the RNA, or the nucleic acid composition of any one of the above embodiments with the vesicle formed by the phospholipid bilayer membrane. In some embodiments, the composition of the liposome comprises a phospholipid and cholesterol.

In some embodiments, the delivery carrier is a cationic protein loaded with a nucleic acid comprising any one of the above embodiments, a genetic engineering vector of any one of the above embodiments, a host cell of any one of the above embodiments, a protein or polypeptide encoded by the nucleic acid of any one of the above embodiments, an immunogen of any one of the above embodiments, an RNA of any one of the above embodiments, or a nucleic acid composition of any one of the above embodiments. In some embodiments, the cationic protein includes, but is not limited to, protamine.

In some embodiments, the delivery carrier is a polymer comprising the nucleic acid of any of the above embodiments, the genetic engineering vector of any of the above embodiments, the host cell of any of the above embodiments, the protein or polypeptide encoded by the nucleic acid of any of the above embodiments, the immunogen of any of the above embodiments, the RNA of any of the above embodiments, or the nucleic acid composition of any of the above embodiments. In some embodiments, the polymer is a lipopolyplex (LPP) and/or hyaluronic acid polymer (e.g., hyaluronic acid gel) comprising the nucleic acid, genetic engineering vector, protein or polypeptide, immunogen, RNA, or nucleic acid composition of any of the above embodiments. In an optional specific example, the polymer is a lipopolyplex or a hyaluronic acid gel. A lipopolyplex is a bilayer structure a polymer-encapsulated nucleic acid (e.g., mRNA) as the core and a lipid (e.g., phospholipid) encapsulated as the shell.

It can be understood that the delivery carrier suitable for the present disclosure is not limited to the above, and may also be other substances capable of transporting the nucleic acid of any of the above embodiments, the genetic engineering vector of any of the above embodiments, the host cell of any of the above embodiments, the protein or polypeptide encoded by the nucleic acid of any of the above embodiments, the immunogen of any of the above embodiments, the RNA of any of the above embodiments, or the nucleic acid composition of any of the above embodiments into an organism. For example, vesicles (e.g., exosomes) and the like.

In addition, the present disclosure further provides a preparation method for the above compound (IV), and the reaction of the preparation method is as follows: 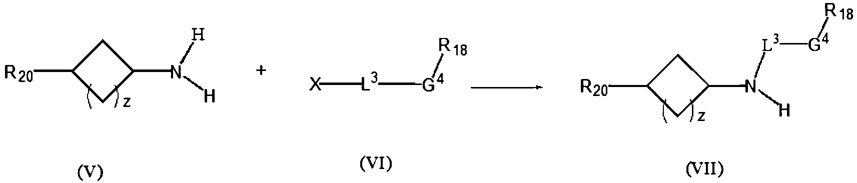 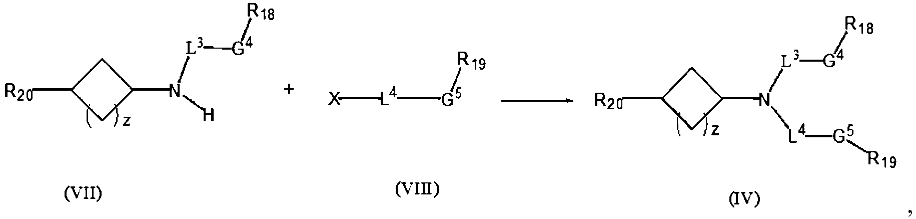 where, R₁₈~R₂₀, G⁴-G⁵, L³-L⁴ and z are as defined above, and X is halogen, preferably bromine.

The preparation method includes step S11 and step S12.

Step S11: subjecting the intermediate compound (V) and the intermediate compound (VI) to a substitution reaction at room temperature (16° C. to 30° C., the same applies below) in an organic solvent in the presence of an acid-binding agent to obtain an intermediate compound (VII). The organic solvent in step S1 is selected from one or more of nitrile organic solvents, alcohol organic solvents, halogenated hydrocarbon organic solvents, amide organic solvents, and aromatic hydrocarbon organic solvents, for example, the organic solvent in step S1 is selected from one or more of acetonitrile, methanol, ethanol, dichloromethane, and dichloroethane (DCE). The acid-binding agent in step S1 is selected from one or more of an organic base and an inorganic base. For example, the acid-binding agent in step S1 is selected from one or more of sodium hydroxide, potassium hydroxide, sodium carbonate, potassium carbonate, triethylamine, and DIPEA.

Step S12: subjecting the intermediate compound (VII) and the intermediate compound (VIII) to a substitution reaction at room temperature in an organic solvent in the presence or absence of an acid-binding agent and an iodide to obtain the compound of formula (IV). The organic solvent in step S2 is selected from one or more of nitrile organic solvents, alcohol organic solvents, halogenated hydrocarbon organic solvents, amide organic solvents and aromatic hydrocarbon organic solvents. For example, the organic solvent in step S2 is selected from one or more of acetonitrile, methanol, ethanol, dichloromethane and dichloroethane. The acid-binding agent is selected from one or more of organic bases and inorganic bases. For example, the acid-binding agent is selected from one or more of sodium hydroxide, potassium hydroxide, sodium carbonate, potassium carbonate, triethylamine and DIPEA. Iodides are common iodides, such as potassium iodide.

In addition, the present disclosure also provides a preparation method for the optical isomer (IV-1- i) of the above compound (IV-1), and the reaction of the preparation method is as follows: 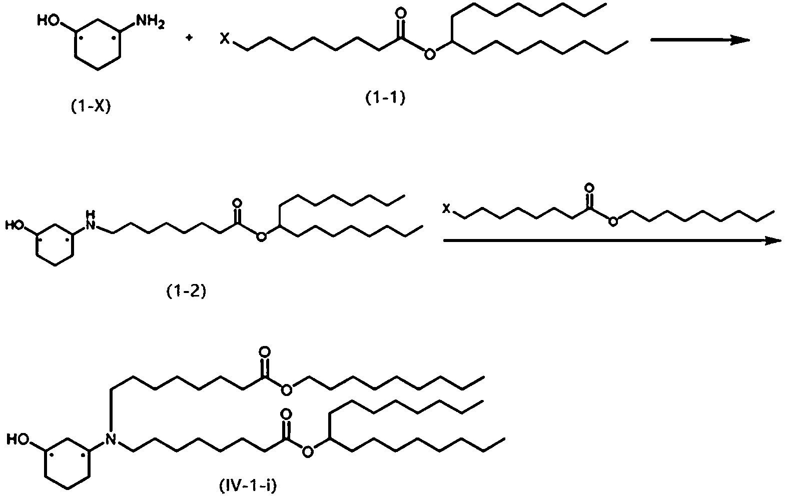 where X is halogen, preferably bromine.

Specifically, the preparation steps for the optical isomer (IV-1- i) of compound (IV-1) of the present disclosure include step S21 and step S22.

Step S21: subjecting compound (1-X) and compound (1-1) to an N-alkylation reaction at 30° C to 50° C in a solvent (such as nitrile, alcohol, halogenated hydrocarbon, amide, aromatic hydrocarbon solvent, specifically acetonitrile, methanol, ethanol, dichloromethane, dichloroethane (DCE), etc.) to prepare compound (1-2);

Step S22: subjecting the compound (1-2) and nonyl 8-halooctanoate to an N-alkylation reaction at 60° C to 110° C in a solvent (such as nitriles, alcohols, halogenated hydrocarbons, amides, aromatic hydrocarbons, ether solvents, specifically acetonitrile, methanol, ethanol, dichloromethane, dichloroethane (DCE), cyclopentylether, methyl tert-butyl ether, etc.), in the presence or absence of an acid binding agent and a catalyst to prepare the compound (IV-1-i), wherein the acid binding agent is selected from one or more of organic bases and inorganic bases. For example, one or more selected from the group consisting of sodium hydroxide, potassium hydroxide, sodium carbonate, potassium carbonate, triethylamine and DIPEA; the catalyst is an iodide, preferably KI.

In some embodiments, according to the above method, when the compound (1-X) as used is (1S,3R)-3-aminocyclohexanol, the compound (1-2) is as shown in structural formula (1-2-1): 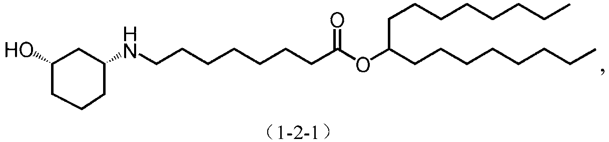 the optical isomer compound (IV-1-1) of the compound (IV-1) was obtained:

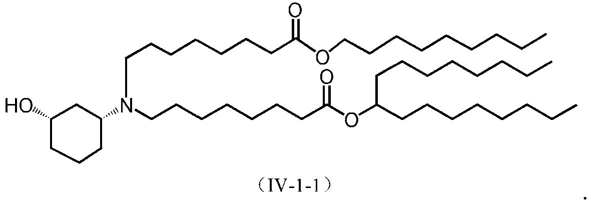

When the compound (1-X) is (1S,3S)-3-aminocyclohexanol, and the compound (1-2) is shown in the structural formula (1-2-2): 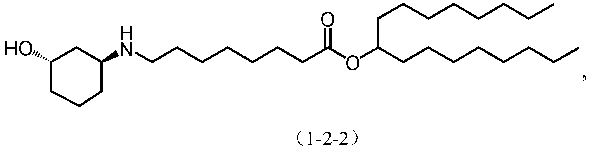 the optical isomer compound (IV-1-2) of the compound (IV-1) was obtained:

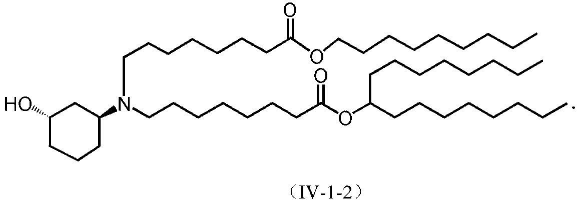

When the compound (1-X) is (1R,3R)-3-aminocyclohexanol, and the compound (1-2) is shown in the structural formula (1-2-3): 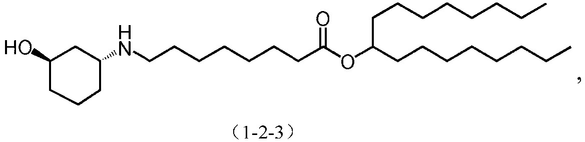 the optical isomer compound (IV-1-3) of the compound (IV-1) was obtained:

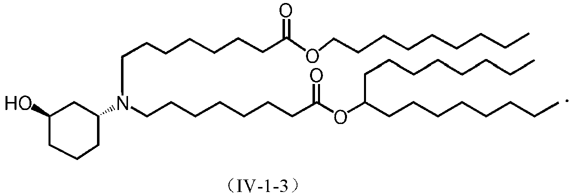

When the compound (1-X) is (1R,3S)-3-aminocyclohexanol, and the compound (1-2) is shown in the structural formula (1-2-4): 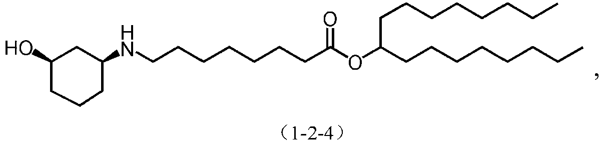 the optical isomer compound (IV-1-4) of the compound (IV-1) was obtained:

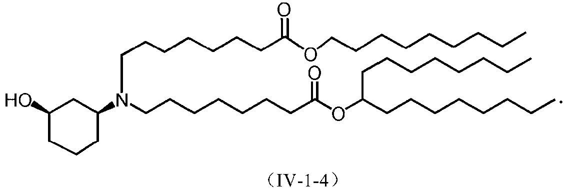

In addition, the present disclosure also provides a pharmaceutical composition comprising the nucleic acid of any of the above embodiments, the genetic engineering vector of any of the above embodiments, the host cell of any of the above embodiments, the nucleic acid composition of any of the above embodiments, the immunogen of any of the above embodiments, the protein or polypeptide encoded by the nucleic acid of any of the above embodiments, the RNA of any of the above embodiments, or the delivery carrier of any of the above embodiments, and a pharmaceutically acceptable carrier.

In some embodiments, the pharmaceutical composition comprises a plurality of delivery carriers, and the proteins or polypeptides encoded by the nucleic acids encoded contained in the plurality of delivery carriers are different. For example, the plurality of delivery carriers are two delivery carriers, the first delivery carrier is a delivery carrier comprising a nucleic acid having a polynucleotide encoding a VZV gE protein or a fragment thereof and a signal peptide, and the second delivery carrier is a delivery carrier comprising a nucleic acid of a polynucleotide encoding a signal peptide and a VZV gI. For another example, the plurality of delivery carriers are three delivery carriers, the first delivery carrier is a delivery carrier comprising a nucleic acid having a polynucleotide encoding a VZV gE protein or a fragment thereof and a signal peptide, the second delivery carrier is a delivery carrier comprising a nucleic acid of a polynucleotide encoding a signal peptide and a VZV gI, and the third delivery carrier is a delivery carrier comprising a nucleic acid of a polynucleotide encoding a signal peptide and an HA protein of an influenza virus.

In some embodiments, the above pharmaceutical composition comprises a plurality of nucleic acids of any of the above embodiments, a plurality of genetic engineering vectors of any of the above embodiments, a plurality of proteins or polypeptides of any of the above embodiments, a plurality of immunogens of any of the above embodiments, a plurality of RNAs of any of the above embodiments, or a plurality of host cells of any of the above embodiments.

As used herein, the term "pharmaceutically acceptable" means approved for use in animals and/or human by a regulatory agency, such as the China Food and Drug Administration (CFDA), the United States Food and Drug Administration (FDA)), or a recognized pharmacopoeia, such as the Chinese pharmacopoeia, European Pharmacopeia. The term "pharmaceutically acceptable carrier" refers to a substance that can be administered with the nucleic acid, genetic engineering vector, nucleic acid composition, host cell, protein or polypeptide or delivery carrier of the present disclosure, including but not limited to diluents, sweeteners, flavoring agents, wetting agents, adjuvants, glidants, preservatives, dyes/colorants, surfactants, dispersants, suspending agents, stabilizers, isotonic agents, solvents or emulsifiers.

In some embodiments, the above pharmaceutical composition does not comprise an adjuvant.

In some embodiments, the above pharmaceutical composition further comprises an adjuvant.

In some embodiments, the adjuvant comprises a flagellin adjuvant. In some embodiments, the flagellin adjuvant is mRNA encoding flagellin.

In addition, the present disclosure also provides use of the nucleic acid of any of the above embodiments, the genetic engineering vector of any of the above embodiments, the host cell of any of the above embodiments, the nucleic acid composition of any of the above embodiments, the protein or polypeptide encoded by the nucleic acid of any of the above embodiments, the RNA of any of the above embodiments, the immunogen of any of the above embodiments, the delivery carrier of any of the above embodiments or the pharmaceutical composition of any of the above embodiments in the preparation of a medicament.

In some embodiments, the medicament is used to prevent or treat VZV infection or diseases caused by VZV infection.

In some embodiments, the medicament is used for preventing, treating or ameliorating neuralgia caused by VZV infection.

In some embodiments, the medicament is used for preventing, treating or ameliorating post-herpes zoster neuralgia.

In some embodiments, the medicament is a vaccine.

### VII. Vaccine

The present disclosure also provides a vaccine comprising the nucleic acid of any of the above embodiments, the genetic engineering vector of any of the above embodiments, the host cell of any of the above embodiments, the protein or polypeptide encoded by the nucleic acid of any of the above embodiments, the immunogen of any of the above embodiments, the RNA of any of the above embodiments, the nucleic acid composition of any of the above embodiments, or the delivery carrier of any of the above embodiments.

In some embodiments, the above vaccine is a VZV vaccine.

In some embodiments, the above vaccine is a multivalent vaccine.

In some embodiments, the above vaccine comprises RNA (e.g., mRNA) comprising a polynucleotide derived from the VZV E1 strain, including, for example, any one or more of genotypes E1_32_5, E1_Kel, E1_Dumas, E1_Russia 1999, E1_SD, E1_MSP, E1_36, E1_49, E1_BC, and E1_NH29. In some embodiments, the above vaccine comprises RNA (e.g., mRNA) comprising a polynucleotide derived from the VZV E2 strain, including, for example, any one or more of genotypes E2_03-500, E2_2, E2_11, and E2_HJO. In some embodiments, the above vaccine comprises RNA (e.g., mRNA) comprising a polynucleotide sequence derived from the VZV J strain, including, for example, genotype pOka. In some embodiments, the RNA (e.g., mRNA) polynucleotide comprises a polynucleotide derived from the VZV M1 strain, including, for example, genotype M1_CA123. In some embodiments, the above vaccine comprises RNA (e.g., mRNA) comprising polynucleotides derived from the VZV M2 strain, including, for example, genotype M2_8 and M2_DR. In some embodiments, the above vaccine comprises RNA (e.g., mRNA) comprising a polynucleotide derived from the VZV M4 strain, including, for example, any one or more of Spanish genotype 4242, Franch genotype 4415, and Italian genotype 4053.

In some embodiments, the above vaccine is a combined nucleic acid vaccine. In some embodiments, the above vaccine comprises a nucleic acid encoding a protein or polypeptide of other virus or a protein or polypeptide of VZV other than VZV gE protein or a fragment thereof that can be used as an immunogen. Of course, the other viruses may be a virus subtype, or a plurality of viruses or a plurality of virus subtypes. For example, the other viruses can be influenza A, and can also be influenza A and SARS-CoV-2, or can further be influenza A and influenza B.

In some embodiments, the above vaccine is a combined protein vaccine. In some embodiments, in addition to any one of the above VZV gE proteins or fragments thereof, the above vaccine further comprises a protein or polypeptide other than VZV gE protein or fragments thereof that can be used as an immunogen, or further comprises a protein or polypeptide of other virus that can be used as an immunogen. Of course, there may be one or more other viruses.

In some embodiments, the vaccine is a quadruple nucleic acid vaccine comprising an active ingredient for preventing measles, mumps, rubella, and varicella or herpes zoster, wherein the active ingredient for preventing varicella or herpes zoster comprises the nucleic acid of any of the above embodiments.

In some embodiments, the above vaccine does not comprise an adjuvant.

In some embodiments, the above vaccine further comprises an adjuvant.

In some embodiments, the adjuvant comprises a flagellin adjuvant. In some embodiments, the flagellin adjuvant is mRNA encoding flagellin. The efficacy of RNA (e.g., m RNA) in a vaccine can be significantly enhanced when combined with a flagellin adjuvant, particularly mRNA encoding an antigen in combination with mRNA encoding a flagellin.

In some embodiments, the above vaccine is an mRNA vaccine.

In some embodiments, the above vaccine is an mRNA vaccine, and the dosage form of the vaccine is nasal, transtracheal or injectable (e.g., intravenous, intraocular, intravitreal, intramuscular, intradermal, intracardiac, intraperitoneal and subcutaneous).

It can be understood that the dosage form of the vaccine is not particularly limited.

The mRNA vaccine of the present disclosure can induce the body to produce a significant immune response, cause the body to produce a high level of IgG antibody against VZV gE protein, and induce a significant IFN-γ-positive cellular immune response.

### VIII. Methods for Prevention or Treatment

The present disclosure also provides a method for preventing or treating VZV viral infection, comprising administering to a subject the nucleic acid of any of the above embodiments, the genetic engineering vector of any of the above embodiments, the host cell of any of the above embodiments, the protein or polypeptide encoded by the nucleic acid of any of the above embodiments, the immunogen of any of the above embodiments, the RNA of any of the above embodiments, the nucleic acid composition of any of the above embodiments, the delivery carrier of any of the above embodiments, the pharmaceutical composition of any of the above embodiments, or the vaccine of any of the above embodiments.

In some embodiments, the subject is a mammal (e.g., a human, a non-human primate (e.g., simian, chimpanzee, monkey, and chimpanzee)), a domestic animal (e.g., dog, cat, and livestock (e.g., horse, cow, pig, sheep, and goat)), or other mammals. Other mammals include, but are not limited to, mouse, rat, guinea pig, rabbit, hamster, etc. In an optional specific example, the subject is a human.

In some embodiments, the frequency of administrations is one, two, three, four, or more.

The present disclosure also provides a method for preventing, treating or ameliorating neuralgia caused by VZV infection, comprising administering to a subject the nucleic acid of any of the above embodiments, the genetic engineering vector of any of the above embodiments, the host cell of any of the above embodiments, the protein or polypeptide encoded by the nucleic acid of any of the above embodiments, the immunogen of any of the above embodiments, the RNA of any of the above embodiments, the nucleic acid composition of any of the above embodiments, the delivery carrier of any of the above embodiments, the pharmaceutical composition of any of the above embodiments or the vaccine of any of the above embodiments.

The present disclosure also provides a method for preventing, treating or improving post-herpes zoster neuralgia, comprising administering to a subject the nucleic acid of any of the above embodiments, the genetic engineering vector of any of the above embodiments, the host cell of any of the above embodiments, the protein or polypeptide encoded by the nucleic acid of any of the above embodiments, the immunogen of any of the above embodiments, the RNA of any of the above embodiments, the nucleic acid composition of any of the above embodiments, the delivery carrier of any of the above embodiments, the pharmaceutical composition of any of the above embodiments or the vaccine of any of the above embodiments.

In some embodiments, the subject is as described above.

In addition, the present disclosure also provides a method of eliciting an immune response against VZV in a subject. In some embodiments, the method comprises administering to a subject the nucleic acid of any of the above embodiments, the genetic engineering vector of any of the above embodiments, the host cell of any of the above embodiments, the protein or polypeptide encoded by the nucleic acid of any of the above embodiments, the immunogen of any of the above embodiments, the RNA of any of the above embodiments, the nucleic acid composition of any of the above embodiments, the delivery carrier of any of the above embodiments, the pharmaceutical composition of any of the above embodiments, or the vaccine of any of the above embodiments. After vaccination with the vaccine of the present disclosure, the anti-antigen antibody titer in the subject is increased as compared to the anti-antigen antibody titer in the subject vaccinated with a prophylactically effective dose of a traditional vaccine against VZV. An "anti-antigen antibody" is a seral antibody that specifically binds to an antigen.

In some embodiments, the method of any of the above embodiments comprises administering the nucleic acid, genetic engineering vector, nucleic acid composition, VZV gE protein or a fragment thereof, RNA, immunogen, delivery carrier, pharmaceutical composition, or vaccine (e.g., mRNA vaccine) of any of the above embodiments to a subject once or twice (or more) at a dosage level sufficient to deliver 0.025 mg/kg to 0.250 mg/kg, 0.025 mg/kg to 0.500 mg/kg, 0.025 mg/kg to 0.750 mg/kg, or 0.025 mg/kg to 1.0 mg/kg, based on the body weight of the subject.

In some embodiments, the method of any of the above embodiments comprises administering the nucleic acid, genetic engineering vector, nucleic acid composition, VZV gE protein or a fragment thereof, RNA, immunogen, delivery carrier, pharmaceutical composition, or vaccine (e.g., mRNA vaccine) of any of the above embodiments to a subject once or twice (or more) at a total dose of 0.0100 mg to 1.0 mg or at a dosage level sufficient to deliver the total dose.

In some embodiments, the method of any one of the above embodiments comprises administering the nucleic acid, genetic engineering vector, nucleic acid composition, VZV gE protein or a fragment thereof, RNA, immunogen, delivery carrier, pharmaceutical composition, or vaccine (e.g., mRNA vaccine) of any one of the above embodiments to the subject twice (e.g., Day 0 and Day 7, Day 0 and Day 14, Day 0 and Day 21, Day 0 and Day 28, Day 0 and Day 60, Day 0 and Day 90, Day 0 and Day 120, Day 0 and Day 150, Day 0 and Day 180, Day 0 and 3 months later, Day 0 and 6 months later Day 0 and 9 months later, Day 0 and 12 months later, Day 0 and 18 months later, Day 0 and 2 years later, Day 0 and 5 years later or Day 0 and 10 years later) at a total dose of 0.0100 mg, 0.025 mg, 0.050 mg, 0.075 mg, 0.100 mg, 0.125 mg, 0.150 mg, 0.175 mg, 0.200 mg, 0.225 mg, 0.250 mg, 0.275 mg, 0.300 mg, 0.325 mg, 0.350 mg, 0.375 mg, 0.400 mg, 0.425 mg, 0.450 mg, 0.475 mg, 0.500 mg, 0.525 mg, 0.550 mg, 0.575 mg, 0.600 mg, 0.625 mg, 0.650 mg, 0.675 mg, 0.700 mg, 0.725 mg, 0.750 mg, 0.775 mg, 0.825 mg, 0.875 mg, 0.900 mg, 0.925 mg, 0.950 mg, 0.975 mg, or 1.0 mg at or at a dosage level sufficient to deliver the total dose. The present disclosure encompasses higher and lower doses and frequencies of administration. For example, the above vaccine (e.g., mRNA vaccine) may be administered for three or four times.

### IX. Detection Reagents or Kits

The present disclosure further provides use of the nucleic acid of any one of the above embodiments, the genetic engineering vector of any one of the above embodiments, the host cell of any one of the above embodiments, the polypeptide or protein encoded by the nucleic acid of any one of the above embodiments, the immunogen of any one of the above embodiments, the RNA of any one of the above embodiments, the nucleic acid composition of any one of the above embodiments, the delivery carrier of any one of the above embodiments, or the pharmaceutical composition of any one of the above embodiments in preparation of a detection reagent or kit for an antibody binding to VZV gE protein.

In addition, the present disclosure also provides a detection reagent or kit for an antibody binding to VZV gE protein, comprising the nucleic acid of any of the above embodiments, the genetic engineering vector of any of the above embodiments, the host cell of any of the above embodiments, the protein or polypeptide encoded by the nucleic acid of any of the above embodiments, the immunogen of any of the above embodiments, the RNA of any of the above embodiments, the nucleic acid composition of any of the above embodiments, the delivery carrier of any of the above embodiments, or the pharmaceutical composition of any of the above embodiments.

In addition, the present disclosure also provides a method for detecting or isolating an antibody binding to VZV gE protein in a subject, comprising: providing an effective amount of the nucleic acid of any of the above embodiments, the genetic engineering vector of any of the above embodiments, the host cell of any of the above embodiments, the protein or polypeptide encoded by the nucleic acid of any of the above embodiments, the immunogen of any of the above embodiments, the RNA of any of the above embodiments, the nucleic acid composition of any of the above embodiments, or the delivery carrier, pharmaceutical composition or vaccine containing the VZV gE protein; contacting a biological sample from the subject with the VZV gE protein under conditions sufficient to form an immune complex of the VZV gE protein and the antibody binding to VZV gE protein; and detecting the immune complex, thereby detecting or isolating the antibody binding to VZV gE protein in the subject.

### Examples

To make the objectives and technical solutions of the present disclosure clearer, they are described in details below in view of the particular Examples. Apparently, the described Examples are only a part of the embodiments of the present invention, rather than all of the embodiments. All other embodiments obtained by a person of ordinary skill in the art based on the Examples in the present invention without creative efforts shall fall within the protection scope of the present invention. Unless otherwise specified, the reagents and instruments used in the Examples are all conventional choices in the art. The experimental methods without specific conditions in the Examples are implemented according to conventional conditions, such as the conditions described in references and books, or the methods recommended by manufacturers.

### Example 1

### I. Synthesis of Compound (IV-1)

### Step 1): Synthesis of nonyl 8-((3-ydroxycyclohexyl) amino)octanoate (Compound (IV-1-p1))

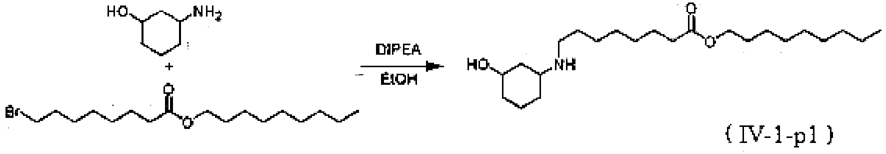

In a 100 mL reaction flask, nonyl 8-bromooctanoate (3.50 g, 10 mmol), 3-aminocyclohexanol (11.5 g, 100 mmol), and 30 mL of ethanol were added in sequence, stirred and dissolved, then N, N-diisopropylethylamine (2.58 g, 20 mmol) was added, reacted at room temperature for 24 h, 100 mL of dichloromethane was added, washed with water for 3 times, dried with anhydrous sodium sulfate, concentrated, and purified by a rapid column chromatography system (dichloromethane: methanol = 20:1 to 5:1) to obtain nonyl 8-((3-hydroxycyclohexyl)amino)octanoate.

¹H NMR (600 MHz, CDCl₃) δ 4.20-4.13 (m, 0.5H), 4.05 (t, 2H), 3.83 (m, 0.5H), 3.09 (m, 0.5H), 2.87 (m, 0.5H), 2.76-2.59 (m, 2H), 2.29 (t, 2H), 2.00 (m, 0.5H), 1.93-1.78 (m, 1.5H), 1.78-1.65 (m, 2H), 1.65-1.46 (m, 8H), 1.40-1.18 (m, 20H), 0.88 (t, 3H).

LCMS:384.3[M+H]⁺ .

### Step 2): Synthesis of Compound (IV-1)

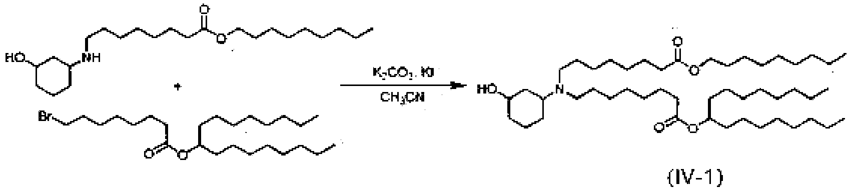

In a 100 mL reaction flask, nonyl 8- ((3-hydroxycyclohexyl) amino) octanoate (383 mg, 1 mmol), 1-octylnonyl 8-bromooctanoate (554 mg, 1.2 mmol), and 20 mL of acetonitrile were sequentially added, stirred and dissolved, then potassium carbonate (276 mg, 2 mmol) and potassium iodide (166 mg, 1 mmol) were added, reacted at room temperature for 24 h, 100 mL of dichloromethane was added, washed with water for 3 times, dried over anhydrous sodium sulfate, concentrated, and purified by a flash column chromatography system (dichloromethane: methanol = 20:1 to 5:1) to obtain compound (IV-1).

¹H NMR (600 MHz, CDCl₃) δ 4.95-4.81 (m, 1H), 4.30-4.12 (m, 0.5H), 4.07 (t, 2H), 3.72-3.61 (m, 0.5H), 2.97 (m, 0.5H), 2.64-2.52 (m, 0.5H), 2.48-2.37 (m, 4H), 2.30 (q, 4H), 1.93-1.81 (m, 2H), 1.72-1.57 (m, 8H), 1.51 (t, 4H), 1.43-1.18 (m, 56H), 0.89 (m, 9H).

LCMS:765.3[M+H]⁺ .

### II. Preparation of Compound (IV-1-1)

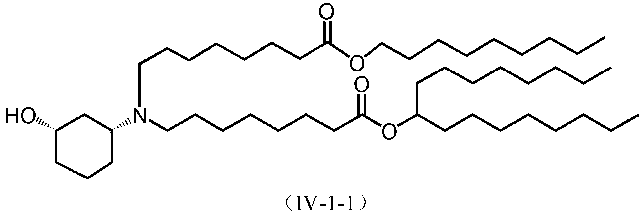

To a 250 mL single-necked flask, 8g (17.3 mmol) of 9-heptadecyl-8-bromooctanoate, 10 g (76.5 mmol) of (1S, 3R)-3-aminocyclohexanol and 100 mL of ethanol were added, and reacted at 50° C for 15 h, and the raw material 9-heptadecyl-8-bromooctanoate was completely reacted. After the solvent was removed by rotary evaporation, the obtained crude product was added with 200 mL of ethyl acetate (EA), washed twice with 100 mL of water, (1S, 3R)-3-aminocyclohexanol was completely washed off, and the EA phase was rotary dried to obtain the crude product of compound (IV-1-1-p) (i.e., 9-heptadecyl-8-(((1R, 3S)-3-hydroxycyclohexyl)amino) octanoate). The crude product was purified by column chromatography (dichloromethane: methanol = 20:1 to 5:1) to obtain compound (IV-1-1-p).

NMR data:
¹H NMR (600 MHz, CDCl₃): δ 4.91-4.81 (m, 1H), 3.86-3.82 (m, 1H), 2.84-2.81 (m, 1H), 2.69-2.54 (qt, J = 11.2, 7.3 Hz, 2H), 2.29-2.26 (t, J = 7.5 Hz, 2H), 1.93-1.86 (m, 1H), 1.77-1.74(d, J = 12.0 Hz, 1H), 1.72-1.57 (m, 6H), 1.54-1.45 (m, 6H), 1.37-1.30 (m, 8H), 1.30-1.22 (m, 24H), 0.88 (t, J = 7.0 Hz, 6H).
LCMS:496.7[M+H]⁺ .

6g (12.1 mmol) of the above compound (IV-1-1-p) was added into a 250 mL single-necked flask, 90 mL of acetonitrile and 60 mL of cyclopentylmethyl ether were added, then 6.7 g of potassium carbonate powder (48.4 mmol), 2 g of potassium iodide (12.1 mmol) and 5 g of nonyl 8-bromooctanoate (17.5 mmol) were added, the reaction was carried out at 90° C for 24 h, the raw material compound ((IV-1-1-p)) was completely reacted, the reaction was removed from the oil bath and cooled to room temperature, the solid was removed by suction filtration, the filtrate was rotary dried to obtain a crude product, and finally purified by column chromatography (dichloromethane: methanol = 20:1 to 5:1) to obtain compound (IV-1-1).

NMR data:
¹H NMR (600 MHz, CDCl₃): δ 4.93-4.84 (m, 1H), 4.09 (t, J = 6.8 Hz, 2H), 3.68-3.61 (m, 1H), 2.62-2.54 (m, 1H), 2.53-2.41 (m, 4H), 2.31-2.26 (q, J = 7.4 Hz, 4H), 1.97 (m, 1H), 1.91-1.78 (m, 2H), 1.71-1.61 (m, 7H), 1.54 (d, J = 6.0 Hz, 4H), 1.43-1.37 (m, 4H), 1.39-1.23 (m, 52H), 0.91 (m, 9H).
Specific rotation:+4.3°
LCMS:765.2[M+H]⁺ .

### III. Preparation of Compound (IV-1-2)

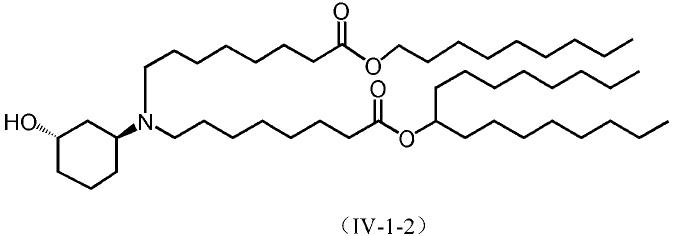

2g of 9-heptadecyl-8-bromooctanoate (4.3 mmol), 2.5 g of (1S, 3S)-3-aminocyclohexanol (20.2 mmol) and 20 mL of ethanol were added to a 100 mL single-necked flask and reacted at 50° C for 15 h, and the raw material 9-heptadecyl-8-bromooctanoate was completely reacted. After the solvent was removed by rotary evaporation, the obtained crude product was added with 50 mL of EA, washed twice with 25 mL of water, (1S, 3S)-3-aminocyclohexanol was thoroughly washed off, and the EA phase was rotary dried to obtain a crude product of the compound (IV-1-2-p) (i.e., 9-heptadecyl-8-(((1S, 3S)-3-hydroxycyclohexyl)amino) octanoate). The crude product was purified by column chromatography (dichloromethane: methanol = 20:1 to 5:1) to obtain compound (IV-1-2-p).

NMR data:
¹H NMR (600 MHz, CDCl₃) : δ 4.91-4.82 (m, 1H), 4.16-4.10 (m, 1H), 2.96-2.87 (m, 1H), 2.65-2.55 (qt, J = 11.2, 7.3 Hz, 2H), 2.28-2.26 (t, J = 7.5 Hz, 2H), 1.93-1.77 (m, 4H), 1.67-1.55 (m, 6H), 1.54-1.45 (m, 6H), 1.33-1.31 (m, 6H), 1.30-1.22 (m, 24H), 0.88 (t, J = 7.0 Hz, 6H).
LCMS:496.7[M+H]⁺ .

1.5g (3.0 mmol) of the above compound (IV-1-2-p) was added into a 100 mL single-necked flask, 25 mL of acetonitrile and 15 mL of cyclopentylmethyl ether were added, then 1.67 g of potassium carbonate powder (12 mmol), 0.5g of potassium iodide (3 mmol) and 1.26 g of nonyl 8-bromooctanoate (3.6 mmol) were added, the reaction was carried out at 90° C for 24 h, the raw material compound (IV-1-2-p) was completely reacted, the reaction was removed from the oil bath and cooled to room temperature, the solid was removed by suction filtration, the filtrate was rotary dried to obtain a crude product, and finally purified by column chromatography (dichloromethane: methanol = 20:1 to 5:1) to obtain compound (IV-1-2).

NMR data:
¹H NMR (600 MHz, CDCl₃): δ 4.91-4.80 (m, 1H), 4.28-4.22 (m, 1H), 4.06-4.04 (t, J = 6.8 Hz, 2H), 3.03-2.97 (m, 1H), 2.47-2.41 (m, 4H), 2.33-2.25 (q, J = 7.5 Hz, 4H), 1.89-1.85 (d, J = 12.1 Hz, 1H), 1.80-1.77 (t, J = 12.2 Hz, 1H), 1.73-1.65 (m, 2H), 1.64-1.59 (m, 6H), 1.54-1.48 (m, 4H), 1.48-1.39 (m, 4H), 1.34-1.29 (m, 14H), 1.29-1.23 (m, 38H), 0.89-0.87 (m, 9H).
Specific rotation: -12.9°
LCMS: 765.2 [M+H]⁺.

### IV. Preparation of Compound (IV-1-3)

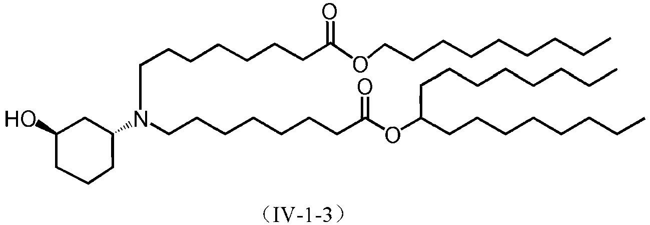

2g of 9-heptadecyl-8-bromooctanoate (4.3 mmol), 2.5 g of (1S, 3S)-3-aminocyclohexanol (20.2 mmol) and 20 mL of ethanol were added to a 100 mL single-necked flask and reacted at 50° C for 15 h, and the raw material 9-heptadecyl-8-bromooctanoate was completely reacted. After the solvent was removed by rotary evaporation, the obtained crude product was added with 50 mL of EA, washed twice with 25 mL of water, (1S, 3S)-3-aminocyclohexanol was thoroughly washed off, and the EA phase was rotary dried to obtain a crude product of the compound (IV-1-3-p) (i.e., 9-heptadecyl-8-(((1R,3R)-3-hydroxycyclohexyl)amino) octanoate). The crude product was purified by column chromatography (dichloromethane: methanol = 20:1 to 5:1) to obtain compound (IV-1-1-p).

NMR data:
¹H NMR (600 MHz, CDCl₃): δ 4.91-4.81 (m, 1H), 4.17-4.08 (m, 1H), 2.94-2.84 (m, 1H), 2.64-2.5 (qt, J = 11.2, 7.3 Hz, 2H), 2.28-2.26 (t, J = 7.5 Hz, 2H), 1.88-1.76 (m, 2H), 1.75-1.54 (m, 8H), 1.54-1.40 (m, 6H), 1.33-1.3 (m, 6H), 1.30-1.23 (m, 24H), 0.88 (t, J = 7.0 Hz, 6H).
LCMS: 496.7[M+H]⁺.

1.5g (3.0 mmol) of the above compound (IV-1-3-p) was added into a 100 mL single-necked flask, 25 mL of acetonitrile and 15 mL of cyclopentylmethyl ether were added, then 1.67 g of potassium carbonate powder (12 mmol), 0.5g of potassium iodide (3 mmol) and 1.26 g of nonyl 8-bromooctanoate (3.6 mmol) were added, the reaction was carried out at 90° C for 24 h, the raw material compound (IV-1-3-p) was completely reacted, the reaction was removed from the oil bath and cooled to room temperature, the solid was removed by suction filtration, the filtrate was rotary dried to obtain a crude product, and finally purified by column chromatography (dichloromethane: methanol = 20:1 to 5:1) to obtain compound (IV-1-2).

NMR data:
¹H NMR (600 MHz, CDCl₃): δ 4.89-4.82 (m, 1H), 4.24-4.22 (m, 1H), 4.06-4.04 (t, J = 6.8 Hz, 2H), 3.00-2.95 (m, 1H), 2.47-2.36 (m, 4H), 2.30-2.26 (q, J = 7.5 Hz, 4H), 1.86-1.84 (d, J = 12.1 Hz, 1H), 1.79-1.77 (t, J = 12.2 Hz, 1H), 1.70-1.66 (m, 2H), 1.65-1.57 (m, 6H), 1.53-1.48 (m, 4H), 1.45-1.38 (m, 4H), 1.35-1.30 (m, 14H), 1.29-1.22 (m, 38H), 0.89-0.87 (m, 9H).
Specific rotation: +12.6°
LCMS: 765.2 [M+H]⁺.

### V. Preparation of Compound (IV-1-4)

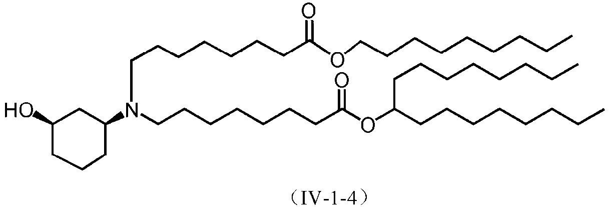

8g (17.3 mmol) of 9-heptadecyl-8-bromooctanoate, 10 g (76.5 mmol) of (1S, 3R)-3-aminocyclohexanol and 100 mL of ethanol were added to a 250 mL single-necked flask, and reacted at 50° C for 15 h, and the raw material 9-heptadecyl-8-bromooctanoate was completely reacted. After the solvent was removed by rotary evaporation, the obtained crude product was added with 50 mL of EA, washed twice with 25 mL of water, (1S,3S)-3-aminocyclohexanol was thoroughly washed off, and the EA phase was rotary dried to obtain a crude product of the compound (IV-1-4-p) (i.e., 9-heptadecyl-8-(((1S, 3R)-3-hydroxycyclohexyl)amino) octanoate).

NMR data:
¹H NMR (600 MHz, CDCl₃) : δ 4.90-4.83 (m, 1H), 3.86-3.82 (m, 1H), 2.86-2.82 (m, 1H), 2.69-2.58 (qt, J = 11.2, 7.3 Hz, 2H), 2.29-2.26 (t, J = 7.5 Hz, 2H), 1.94-1.86 (m, 1H), 1.79-1.77 (d, J = 12.0 Hz, 1H), 1.74-1.64 (m, 3H), 1.64-1.59 (m, 3H), 1.51-1.46 (m, 6H), 1.36-1.30 (m, 8H), 1.30-1.22 (m, 24H), 0.88 (t, J = 7.0 Hz, 6H).
LCMS: 496.7[M+H]⁺ .

6g (12.1 mmol) of the above compound (IV-1-4- p) was added into a 250 mL single-necked flask, 90 mL of acetonitrile and 60 mL of cyclopentylmethyl ether were added, then 6.7 g of potassium carbonate powder (48.4 mmol), 2 g of potassium iodide (12.1 mmol) and 5 g of nonyl 8-bromooctanoate (17.4) were added, the reaction was carried out at 90° C for 24 h, the raw material compound (IV-1-4-p) was completely reacted, the reaction was removed from the oil bath and cooled to room temperature, the solid was removed by suction filtration, the filtrate was rotary dried to obtain a crude product, and finally purified by column chromatography (dichloromethane: methanol = 20:1 to 5:1) to obtain compound (IV-1-4).

NMR data:
¹H NMR (600 MHz, CDCl₃) : δ 4.90-4.82 (m, 1H), 4.05 (t, J = 6.8 Hz, 2H), 3.70-3.60 (m, 1H), 2.64-2.54 (m, 1H), 2.51-2.41 (m, 4H), 2.30-2.26 (q, J = 7.5 Hz, 4H), 1.95 (m, 1H), 1.91-1.77 (m, 2H), 1.69-1.56 (m, 7H), 1.50 (d, J = 6.0 Hz, 4H), 1.41-1.37 (m, 4H), 1.34-1.18 (m, 52H), 0.88 (m, 9H).
Specific rotation: - 4.5°
LCMS: 765.2 [M+H]⁺.

### Example 2 Design of VZV gE Protein and Coding Sequence Thereof

The VZV gE protein as set forth in Table 1 was designed, and the codon-optimized DNA sequence (the DNA sequence corresponding to the coding region of VZV gE protein in Table 1) was further designed according to the amino acid sequence of VZV gE protein, and the synthesis was entrusted to GenScript Biotech, and the detection showed that all DNA sequences were correct.

### Example 3 Construction of Engineered Plasmids

The construction method is as follows:
1, Construction of Plasmid B: GENEWIZ was entrusted to obtain Plasmid B by replacing the Amp resistance gene in plasmid pcDNA3.1 with the Kana resistance gene, adding new restriction sites HindIII, BamHI, KpnI and ApaI subsequent to T7 promoter, and deleting the NeoR/KanR sequence starting from 2136 bp to 2930 bp.
2, Construction of Plasmid C: Plasmid B was subjected to Apal linearized digestion and homologous recombination with a nucleic acid fragment having a nucleotide sequence as set forth in SEQ ID NO: 27 (poly(A) tail) synthesized by IDT (Integrated DNA Technologies) to obtain Plasmid C.
3. Construction of Plasmid D: Plasmid C was double-digested with KpnI and ApaI, the large fragment of about 4.7 kb was recovered by agarose gel electrophoresis, and then ligated with a nucleic acid fragment having a nucleotide sequence as set forth in SEQ ID NO: 26 (3'-UTR), which was synthesized by IDT, with T4 enzyme to obtain Plasmid D.
4, Construction of Plasmid F: Plasmid D was double-digested with BamHI and HindIII, a large fragment of about 4.8 kb was recovered by agarose gel electrophoresis, and then ligated with a nucleic acid fragment having a nucleotide sequence as set forth in SEQ ID NO: 25 (5'-UTR), which was synthesized by IDT, with T4 enzyme to obtain Plasmid F.
5, Construction of engineering plasmids: the homologous arm sequences were introduced to the both ends of the DNA sequences corresponding to the mRNAs numbered as 466, 468, 1086, 1087, 1104, 1106, 1107, 1116 and 1117 without Cap1 cap structure by PCR, respectively, and then the PCR products were subjected to homologous recombination with the fragments of about 4.8 kb obtained by double digestion of plasmid F with BamHI and KpnI and agarose gel electrophoresis, respectively, to finally obtain a plurality of engineering plasmids which were completely correct upon sequencing.

### Example 4 Preparation of mRNA Encoding VZV gE Protein

1. The engineered plasmid was extracted, ensuring that the superhelical rate of the plasmid was 85% or more.
2. Linearization of Plasmid
(1) Linearization by Enzymatic Digestion: 20µg of different engineering plasmids containing the coding sequence for the VZV gE protein as prepared in Example 3 were taken to prepare the reaction systems according to Table 2, which were blended, and placed in an incubator at 37° C for enzymatic digestion reaction for 16 h.

**Table 2**

| | |
|---|---|
| Plasmid | 20µg |
| Buffer | 8µL |
| Endonuclease (BsaI) | 1 µL |
| H₂O | Supplementing to 80µL in total |

(2) Purification of Linearized Plasmid : After complete enzymatic digestion, the linearized plasmid was recovered using a DNA fragment purification and recovering kit (Takara, Cat. No. 9761).
(3) Identification: 100 ng of purified linear plasmid and original circular plasmid were subjected to 1% agarose gel electrophoresis to confirm whether the plasmid was completely linearized.
3. In Vitro Transcription of mRNA
(1) The IVT reaction system was prepared according to Table 3.

**Table 3**

| H₂O | Supplementing to 100µL in total | |
|---|---|---|
| Linearized plasmid | | 5µg |
| 10 × Reaction Buffer | | 10µL |
| ATP (75mM) | | 10µL |
| GTP (75mM) | | 10µL |
| CTP (75mM) | | 10µL |
| N1-Me-Pseudo UTP (100mM) | | 7.5µL |
| T7 RNA polymerase | | 10µL |

(2) The reaction system was reacted at 37° C for 2 h, 5µL of DNase was added to the reaction system at the end of the reaction, and incubated at 37° C for 15min (removing the linearized plasmid template with DNase).
(3) The above IVT product was subjected to magnetic bead purification according to the instructions of magnetic beads for purification (HieffNGS ^{®} RNA Cleaner, YEASEN, Cat. No. 12602 ES56).
(4) Capping:
The mRNA Cap1-type capping reaction:
The structure and reaction principle of the Cap1 type cap are as follows:
pppN1(p)Nx-OH(3') → ppN1(pN)x-OH(3') + Pi
ppN1(pN)x-OH(3') + GTP → G(5')ppp(5')N1(pN)x-OH(3') + PPi
G(5')ppp(5')N1(pN)x-OH(3') + AdoMet → m7G(5')ppp(5')N1(pN)x-OH(3') + AdoHyc
m7GpppN1(pN)x-OH(3') + AdoMet → m7Gppp[m2'-O]N1(pN)x-OH(3') + AdoHyc
5'-Cap1-type cap structure:
   cap G¹G² = m⁷G-5'-ppp-5'-Gm²'-3'-p- [m⁷ = 7-CH₃; m^{2'} = 2'-O-CH₃; -ppp- = -PO₂H-O-PO₂H-O-PO₂H)-; -p- = -PO₂H-].
   67µL of an aqueous solution of mRNA (25 pmol capless mRNA supplemented with water to 67µL) was preheated at 65°C for 5 min, then mixed with a mixture comprising 10µL of 10× ScriptCap Casing Buffer, 10µL of 10 mM GTP, 2.5µL of 20 mM SAM, 2.5µL of ScriptGuard RNase Inhibitor, 4µL of ScriptCap 2'-O-Methyltransferase (100 U/µL) and 4µL of ScriptCap Casing Enzyme (10 U/µL), and incubated at 37°C for 1 h. The above reagents for the capping reaction were purchased from Cellscript.

(5) Purification of Capped Product:
The capped product was purified by magnetic beads according to the instructions of magnetic beads for purification (Hieff NGS^{®} RNA Cleaner, YEASEN, Cat. No. 12602ES56). Characterization data: the concentration was determined by onedrop or Qubit.

Experimental results: The plurality of mRNAs encoding the VZV gE protein or fragment thereof containing Cap1 type cap structure, 5'-UTR, 3'-UTR, poly(A) tail were obtained and all uridines were replaced by N1-methylpseudouridine.

### Example 5 Preparation of LNP Formulation Encapsulating mRNA Encoding VZV gE Protein

(1) Encapsulation: A trace injector and a microfluidic chip (SN. 000038) were used to encapsulate mRNA at a rate of 9 mL/min for the aqueous phase and 3 mL/min for the alcohol phase to prepare a crude product of LNP preparations containing mRNAs encoding a VZV gE protein or a fragment thereof, wherein the LNP preparations differed from each other in the encapsulated mRNAs only, wherein the mRNAs used in this Example were prepared according to the method described in Example 4, the aqueous phase was an acetic acid-sodium acetate solution (pH 5.0) containing mRNA encoding a VZV gE protein, the alcohol phase contained the cationic lipid compounds (IV-1-1), DSPC, CHO-HP and DMG-PEG provided by the present disclosure, and the molar ratio of the cationic lipid compounds (IV-1-1), DSPC, CHO-HP and DMG PEG was 49.5:10:39:1.5.
(2) Solution Exchange by Dialysis:
   (a) Preparation of Dialysis Solution: 1× PBS+8% (m/V) sucrose solution: 2 packets of 1× PBS prefabricated powder were transferred into a beaker, dissolved with 2L DEPC water and blended, 160 g sucrose was subsequently added to obtain 1× PBS+8% sucrose solution upon blending.
   (b) Dialysis: the encapsulated crude product was loaded into a 100 KD dialysis bag, immersed in a beaker containing 1L of dialysis solution, which was wrapped with aluminum foil paper and the dialysis was performed at 100 rpm for 1 h at room temperature, and then the dialysis was continued for 1 h after replacing the dialysis solution. Upon dialysis, the preparation samples were sterilized by filtering with a 0.22µm disposable filter membrane to prepare various LNP preparations encapsulating different mRNAs encoding VZV gE proteins, wherein the mRNA concentration in the LNP preparation was 0.4µg/µL, the mass ratio of mRNA: Lipid was 1:10, the particle size was 80-130 nm, and the encapsulation rate was 85% or more.

### Example 6 In Vivo Immune in Mice

1. Preliminary screening: 7-9 weeks old Balb/c female mice (17g-22g, adaptive feeding in SPF environment) were divided into 2 groups, blank LNP group and mRNA vaccine test group (10µg/mouse), respectively, wherein LNP preparations encapsulating different mRNAs encoding VZV gE protein (as shown in Table 1) were used for the mRNA vaccine test group. The mice were separately subjected to a single intramuscular injection, the time for the first injection was recorded as day 0, spleens were collected on day 6 and day 26 after immunization, and spleen lymphocytes were isolated for subsequent ELIspot experiments.
2. Further screening: 7-9 weeks old Balb/c female mice (17g-22g, adaptive feeding in SPF environment) were divided into 3 groups: blank LNP group, Shingrix positive control group (8µg/mouse, GSK herpes recombinant protein vaccine), mRNA vaccine test group (8µg/mouse), wherein the LNP preparation encapsulating m RNA (1087) encoding VZV gE protein were used for the mRNA vaccine test group. The mice were intramuscularly injected twice, the time for the first injection was recorded as day 0, and the second injection was performed on day 21. On day 14, day 35, day 42, day 49, day 56, day 63, day 70, day 77, and day 81 after immunization (after the first injection), orbital blood was collected, the blood was collected in a non-anticoagulant tube, placed on ice for 30 minutes, centrifuged at 4° C and 3500 rpm for 10 minutes, and after stratification, the upper pale yellow liquid was carefully collected with a pipette to prepare immunoserum.

Spleens of some mice were collected on day 7 and day 81 after immunization, respectively, and spleen lymphocytes were isolated for subsequent ELIspot experiments.

3. The specific preparation steps for spleen lymphocytes include:
(1) The mice were sacrificed by neck breaking, soaked in 75% ethanol, and the spleens of the mice were taken out in the ultra-clean hood.
(2) In a 6-well cell culture plate, 7 mL of mouse lymphocyte separation solution was added to each well (recovered to room temperature and shaken up before use). During grinding with a syringe piston, the upward rebound force of the cell sieve was used to control the grinding force and minimize possible mechanical damage to the cells.
(3) The isolation solution with spleen cells suspended therein was immediately transferred to a 15 mL centrifugation tube, and 10 mL of RPMI 1640 medium was slowly added, keeping the liquid level boundary obvious.
(4) Centrifuging with a horizontal rotor at 800g for 30 min at room temperature.
(5) The lymphocyte layer was pepitted, and then, 10 mL of RPMI 1640 medium was added for washing by overturning the tube. Cells were collected by the centrifugation at 250g for 10 min at room temperature.
(6) The erythrocytes were lysed according to the instructions of the erythrocyte lysis solution. 2 mL of the erythrocyte lysis solution was added to each tube for resuspending. After placing at 4° C. for 2 minutes, 10 mL of DPBS was added to terminate the erythrocyte lysis reaction. After centrifugation at 250g of 4° C. for 10 minutes, and then cells were collected.
(7) 10 mL of RPMI 1640 medium was added to the cells collected in the above step for washing by overturning the tube. The cells were collected by centrifugation at 250g for 10 min at room temperature, and then resuspended with a culture broth, and counted.

### Example 7 ELISpot Experiment on Mouse Spleen Lymphocytes

Mouse spleen lymphocytes required for the ELISpot experiment were provided by the experiment of Example 6.

Reagents: RPMI Medium 1640 basic (1×), mouse lymphocyte isolation solution, positive reference PMA+Ionomycin, ELISpot Plus Mouse IFN-gamma (HRP), VZV gE protein peptide library (see Table 4), ELISpot Plus Mouse IL-4 (HRP), erythrocyte lysis solution.

**Table 4**

| ID | Number of amino acids | Sequence of polypeptide (N to C terminus) |
|---|---|---|
| **1** | 15 | GVYNQGRGIDSGERL |
| **2** | 15 | NQGRGIDSGERLMQP |
| **3** | 15 | RGIDSGERLMQPTQM |
| **4** | 15 | DSGERLMQPTQMSAQ |
| **5** | 15 | ERLMQPTQMSAQEDL |
| **6** | 15 | MQPTQMSAQEDLGDD |
| **7** | 15 | TQMSAQEDLGDDTGI |
| **8** | 15 | SAQEDLGDDTGIHVI |
| **9** | 15 | EDLGDDTGIHVIPTL |
| **10** | 15 | GDDTGIHVIPTLNGD |
| **11** | 15 | TGIHVIPTLNGDDRH |
| **12** | 15 | HVIPTLNGDDRHKIV |
| **13** | 15 | PTLNGDDRHKIVNVD |
| **14** | 15 | NGDDRHKIVNVDQRQ |
| **15** | 15 | DRHKIVNVDQRQYGD |
| **16** | 15 | KIVNVDQRQYGDVFK |
| **17** | 15 | NVDQRQYGDVFKGDL |
| **18** | 15 | QRQYGDVFKGDLNPK |
| **19** | 15 | YGDVFKGDLNPKPQG |
| **20** | 15 | VFKGDLNPKPQGQRL |
| **21** | 15 | GDLNPKPQGQRLIEV |
| **22** | 15 | NPKPQGQRLIEVSVE |
| **23** | 15 | PQGQRLIEVSVEENH |
| **24** | 15 | QRLIEVSVEENHPFT |
| **25** | 15 | IEVSVEENHPFTLRA |
| **26** | 15 | SVEENHPFTLRAPIQ |
| **27** | 15 | ENHPFTLRAPIQRIY |
| **28** | 15 | PFTLRAPIQRIYGVR |
| **29** | 15 | LRAPIQRIYGVRYTE |
| **30** | 15 | PIQRIYGVRYTETWS |
| **31** | 15 | RIYGVRYTETWSFLP |
| **32** | 15 | GVRYTETWSFLPSLT |
| **33** | 15 | YTETWSFLPSLTCTG |
| **34** | 15 | TWSFLPSLTCTGDAA |
| **35** | 15 | FLPSLTCTGDAAPAI |
| **36** | 15 | SLTCTGDAAPAIQHI |
| **37** | 15 | CTGDAAPAIQHICLK |
| **38** | 15 | DAAPAIQHICLKHTT |
| **39** | 15 | PAIQHICLKHTTQRA |
| **40** | 15 | QHICLKHTTQRAIER |
| **41** | 15 | CLKHTTQRAIERYAG |
| **42** | 15 | HTTQRAIERYAGAET |
| **43** | 15 | QRAIERYAGAETAEY |
| **44** | 15 | IERYAGAETAEYSYR |
| **45** | 15 | YAGAETAEYSYRFQG |
| **46** | 15 | AETAEYSYRFQGKKE |
| **47** | 15 | AEYSYRFQGKKEADQ |
| **48** | 15 | SYRFQGKKEADQPWI |
| **49** | 15 | FQGKKEADQPWIVVN |
| **50** | 15 | KKEADQPWIVVNTST |
| **51** | 15 | ADQPWIVVNTSTLFD |
| **52** | 15 | PWIVVNTSTLFDELE |
| **53** | 15 | VVNTSTLFDELELDP |
| **54** | 15 | TSTLFDETELDPPEI |
| **55** | 15 | LFDELELDPPEIEPG |
| **56** | 15 | FTETDPPEIEPGVLK |
| **57** | 15 | LDPPEIEPGVLKVLR |
| **58** | 15 | PEIEPGVLKVLRTEK |
| **59** | 15 | EPGVLKVLRTEKQYL |
| **60** | 15 | VLKVLRTEKQYLGVY |
| **61** | 15 | VLRTEKQYLGVYIWN |
| **62** | 15 | TEKQYLGVYIWNMRG |
| **63** | 15 | QYLGVYIWNMRGSDG |
| **64** | 15 | GVYIWNMRGSDGTST |
| **65** | 15 | IWNMRGSDGTSTYAT |
| **66** | 15 | MRGSDGTSTYATFLV |
| **67** | 15 | SDGTSTYATFLVTWK |
| **68** | 15 | TSTYATFLVTWKGDE |
| **69** | 15 | YATFLVTWKGDEKTR |
| **70** | 15 | FLVTWKGDEKTRNPT |
| **71** | 15 | TWKGDEKTRNPTPAV |
| **72** | 15 | GDEKTRNPTPAVTPQ |
| **73** | 15 | KTRNPTPAVTPQPRG |
| **74** | 15 | NPTPAVTPQPRGAEF |
| **75** | 15 | PAVTPQPRGAEFHMW |
| **76** | 15 | TPQPRGAEFHMWNYH |
| **77** | 15 | PRGAEFHMWNYHSHV |
| **78** | 15 | AEFHMWNYHSHVFSV |
| **79** | 15 | HMWNYHSHVFSVGDT |
| **80** | 15 | NYHSHVFSVGDTFSL |
| **81** | 15 | SHVFSVGDTFSLAMH |
| **82** | 15 | FSVGDTFSLAMHLQY |
| **83** | 15 | GDTFSLAMHLQYKIH |
| **84** | 15 | FSLAMHLQYKIHEAP |
| **85** | 15 | AMHLQYKIHEAPFDL |
| **86** | 15 | LQYKIHEAPFDLLLE |
| **87** | 15 | KIHEAPFDLLLEWLY |
| **88** | 15 | EAPFDLLLEWLYVPI |
| **89** | 15 | FDLLLEWLYVPIDPT |
| **90** | 15 | LLEWLYVPIDPTCQP |
| **91** | 15 | WLYVPIDPTCQPMRL |
| **92** | 15 | VPIDPTCQPMRLYST |
| **93** | 15 | DPTCQPMRLYSTCLY |
| **94** | 15 | CQPMRLYSTCLYHPN |
| **95** | 15 | MRLYSTCLYHPNAPQ |
| **96** | 15 | YSTCLYHPNAPQCLS |
| **97** | 15 | CLYHPNAPQCLSHMN |
| **98** | 15 | HPNAPQCLSHMNSGC |
| **99** | 15 | APQCLSHMNSGCTFT |
| **100** | 15 | CLSHMNSGCTFTSPH |
| **101** | 15 | HMNSGCTFTSPHLAQ |
| **102** | 15 | SGCTFTSPHLAQRVA |
| **103** | 15 | TFTSPHLAQRVASTV |
| **104** | 15 | SPHLAQRVASTVYQN |
| **105** | 15 | LAQRVASTVYQNCEH |
| **106** | 15 | RVASTVYQNCEHADN |
| **107** | 15 | STVYQNCEHADNYTA |
| **108** | 15 | YQNCEHADNYTAYCL |
| **109** | 15 | CEHADNYTAYCLGIS |
| **110** | 15 | ADNYTAYCLGISHME |
| **111** | 15 | YTAYCLGISHMEPSF |
| **112** | 15 | YCLGISHMEPSFGLI |
| **113** | 15 | GISHMEPSFGLILHD |
| **114** | 15 | HMEPSFGLILHDGGT |
| **115** | 15 | PSFGLILHDGGTTLK |
| **116** | 15 | GLILHDGGTTLKFVD |
| **117** | 15 | LHDGGTTLKFVDTPE |
| **118** | 15 | GGTTLKFVDTPESLS |
| **119** | 15 | TLKFVDTPESLSGLY |
| **120** | 15 | FVDTPESLSGLYVFV |
| **121** | 15 | TPESLSGLYVFVVYF |
| **122** | 15 | SLSGLYVFVVYFNGH |
| **123** | 15 | GLYVFVVYFNGHVEA |
| **124** | 15 | VFVVYFNGHVEAVAY |
| **125** | 15 | VYFNGHVEAVAYTVV |
| **126** | 15 | NGHVEAVAYTVVSTV |
| **127** | 15 | VEAVAYTVVSTVDHF |
| **128** | 15 | VAYTVVSTVDHFVNA |
| **129** | 15 | TVVSTVDHFVNAIEE |
| **130** | 15 | STVDHFVNAIEERGF |
| **131** | 15 | DHFVNAIEERGFPPT |
| **132** | 15 | VNAIEERGFPPTAGQ |
| **133** | 15 | IEERGFPPTAGQPPA |

The operation steps include:
(1) The concentration of mouse spleen lymphocytes was adjusted with culture medium, which was added to each experimental well at 1×10⁵ cells/100µL/well.
(2) Stimulators (PMA+Ionomycin working solution and VZV gE protein peptide library (1µg/mL)) were added, 10µL/well.
(3) After all samples and stimulators were added, the plate was covered with a lid. The cells were incubated in a 37°C, 5% CO₂ incubator for 21 hours.
(4) The liquid was thrown out of the well, PBS buffer was added, 200µL/well, which was repeated five times with drying on absorbent paper each time.
(5) The detection antibody was diluted to 1µg/mL with the solution for diluting sample, 100µL/well. The mixture was incubated at room temperature for 2 hours.
(6) The above plate washing operation was repeated.
(7) Enzyme-labeled avidin (Streptavidin-HRP) was diluted for 1000 folds with the solution for diluting sample, 100µL/well, followed by incubation at room temperature for 1 hour.
(8) The above plate washing operation was repeated.
(9) The TMB substrate developing solution was equilibrated to room temperature in advance, 100µL was added to each well, followed by incubation at room temperature in dark for 10 min.
(10) The liquid in the wells was poured, the base of the plate was opened, and the front and back surfaces and the base were washed with deionized water for 3-5 times to terminate the development. The plate was placed in a shady and dark place at room temperature, and the base was closed after the plate was naturally dried.
(11) The plate was read with an enzyme-linked immunospot analyzer for various parameters of the spots and statistical analysis was performs.

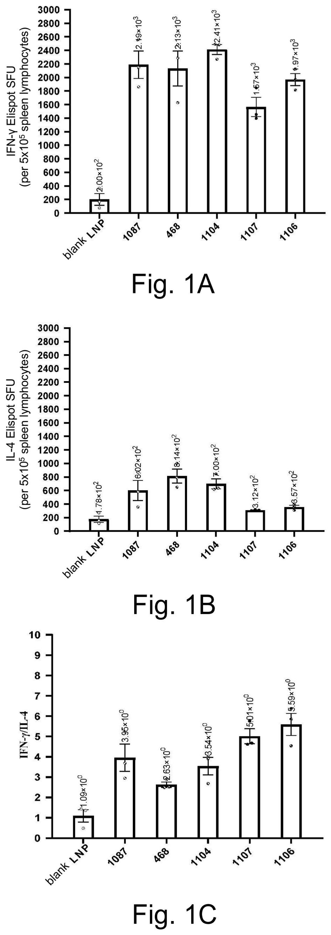

The ELIspot detection of the preliminarily screened mRNA vaccines was shown in FIGS. 1A--1C, and different mRNA vaccines exhibit different levels of IFN-γ-positive cellular immune responses, wherein the Th1 bias of the mRNA vaccine (1087) is stronger than that of the mRNA vaccine (468).

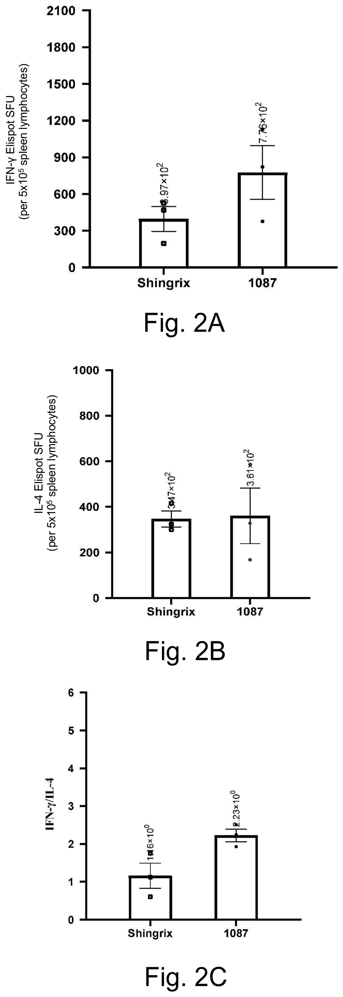

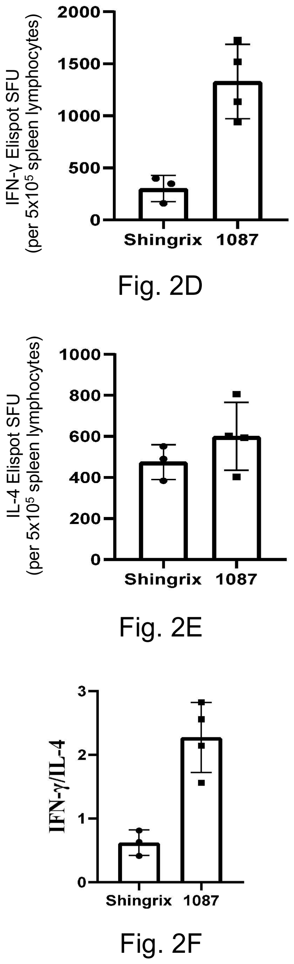

The ELIspot detection of the further screened mRNA vaccines was shown in FIGS. 2A-2F, both mRNA vaccine (1087) and Shingrix can induce the body to produce a significant IFN-γ-positive cellular immune response, and it was revealed that the immune response was Th1 bias rather than Th2 bias, and the mRNA vaccine (1087) had a stronger Th1 bias.

### Example 8 Experiment for IgG Antibody Titer

The immunoserum used in this Example was provided by the experiment in Example 6.

Reagents: Varicella zoster virus (strain Oka vaccine) Envelope Glycoprotein E (gE), His Tag (acrobiosystems, catalog# GLE-V52H3), Goat anti-Mouse IgG (H+L) HRP Conjugate, PBS, FBS, single-component TMB developing solution, and stop solution.

The operation steps include:
(1) The gE protein was diluted to 0.5µg/mL with a coating solution and blended for standby; added to a 96-well ELISA plate at 100µL per well, sealed with a plate sealing membrane, and placed at 2-8°C overnight (16-20 hours).
(2) After incubation, the plate was subjected to machine wash, 300µL/well, for 3 times with washing solution, and drying on clean paper.
(3) The ELISA plate was added with a blocking solution at 250µL/well, sealed with a sealing membrane, and incubated at 37° C for 60 min.
(4) After blocking, the plate was subjected to machine wash, 300µL/well, for 3 times with washing solution, and drying on clean paper.
(5) The serum separated in advance was taken out and mixed by vortex for IgG titer detection.
(6) The separated serum was taken, the initial dilution factor was determined according to different immunization times, and the dilution factor was used as the first dilution factor for gradient dilution. The diluted serum was added to an ELISA plate, 100µL/well, and incubated at 37°C for 2 h.
(7) After incubation, the plate was subjected to machine wash, 300µL/well, for 3 times with washing solution, and drying on clean paper.
(8) The enzyme-labeled secondary antibody was diluted by 10,000 folds with the sample diluting solution, 100µL/well, and incubated at 37° C for 60 min.
(9) After incubation, the plate was subjected to machine wash, 300µL/well, for 3 times with washing solution, and drying on clean paper.
(10) The TMB single-component developing solution was equilibrated to room temperature in advance, 100µL was added to each well, and incubated at room temperature in the dark for 15 min.
(11) At the end of development, 50µL/well of stop solution was added.
(12) A detection wavelength of 450 nm and a reference wavelength of 630 nm were selected for reading and analysis in a microplate reader.

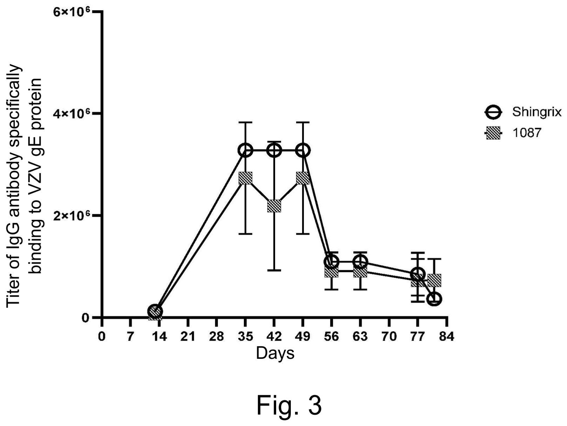

The detection of IgG antibody titer was shown in FIG. 3, and both the mRNA vaccine (1087) and Shingrix induced high levels of IgG antibodies against VZV gE protein, wherein the IgG titer on day 35 was about 3×10⁶.

### Example 9 Detection of Immunogenicity in Rats

In vivo immunization and sampling: 6-8 weeks old Sprague Dawley rats (180g-200g, adaptive feeding in SPF environment) were divided to groups (5 groups for each of male and female, respectively): blank LNP group, mRNA vaccine group 1 (10µg/mouse), mRNA vaccine group 2 (50µg/mouse), mRNA vaccine group 3 (100µg/mouse), mRNA vaccine group 4 (200µg/mouse), wherein the mRNA vaccine group 1-4 employed the LNP preparation encapsulating mRNA numbered as 1087. The rats were subjected to three intramuscular injections respectively, the time for the first injection was recorded as day 0, the time for the second injection needle was day 21, and the time for the third injection was day 36. On day 7, day 14, day 28, day 35, day 42, day 49, and day 56 after immunization, orbital blood was collected, and the rat immune serum was obtained with reference to Example 6, and the titer of IgG antibody in the rat immune serum was determined with reference to Example 7. On day 58 after immunization, spleen collection was performed on all of the rats, and the rat spleen lymphocytes were obtained with reference to Example 6, and then ELIspot experiment for rat spleen lymphocytes was performed with reference to Example 8.

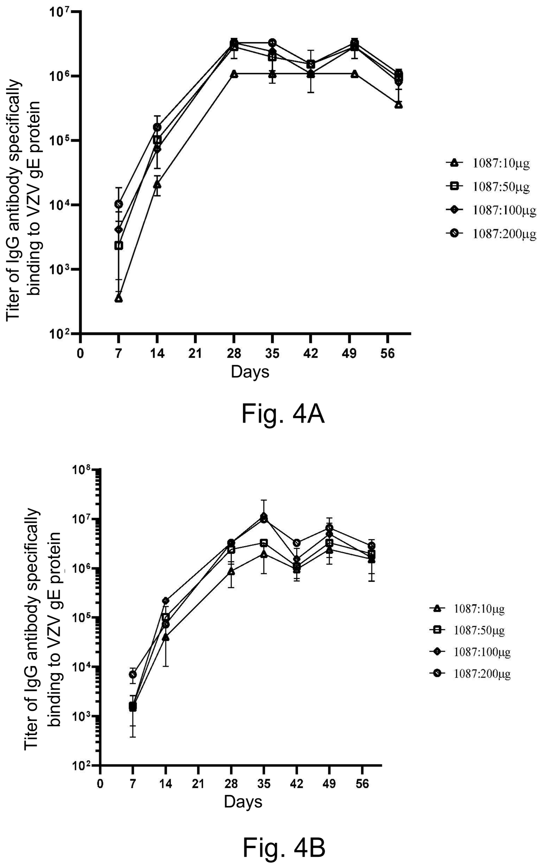

The detection of IgG antibody titer was shown in FIG. 4A to FIG. 4B, in rats of different genders, the mRNA vaccine (1087) produced a higher level of IgG antibody titer, when the injection dose was 10µg, the antibody titer produced by the mRNA vaccine (1807) was lower than that in other dose groups, and when the injection dose is 50µg or more, the further increase in the dose did not make the mRNA vaccine (1087) produce a stronger dose-dependent effect.

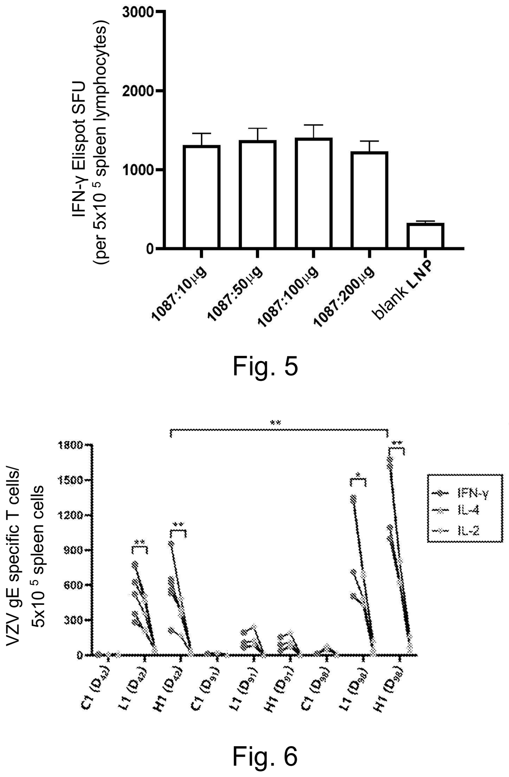

The detection of ELIspot was shown in FIG. 5, 10µg of mRNA vaccine (1087) induced a significant IFN-γ-positive cellular immune response in rats, and no significant dose-dependent effect was observed with the increase of dose.

### Example 10 Immunogenicity of Herpes Zoster mRNA Vaccine (1087) in Pre-immunized Mice

64 C57BL/6 mice (half male and half female, 7-8 weeks old) were randomly divided into 3 groups. The respective groups were labeled as control group (C1), low-dose group (L1) and high-dose group (H1), wherein C1 comprised 16 mice, and L1 and H1 comprised 24 mice, respectively, and all of the mice were injected with vaccinia attenuated live vaccine (LAV, Shanghai Institute of Biological Products Co., Ltd.) at a dose of 600 PFU/mouse at D0, and the spleen was taken at D7 for intracellular cytokine staining (ICS). The first immunization was performed on D35, wherein the control group was injected with blank LNP without herpes zoster mRNA, each mouse in the low-dose group (L1) was injected with 5µg of herpes zoster mRNA vaccine (1087), and each mouse in the high-dose group (H1) was injected with 10µg of herpes zoster mRNA vaccine (1087). Spleens were collected on D42 and D91 for ELISpot and ICS detection. The second immunization was performed on D91 in the remaining mice, and the immunization doses for the control group, the low-dose group and the high-dose group were the same as those of the first immunization. The mouse spleens were collected on D98 for ELISpot and ICS detection. Serum was collected on D28, D35, D42, D63, D91 and D98, and tested for IgG antibody titers.

The results were shown in FIGS. 6-8.

It can be seen from FIG. 6 that after immunization with mRNA vaccine (1087), the average number of IFN-γ SFCs was significantly higher than IL-4 ones, suggesting that the immune response induced by mRNA vaccine (1087) was Th1 bias. Single immunization of mRNA vaccine (1087) in both the high dose group and the low dose group induced mouse spleen cells to produce high levels of T cells with gE-specific secretion of IFN-γ and IL-2, and the second immunization in the high dose group significantly enhanced the above responses.

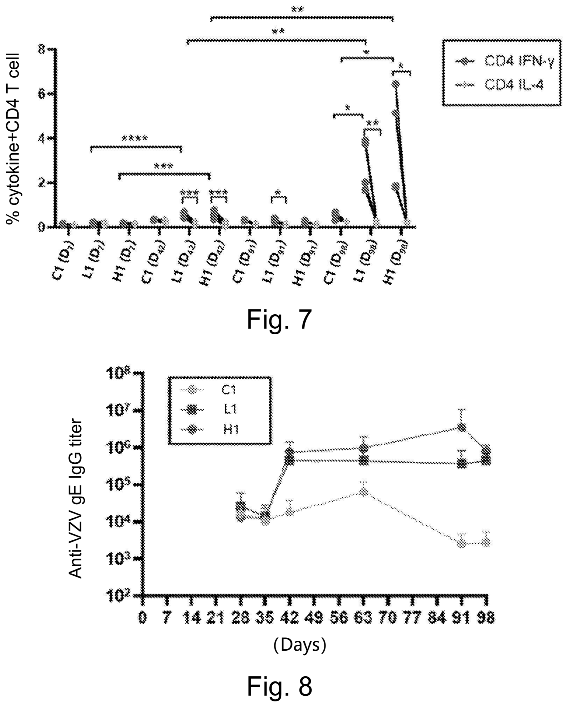

It can be seen from FIG. 7 that a significant CD4 T cell response was induced 7 days after the first mRNA vaccine immunization (D42) in the low-dose group and the high-dose group, and the immune response was significantly enhanced 7 days after the second immunization (D98) (P < 0.01). In addition, the proportion of IFN-γ⁺ cells was significantly higher than that of IL-4⁺ cells in the same animal, indicating that Th cells in the animal tended to differentiate into Th1 cells rather than Th2 cells after the second immunization.

It can be seen from FIG. 8 that a certain level of baseline gE-specific antibody titer was induced 4 week after LAV inoculation (D28) . One week after vaccination with the first dose of mRNA vaccine (1087) (D42), gE-specific antibody IgG titer was significantly increased from baseline.

### SEQUENCES

SEQ ID NO: 1 
MGTVNKPVVGVLMGFGIITGTLRITNPVRASVLRYDDFHIDEDKLDTNSVYEPYYHSDHAESSWVN
RGESSRKAYDHNSPYIWPRNDYDGFLENAHEHHGVYNQGRGIDSGERLMQPTQMSAQEDLGDDTGI
HVIPTLNGDDRHKIVNVDQRQYGDVFKGDLNPKPQGQRLIEVSVEENHPFTLRAPIQRIYGVRYTE
TWSFLPSLTCTGDAAPAIQHICLKHTTCFQDVVVDVDCAENTKEDQLAEISYRFQGKKEADQPWIV
VNTSTLFDELELDPPEIEPGVLKVLRTEKQYLGVYIWNMRGSDGTSTYATFLVTWKGDEKTRNPTP
AVTPQPRGAEFHMWNYHSHVFSVGDTFSLAMHLQYKIHEAPFDLLLEWLYVPIDPTCQPMRLYSTC
LYHPNAPQCLSHMNSGCTFTSPHLAQRVASTVYQNCEHADNYTAYCLGISHMEPSFGLILHDGGTT
LKFVDTPESLSGLYVFVVYFNGHVEAVAYTVVSTVDHFVNAIEERGFPPTAGQPPATTKPKEITPV
NPGTSPLLRYAAWTGGLAAVVLLCLVIFLICTAKRMRVKAYRVDKSPYNQSMYYAGLPVDDFEDSE
STDTEEEFGNAIGGSHGGSSYTVYIDKTR
SEQ ID NO: 2 
RITNPVRASVLRYDDFHIDEDKLDTNSVYEPYYHSDHAESSWVNRGESSRKAYDHNSPYIWPRNDY
DGFLENAHEHHGVYNQGRGIDSGERLMQPTQMSAQEDLGDDTGIHVIPTLNGDDRHKIVNVDQRQY
GDVFKGDLNPKPQGQRLIEVSVEENHPFTLRAPIQRIYGVRYTETWSFLPSLTCTGDAAPAIQHIC
LKHTTCFQDVVVDVDCAENTKEDQLAEISYRFQGKKEADQPWIVVNTSTLFDELELDPPEIEPGVL
KVLRTEKQYLGVYIWNMRGSDGTSTYATFLVTWKGDEKTRNPTPAVTPQPRGAEFHMWNYHSHVFS
VGDTFSLAMHLQYKIHEAPFDLLLEWLYVPIDPTCQPMRLYSTCLYHPNAPQCLSHMNSGCTFTSP
HLAQRVASTVYQNCEHADNYTAYCLGISHMEPSFGLILHDGGTTLKFVDTPESLSGLYVFVVYFNG
HVEAVAYTVVSTVDHFVNAIEERGFPPTAGQPPATTKPKEITPVNPGTSPLLRYAAWTGGLAAVVL
LCLVIFLICTAKRMRVKAYRVDKSPYNQSMYYAGLPVDDFEDSESTDTEEEFGNAIGGSHGGSSYT
VYIDKTR
SEQ ID NO: 3 
SVLRYDDFHIDEDKLDTNSVYEPYYHSDHAESSWVNRGESSRKAYDHNSPYIWPRNDYDGFLENAH
EHHGVYNQGRGIDSGERLMQPTQMSAQEDLGDDTGIHVIPTLNGDDRHKIVNVDQRQYGDVFKGDL
NPKPQGQRLIEVSVEENHPFTLRAPIQRIYGVRYTETWSFLPSLTCTGDAAPAIQHICLKHTTCFQ
DVVVDVDCAENTKEDQLAEISYRFQGKKEADQPWIVVNTSTLFDELELDPPEIEPGVLKVLRTEKQ
YLGVYIWNMRGSDGTSTYATFLVTWKGDEKTRNPTPAVTPQPRGAEFHMWNYHSHVFSVGDTFSLA
MHLQYKIHEAPFDLLLEWLYVPIDPTCQPMRLYSTCLYHPNAPQCLSHMNSGCTFTSPHLAQRVAS
TVYQNCEHADNYTAYCLGISHMEPSFGLILHDGGTTLKFVDTPESLSGLYVFVVYFNGHVEAVAYT
VVSTVDHFVNAIEERGFPPTAGQPPATTKPKEITPVNPGTSPLLRYAAWTGGLAAVVLLCLVIFLI
 CTAKRMRVKAYRVDKSPYNQSMYYAGLPVDDFEDSESTDTEEEFGNAIGGSHGGSSYTVYIDKTR
SEQ ID NO: 4 
MGTVNKPVVGVLMGFGIITGTLRITNPVRASVLRYDDFHIDEDKLDTNSVYEPYYHSDHAESSWVN
RGESSRKAYDHNSPYIWPRNDYDGFLENAHEHHGVYNQGRGIDSGERLMQPTQMSAQEDLGDDTGI
HVIPTLNGDDRHKIVNVDQRQYGDVFKGDLNPKPQGQRLIEVSVEENHPFTLRAPIQRIYGVRYTE
TWSFLPSLTCTGDAAPAIQHICLKHTTCFQDVVVDVDCAENTKEDQLAEISYRFQGKKEADQPWIV
VNTSTLFDELELDPPEIEPGVLKVLRTEKQYLGVYIWNMRGSDGTSTYATFLVTWKGDEKTRNPTP
AVTPQPRGAEFHMWNYHSHVFSVGDTFSLAMHLQYKIHEAPFDLLLEWLYVPIDPTCQPMRLYSTC
LYHPNAPQCLSHMNSGCTFTSPHLAQRVASTVYQNCEHADNYTAYCLGISHMEPSFGLILHDGGTT
LKFVDTPESLSGLYVFVVYFNGHVEAVAYTVVSTVDHFVNAIEERGFPPTAGQPPATTKPKEITPV
NPGTSPLLRYAAWTGGLAAVVLLCLVIFLICTA
SEQ ID NO: 5 
MGTVNKPVVGVLMGFGIITGTLRITNPVRASVLRYDDFHIDEDKLDTNSVYEPYYHSDHAESSWVN
RGESSRKAYDHNSPYIWPRNDYDGFLENAHEHHGVYNQGRGIDSGERLMQPTQMSAQEDLGDDTGI
HVIPTLNGDDRHKIVNVDQRQYGDVFKGDLNPKPQGQRLIEVSVEENHPFTLRAPIQRIYGVRYTE
TWSFLPSLTCTGDAAPAIQHICLKHTTCFQDVVVDVDCAENTKEDQLAEISYRFQGKKEADQPWIV
VNTSTLFDELELDPPEIEPGVLKVLRTEKQYLGVYIWNMRGSDGTSTYATFLVTWKGDEKTRNPTP
AVTPQPRGAEFHMWNYHSHVFSVGDTFSLAMHLQYKIHEAPFDLLLEWLYVPIDPTCQPMRLYSTC
LYHPNAPQCLSHMNSGCTFTSPHLAQRVASTVYQNCEHADNYTAYCLGISHMEPSFGLILHDGGTT
LKFVDTPESLSGLYVFVVYFNGHVEAVAYTVVSTVDHFVNAIEERGFPPTAGQPPATTKPKEITPV
NPGTSPLLRYAAWTGGLAAVVLLCLVIFLICTAKRMRVKAARVDK
SEQ ID NO: 6 
MGTVNKPVVGVLMGFGIITGTLRITNPVRASVLRYDDFHIDEDKLDTNSVYEPYYHSDHAESSWVN
RGESSRKAYDHNSPYIWPRNDYDGFLENAHEHHGVYNQGRGIDSGERLMQPTQMSAQEDLGDDTGI
HVIPTLNGDDRHKIVNVDQRQYGDVFKGDLNPKPQGQRLIEVSVEENHPFTLRAPIQRIYGVRYTE
TWSFLPSLTCTGDAAPAIQHICLKHTTCFQDVVVDVDCAENTKEDQLAEISYRFQGKKEADQPWIV
VNTSTLFDELELDPPEIEPGVLKVLRTEKQYLGVYIWNMRGSDGTSTYATFLVTWKGDEKTRNPTP
AVTPQPRGAEFHMWNYHSHVFSVGDTFSLAMHLQYKIHEAPFDLLLEWLYVPIDPTCQPMRLYSTC
LYHPNAPQCLSHMNSGCTFTSPHLAQRVASTVYQNCEHADNYTAYCLGISHMEPSFGLILHDGGTT
LKFVDTPESLSGLYVFVVYFNGHVEAVAYTVVSTVDHFVNAIEERGFPPTAGQPPATTKPKEITPV
NPGTSPLLRYAAWTG
SEQ ID NO: 7 
SVLRYDDFHIDEDKLDTNSVYEPYYHSDHAESSWVNRGESSRKAYDHNSPYIWPRNDYDGFLENAH
EHHGVYNQGRGIDSGERLMQPTQMSAQEDLGDDTGIHVIPTLNGDDRHKIVNVDQRQYGDVFKGDL
NPKPQGQRLIEVSVEENHPFTLRAPIQRIYGVRYTETWSFLPSLTCTGDAAPAIQHICLKHTTCFQ
DVVVDVDCAENTKEDQLAEISYRFQGKKEADQPWIVVNTSTLFDELELDPPEIEPGVLKVLRTEKQ
YLGVYIWNMRGSDGTSTYATFLVTWKGDEKTRNPTPAVTPQPRGAEFHMWNYHSHVFSVGDTFSLA
MHLQYKIHEAPFDLLLEWLYVPIDPTCQPMRLYSTCLYHPNAPQCLSHMNSGCTFTSPHLAQRVAS
TVYQNCEHADNYTAYCLGISHMEPSFGLILHDGGTTLKFVDTPESLSGLYVFVVYFNGHVEAVAYT
VVSTVDHFVNAIEERGFPPTAGQPPATTKPKEITPVNPGTSPLLRYAAWTGGLAAVVLLCLVIFLI
CTAKRMRVKAARVDK
SEQ ID NO: 8 
SVLRYDDFHIDEDKLDTNSVYEPYYHSDHAESSWVNRGESSRKAYDHNSPYIWPRNDYDGFLENAH
EHHGVYNQGRGIDSGERLMQPTQMSAQEDLGDDTGIHVIPTLNGDDRHKIVNVDQRQYGDVFKGDL
NPKPQGQRLIEVSVEENHPFTLRAPIQRIYGVRYTETWSFLPSLTCTGDAAPAIQHICLKHTTCFQ
DVVVDVDCAENTKEDQLAEISYRFQGKKEADQPWIVVNTSTLFDELELDPPEIEPGVLKVLRTEKQ
YLGVYIWNMRGSDGTSTYATFLVTWKGDEKTRNPTPAVTPQPRGAEFHMWNYHSHVFSVGDTFSLA
MHLQYKIHEAPFDLLLEWLYVPIDPTCQPMRLYSTCLYHPNAPQCLSHMNSGCTFTSPHLAQRVAS
TVYQNCEHADNYTAYCLGISHMEPSFGLILHDGGTTLKFVDTPESLSGLYVFVVYFNGHVEAVAYT
VVSTVDHFVNAIEERGFPPTAGQPPATTKPKEITPVNPGTSPLLRYAAWTG
SEQ ID NO: 9
   METDTLLLWVLLLWVPGSTG
SEQ ID NO: 10 
MESQVRQQFSKDIEKLLNEQVNKEMNSSNLYMSMSSWCYTHSLDGAGLFLFDHAAEEYEHAKKLII
FLNENNVPVQLTSISAPEHKFEGLTQIFQKAYEHEQHISESINNIVDHAIKSKDHATFNFLQWYVA
EQHEEEVLFKDILDKIELIGNENHGLYLADQYVKGIAKSRKS
SEQ ID NO: 11 
ATGGGCACAGTGAACAAGCCTGTTGTGGGCGTGCTGATGGGCTTCGGCATCATCACAGGCACCCTG
AGAATCACCAATCCTGTGCGGGCCAGCGTGCTGAGATACGACGATTTCCACATCGACGAGGACAAG
CTGGACACCAACAGCGTGTACGAGCCCTACTACCACAGCGATCACGCCGAGTCTAGCTGGGTCAAC
 
AGAGGCGAAAGCAGCAGAAAGGCCTACGACCACAACAGCCCCTACATCTGGCCCAGAAACGACTAC
GACGGCTTCCTGGAAAATGCCCACGAACACCACGGCGTGTACAATCAAGGCAGAGGCATCGATAGC
GGCGAGAGACTGATGCAGCCTACACAGATGAGCGCCCAAGAGGATCTGGGCGACGATACAGGCATC
CACGTGATCCCTACACTGAACGGCGACGACCGGCACAAGATCGTGAATGTGGATCAGAGACAGTAC
GGCGACGTGTTCAAGGGCGATCTGAACCCTAAGCCTCAGGGCCAGCGCCTGATCGAAGTGTCCGTG
GAAGAAAATCACCCCTTCACACTGAGAGCCCCTATCCAGAGAATCTACGGCGTGCGCTATACCGAG
ACATGGTCCTTTCTGCCTAGCCTGACATGTACTGGCGACGCCGCTCCTGCCATCCAGCACATTTGT
CTGAAGCACACCACCTGTTTCCAGGACGTGGTGGTGGATGTGGACTGCGCCGAGAACACCAAAGAG
GATCAGCTGGCCGAGATCAGCTACCGGTTCCAGGGAAAGAAAGAGGCCGACCAGCCTTGGATCGTG
GTCAACACCAGCACACTGTTCGACGAGCTGGAACTGGACCCTCCTGAGATTGAGCCTGGGGTGCTG
AAGGTGCTGAGAACCGAGAAGCAGTACCTGGGAGTGTACATCTGGAACATGAGAGGCTCCGACGGC
ACCAGCACCTACGCCACATTTCTGGTTACCTGGAAGGGCGACGAAAAGACCAGAAATCCCACACCA
GCCGTGACACCTCAACCTAGAGGCGCCGAGTTTCACATGTGGAATTACCACAGCCACGTGTTCAGC
GTGGGCGATACCTTTAGCCTGGCCATGCATCTGCAGTACAAGATCCACGAGGCCCCTTTCGACCTG
CTGCTGGAATGGCTGTACGTGCCCATCGATCCTACCTGCCAGCCTATGAGACTGTACAGCACCTGT
CTGTATCACCCCAACGCTCCTCAGTGCCTGAGCCACATGAATAGCGGCTGCACCTTCACAAGCCCT
CACCTGGCTCAGAGAGTGGCCAGCACAGTCTACCAGAATTGCGAGCACGCCGACAATTACACCGCC
TACTGTCTGGGCATCAGCCACATGGAACCTAGCTTCGGCCTGATCCTGCACGATGGCGGCACCACA
CTGAAGTTCGTGGATACACCTGAGAGCCTGAGCGGCCTGTATGTGTTTGTGGTGTACTTCAACGGC
CACGTGGAAGCCGTGGCCTACACTGTGGTGTCCACCGTGGATCACTTCGTGAACGCCATCGAGGAA
CGGGGATTTCCACCAACTGCTGGACAGCCTCCTGCCACCACCAAGCCTAAAGAAATCACACCCGTG
AATCCCGGCACCTCTCCACTGCTTAGATATGCCGCTTGGACAGGCGGACTGGCTGCTGTGGTTCTG
CTGTGCCTGGTCATCTTCCTGATCTGCACCGCCAAGCGGATGAGAGTGAAGGCCTACAGAGTGGAC
AAGAGCCCTTACAACCAGAGCATGTACTACGCCGGCCTGCCTGTGGACGATTTCGAGGATAGCGAG
AGCACCGACACCGAGGAAGAGTTTGGCAACGCCATTGGAGGCTCTCACGGCGGCAGCAGCTACACC
GTGTACATCGACAAGACCCGGTGA
SEQ ID NO: 12 
AGAATCACCAATCCTGTGCGGGCCAGCGTGCTGAGATACGACGATTTCCACATCGACGAGGACAAG
CTGGACACCAACAGCGTGTACGAGCCCTACTACCACAGCGATCACGCCGAGTCTAGCTGGGTCAAC
AGAGGCGAAAGCAGCAGAAAGGCCTACGACCACAACAGCCCCTACATCTGGCCCAGAAACGACTAC
GACGGCTTCCTGGAAAATGCCCACGAACACCACGGCGTGTACAATCAAGGCAGAGGCATCGATAGC
GGCGAGAGACTGATGCAGCCTACACAGATGAGCGCCCAAGAGGATCTGGGCGACGATACAGGCATC
CACGTGATCCCTACACTGAACGGCGACGACCGGCACAAGATCGTGAATGTGGATCAGAGACAGTAC
GGCGACGTGTTCAAGGGCGATCTGAACCCTAAGCCTCAGGGCCAGCGCCTGATCGAAGTGTCCGTG
 
GAAGAAAATCACCCCTTCACACTGAGAGCCCCTATCCAGAGAATCTACGGCGTGCGCTATACCGAG
ACATGGTCCTTTCTGCCTAGCCTGACATGTACTGGCGACGCCGCTCCTGCCATCCAGCACATTTGT
CTGAAGCACACCACCTGTTTCCAGGACGTGGTGGATGTGGACTGCGCCGAGAACACCAAAGAG
GATCAGCTGGCCGAGATCAGCTACCGGTTCCAGGGAAAGAAAGAGGCCGACCAGCCTTGGATCGTG
GTCAACACCAGCACACTGTTCGACGAGCTGGAACTGGACCCTCCTGAGATTGAGCCTGGGGTGCTG
AAGGTGCTGAGAACCGAGAAGCAGTACCTGGGAGTGTACATCTGGAACATGAGAGGCTCCGACGGC
ACCAGCACCTACGCCACATTTCTGGTTACCTGGAAGGGCGACGAAAAGACCAGAAATCCCACACCA
GCCGTGACACCTCAACCTAGAGGCGCCGAGTTTCACATGTGGAATTACCACAGCCACGTGTTCAGC
GTGGGCGATACCTTTAGCCTGGCCATGCATCTGCAGTACAAGATCCACGAGGCCCCTTTCGACCTG
CTGCTGGAATGGCTGTACGTGCCCATCGATCCTACCTGCCAGCCTATGAGACTGTACAGCACCTGT
CTGTATCACCCCAACGCTCCTCAGTGCCTGAGCCACATGAATAGCGGCTGCACCTTCACAAGCCCT
CACCTGGCTCAGAGAGTGGCCAGCACAGTCTACCAGAATTGCGAGCACGCCGACAATTACACCGCC
TACTGTCTGGGCATCAGCCACATGGAACCTAGCTTCGGCCTGATCCTGCACGATGGCGGCACCACA
CTGAAGTTCGTGGATACACCTGAGAGCCTGAGCGGCCTGTATGTGTTTGTGGTGTACTTCAACGGC
CACGTGGAAGCCGTGGCCTACACTGTGGTGTCCACCGTGGATCACTTCGTGAACGCCATCGAGGAA
CGGGGATTTCCACCAACTGCTGGACAGCCTCCTGCCACCACCAAGCCTAAAGAAATCACACCCGTG
AATCCCGGCACCTCTCCACTGCTTAGATATGCCGCTTGGACAGGCGGACTGGCTGCTGTGGTTCTG
CTGTGCCTGGTCATCTTCCTGATCTGCACCGCCAAGCGGATGAGAGTGAAGGCCTACAGAGTGGAC
AAGAGCCCTTACAACCAGAGCATGTACTACGCCGGCCTGCCTGTGGACGATTTCGAGGATAGCGAG
AGCACCGACACCGAGGAAGAGTTTGGCAACGCCATTGGAGGCTCTCACGGCGGCAGCAGCTACACC
GTGTACATCGACAAGACCCGGTGA
SEQ ID NO: 13 
AGCGTGCTGAGATACGACGATTTCCACATCGACGAGGACAAGCTGGACACCAACAGCGTGTACGAG
CCCTACTACCACAGCGATCACGCCGAGTCTAGCTGGGTCAACAGAGGCGAAAGCAGCAGAAAGGCC
TACGACCACAACAGCCCCTACATCTGGCCCAGAAACGACTACGACGGCTTCCTGGAAAATGCCCAC
GAACACCACGGCGTGTACAATCAAGGCAGAGGCATCGATAGCGGCGAGAGACTGATGCAGCCTACA
CAGATGAGCGCCCAAGAGGATCTGGGCGACGATACAGGCATCCACGTGATCCCTACACTGAACGGC
GACGACCGGCACAAGATCGTGAATGTGGATCAGAGACAGTACGGCGACGTGTTCAAGGGCGATCTG
AACCCTAAGCCTCAGGGCCAGCGCCTGATCGAAGTGTCCGTGGAAGAAAATCACCCCTTCACACTG
AGAGCCCCTATCCAGAGAATCTACGGCGTGCGCTATACCGAGACATGGTCCTTTCTGCCTAGCCTG
ACATGTACTGGCGACGCCGCTCCTGCCATCCAGCACATTTGTCTGAAGCACACCACCTGTTTCCAG
GACGTGGTGGTGGATGTGGACTGCGCCGAGAACACCAAAGAGGATCAGCTGGCCGAGATCAGCTAC
CGGTTCCAGGGAAAGAAAGAGGCCGACCAGCCTTGGATCGTGGTCAACACCAGCACACTGTTCGAC
GAGCTGGAACTGGACCCTCCTGAGATTGAGCCTGGGGTGCTGAAGGTGCTGAGAACCGAGAAGCAG
 
TACCTGGGAGTGTACATCTGGAACATGAGAGGCTCCGACGGCACCAGCACCTACGCCACATTTCTG
GTTACCTGGAAGGGCGACGAAAAGACCAGAAATCCCACACCAGCCGTGACACCTCAACCTAGAGGC
GCCGAGTTTCACATGTGGAATTACCACAGCCACGTGTTCAGCGTGGGCGATACCTTTAGCCTGGCC
ATGCATCTGCAGTACAAGATCCACGAGGCCCCTTTCGACCTGCTGCTGGAATGGCTGTACGTGCCC
ATCGATCCTACCTGCCAGCCTATGAGACTGTACAGCACCTGTCTGTATCACCCCAACGCTCCTCAG
TGCCTGAGCCACATGAATAGCGGCTGCACCTTCACAAGCCCTCACCTGGCTCAGAGAGTGGCCAGC
ACAGTCTACCAGAATTGCGAGCACGCCGACAATTACACCGCCTACTGTCTGGGCATCAGCCACATG
GAACCTAGCTTCGGCCTGATCCTGCACGATGGCGGCACCACACTGAAGTTCGTGGATACACCTGAG
AGCCTGAGCGGCCTGTATGTGTTTGTGGTGTACTTCAACGGCCACGTGGAAGCCGTGGCCTACACT
GTGGTGTCCACCGTGGATCACTTCGTGAACGCCATCGAGGAACGGGGATTTCCACCAACTGCTGGA
CAGCCTCCTGCCACCACCAAGCCTAAAGAAATCACACCCGTGAATCCCGGCACCTCTCCACTGCTT
AGATATGCCGCTTGGACAGGCGGACTGGCTGCTGTGGTTCTGCTGTGCCTGGTCATCTTCCTGATC
TGCACCGCCAAGCGGATGAGAGTGAAGGCCTACAGAGTGGACAAGAGCCCTTACAACCAGAGCATG
TACTACGCCGGCCTGCCTGTGGACGATTTCGAGGATAGCGAGAGCACCGACACCGAGGAAGAGTTT
GGCAACGCCATTGGAGGCTCTCACGGCGGCAGCAGCTACACCGTGTACATCGACAAGACCCGGTGA
SEQ ID NO: 14 
ATGGGCACAGTGAACAAGCCTGTTGTGGGCGTGCTGATGGGCTTCGGCATCATCACAGGCACCCTG
AGAATCACCAATCCTGTGCGGGCCAGCGTGCTGAGATACGACGATTTCCACATCGACGAGGACAAG
CTGGACACCAACAGCGTGTACGAGCCCTACTACCACAGCGATCACGCCGAGTCTAGCTGGGTCAAC
AGAGGCGAAAGCAGCAGAAAGGCCTACGACCACAACAGCCCCTACATCTGGCCCAGAAACGACTAC
GACGGCTTCCTGGAAAATGCCCACGAACACCACGGCGTGTACAATCAAGGCAGAGGCATCGATAGC
GGCGAGAGACTGATGCAGCCTACACAGATGAGCGCCCAAGAGGATCTGGGCGACGATACAGGCATC
CACGTGATCCCTACACTGAACGGCGACGACCGGCACAAGATCGTGAATGTGGATCAGAGACAGTAC
GGCGACGTGTTCAAGGGCGATCTGAACCCTAAGCCTCAGGGCCAGCGCCTGATCGAAGTGTCCGTG
GAAGAAAATCACCCCTTCACACTGAGAGCCCCTATCCAGAGAATCTACGGCGTGCGCTATACCGAG
ACATGGTCCTTTCTGCCTAGCCTGACATGTACTGGCGACGCCGCTCCTGCCATCCAGCACATTTGT
CTGAAGCACACCACCTGTTTCCAGGACGTGGTGGATGTGGACTGCGCCGAGAACACCAAAGAG
GATCAGCTGGCCGAGATCAGCTACCGGTTCCAGGGAAAGAAAGAGGCCGACCAGCCTTGGATCGTG
GTCAACACCAGCACACTGTTCGACGAGCTGGAACTGGACCCTCCTGAGATTGAGCCTGGGGTGCTG
AAGGTGCTGAGAACCGAGAAGCAGTACCTGGGAGTGTACATCTGGAACATGAGAGGCTCCGACGGC
ACCAGCACCTACGCCACATTTCTGGTTACCTGGAAGGGCGACGAAAAGACCAGAAATCCCACACCA
GCCGTGACACCTCAACCTAGAGGCGCCGAGTTTCACATGTGGAATTACCACAGCCACGTGTTCAGC
GTGGGCGATACCTTTAGCCTGGCCATGCATCTGCAGTACAAGATCCACGAGGCCCCTTTCGACCTG
CTGCTGGAATGGCTGTACGTGCCCATCGATCCTACCTGCCAGCCTATGAGACTGTACAGCACCTGT
 
CTGTATCACCCCAACGCTCCTCAGTGCCTGAGCCACATGAATAGCGGCTGCACCTTCACAAGCCCT
CACCTGGCTCAGAGAGTGGCCAGCACAGTCTACCAGAATTGCGAGCACGCCGACAATTACACCGCC
TACTGTCTGGGCATCAGCCACATGGAACCTAGCTTCGGCCTGATCCTGCACGATGGCGGCACCACA
CTGAAGTTCGTGGATACACCTGAGAGCCTGAGCGGCCTGTATGTGTTTGTGGTGTACTTCAACGGC
CACGTGGAAGCCGTGGCCTACACTGTGGTGTCCACCGTGGATCACTTCGTGAACGCCATCGAGGAA
CGGGGATTTCCACCAACTGCTGGACAGCCTCCTGCCACCACCAAGCCTAAAGAAATCACACCCGTG
AATCCCGGCACCTCTCCACTGCTTAGATATGCCGCTTGGACAGGCGGACTGGCTGCTGTGGTTCTG
CTGTGCCTGGTCATCTTCCTGATCTGCACCGCCTGA
SEQ ID NO: 15 
ATGGGCACAGTGAACAAGCCTGTTGTGGGCGTGCTGATGGGCTTCGGCATCATCACAGGCACCCTG
AGAATCACCAATCCTGTGCGGGCCAGCGTGCTGAGATACGACGATTTCCACATCGACGAGGACAAG
CTGGACACCAACAGCGTGTACGAGCCCTACTACCACAGCGATCACGCCGAGTCTAGCTGGGTCAAC
AGAGGCGAAAGCAGCAGAAAGGCCTACGACCACAACAGCCCCTACATCTGGCCCAGAAACGACTAC
GACGGCTTCCTGGAAAATGCCCACGAACACCACGGCGTGTACAATCAAGGCAGAGGCATCGATAGC
GGCGAGAGACTGATGCAGCCTACACAGATGAGCGCCCAAGAGGATCTGGGCGACGATACAGGCATC
CACGTGATCCCTACACTGAACGGCGACGACCGGCACAAGATCGTGAATGTGGATCAGAGACAGTAC
GGCGACGTGTTCAAGGGCGATCTGAACCCTAAGCCTCAGGGCCAGCGCCTGATCGAAGTGTCCGTG
GAAGAAAATCACCCCTTCACACTGAGAGCCCCTATCCAGAGAATCTACGGCGTGCGCTATACCGAG
ACATGGTCCTTTCTGCCTAGCCTGACATGTACTGGCGACGCCGCTCCTGCCATCCAGCACATTTGT
CTGAAGCACACCACCTGTTTCCAGGACGTGGTGGATGTGGACTGCGCCGAGAACACCAAAGAG
GATCAGCTGGCCGAGATCAGCTACCGGTTCCAGGGAAAGAAAGAGGCCGACCAGCCTTGGATCGTG
GTCAACACCAGCACACTGTTCGACGAGCTGGAACTGGACCCTCCTGAGATTGAGCCTGGGGTGCTG
AAGGTGCTGAGAACCGAGAAGCAGTACCTGGGAGTGTACATCTGGAACATGAGAGGCTCCGACGGC
ACCAGCACCTACGCCACATTTCTGGTTACCTGGAAGGGCGACGAAAAGACCAGAAATCCCACACCA
GCCGTGACACCTCAACCTAGAGGCGCCGAGTTTCACATGTGGAATTACCACAGCCACGTGTTCAGC
GTGGGCGATACCTTTAGCCTGGCCATGCATCTGCAGTACAAGATCCACGAGGCCCCTTTCGACCTG
CTGCTGGAATGGCTGTACGTGCCCATCGATCCTACCTGCCAGCCTATGAGACTGTACAGCACCTGT
CTGTATCACCCCAACGCTCCTCAGTGCCTGAGCCACATGAATAGCGGCTGCACCTTCACAAGCCCT
CACCTGGCTCAGAGAGTGGCCAGCACAGTCTACCAGAATTGCGAGCACGCCGACAATTACACCGCC
TACTGTCTGGGCATCAGCCACATGGAACCTAGCTTCGGCCTGATCCTGCACGATGGCGGCACCACA
CTGAAGTTCGTGGATACACCTGAGAGCCTGAGCGGCCTGTATGTGTTTGTGGTGTACTTCAACGGC
CACGTGGAAGCCGTGGCCTACACTGTGGTGTCCACCGTGGATCACTTCGTGAACGCCATCGAGGAA
CGGGGATTTCCACCAACTGCTGGACAGCCTCCTGCCACCACCAAGCCTAAAGAAATCACACCCGTG
AATCCCGGCACCTCTCCACTGCTTAGATATGCCGCTTGGACAGGCGGACTGGCTGCTGTGGTTCTG
 
CTGTGCCTGGTCATCTTCCTGATCTGCACCGCCAAGCGGATGAGAGTGAAGGCCGCAAGAGTGGAC
AAGTGA
SEQ ID NO: 16 
ATGGGCACAGTGAACAAGCCTGTTGTGGGCGTGCTGATGGGCTTCGGCATCATCACAGGCACCCTG
AGAATCACCAATCCTGTGCGGGCCAGCGTGCTGAGATACGACGATTTCCACATCGACGAGGACAAG
CTGGACACCAACAGCGTGTACGAGCCCTACTACCACAGCGATCACGCCGAGTCTAGCTGGGTCAAC
AGAGGCGAAAGCAGCAGAAAGGCCTACGACCACAACAGCCCCTACATCTGGCCCAGAAACGACTAC
GACGGCTTCCTGGAAAATGCCCACGAACACCACGGCGTGTACAATCAAGGCAGAGGCATCGATAGC
GGCGAGAGACTGATGCAGCCTACACAGATGAGCGCCCAAGAGGATCTGGGCGACGATACAGGCATC
CACGTGATCCCTACACTGAACGGCGACGACCGGCACAAGATCGTGAATGTGGATCAGAGACAGTAC
GGCGACGTGTTCAAGGGCGATCTGAACCCTAAGCCTCAGGGCCAGCGCCTGATCGAAGTGTCCGTG
GAAGAAAATCACCCCTTCACACTGAGAGCCCCTATCCAGAGAATCTACGGCGTGCGCTATACCGAG
ACATGGTCCTTTCTGCCTAGCCTGACATGTACTGGCGACGCCGCTCCTGCCATCCAGCACATTTGT
CTGAAGCACACCACCTGTTTCCAGGACGTGGTGGATGTGGACTGCGCCGAGAACACCAAAGAG
GATCAGCTGGCCGAGATCAGCTACCGGTTCCAGGGAAAGAAAGAGGCCGACCAGCCTTGGATCGTG
GTCAACACCAGCACACTGTTCGACGAGCTGGAACTGGACCCTCCTGAGATTGAGCCTGGGGTGCTG
AAGGTGCTGAGAACCGAGAAGCAGTACCTGGGAGTGTACATCTGGAACATGAGAGGCTCCGACGGC
ACCAGCACCTACGCCACATTTCTGGTTACCTGGAAGGGCGACGAAAAGACCAGAAATCCCACACCA
GCCGTGACACCTCAACCTAGAGGCGCCGAGTTTCACATGTGGAATTACCACAGCCACGTGTTCAGC
GTGGGCGATACCTTTAGCCTGGCCATGCATCTGCAGTACAAGATCCACGAGGCCCCTTTCGACCTG
CTGCTGGAATGGCTGTACGTGCCCATCGATCCTACCTGCCAGCCTATGAGACTGTACAGCACCTGT
CTGTATCACCCCAACGCTCCTCAGTGCCTGAGCCACATGAATAGCGGCTGCACCTTCACAAGCCCT
CACCTGGCTCAGAGAGTGGCCAGCACAGTCTACCAGAATTGCGAGCACGCCGACAATTACACCGCC
TACTGTCTGGGCATCAGCCACATGGAACCTAGCTTCGGCCTGATCCTGCACGATGGCGGCACCACA
CTGAAGTTCGTGGATACACCTGAGAGCCTGAGCGGCCTGTATGTGTTTGTGGTGTACTTCAACGGC
CACGTGGAAGCCGTGGCCTACACTGTGGTGTCCACCGTGGATCACTTCGTGAACGCCATCGAGGAA
CGGGGATTTCCACCAACTGCTGGACAGCCTCCTGCCACCACCAAGCCTAAAGAAATCACACCCGTG
AATCCCGGCACCTCTCCACTGCTTAGATATGCCGCTTGGACAGGCTGA
SEQ ID NO: 17 
AGCGTGCTGAGATACGACGATTTCCACATCGACGAGGACAAGCTGGACACCAACAGCGTGTACGAG
CCCTACTACCACAGCGATCACGCCGAGTCTAGCTGGGTCAACAGAGGCGAAAGCAGCAGAAAGGCC
TACGACCACAACAGCCCCTACATCTGGCCCAGAAACGACTACGACGGCTTCCTGGAAAATGCCCAC
 
GAACACCACGGCGTGTACAATCAAGGCAGAGGCATCGATAGCGGCGAGAGACTGATGCAGCCTACA
CAGATGAGCGCCCAAGAGGATCTGGGCGACGATACAGGCATCCACGTGATCCCTACACTGAACGGC
GACGACCGGCACAAGATCGTGAATGTGGATCAGAGACAGTACGGCGACGTGTTCAAGGGCGATCTG
AACCCTAAGCCTCAGGGCCAGCGCCTGATCGAAGTGTCCGTGGAAGAAAATCACCCCTTCACACTG
AGAGCCCCTATCCAGAGAATCTACGGCGTGCGCTATACCGAGACATGGTCCTTTCTGCCTAGCCTG
ACATGTACTGGCGACGCCGCTCCTGCCATCCAGCACATTTGTCTGAAGCACACCACCTGTTTCCAG
GACGTGGTGGTGGATGTGGACTGCGCCGAGAACACCAAAGAGGATCAGCTGGCCGAGATCAGCTAC
CGGTTCCAGGGAAAGAAAGAGGCCGACCAGCCTTGGATCGTGGTCAACACCAGCACACTGTTCGAC
GAGCTGGAACTGGACCCTCCTGAGATTGAGCCTGGGGTGCTGAAGGTGCTGAGAACCGAGAAGCAG
TACCTGGGAGTGTACATCTGGAACATGAGAGGCTCCGACGGCACCAGCACCTACGCCACATTTCTG
GTTACCTGGAAGGGCGACGAAAAGACCAGAAATCCCACACCAGCCGTGACACCTCAACCTAGAGGC
GCCGAGTTTCACATGTGGAATTACCACAGCCACGTGTTCAGCGTGGGCGATACCTTTAGCCTGGCC
ATGCATCTGCAGTACAAGATCCACGAGGCCCCTTTCGACCTGCTGCTGGAATGGCTGTACGTGCCC
ATCGATCCTACCTGCCAGCCTATGAGACTGTACAGCACCTGTCTGTATCACCCCAACGCTCCTCAG
TGCCTGAGCCACATGAATAGCGGCTGCACCTTCACAAGCCCTCACCTGGCTCAGAGAGTGGCCAGC
ACAGTCTACCAGAATTGCGAGCACGCCGACAATTACACCGCCTACTGTCTGGGCATCAGCCACATG
GAACCTAGCTTCGGCCTGATCCTGCACGATGGCGGCACCACACTGAAGTTCGTGGATACACCTGAG
AGCCTGAGCGGCCTGTATGTGTTTGTGGTGTACTTCAACGGCCACGTGGAAGCCGTGGCCTACACT
GTGGTGTCCACCGTGGATCACTTCGTGAACGCCATCGAGGAACGGGGATTTCCACCAACTGCTGGA
CAGCCTCCTGCCACCACCAAGCCTAAAGAAATCACACCCGTGAATCCCGGCACCTCTCCACTGCTT
AGATATGCCGCTTGGACAGGCGGACTGGCTGCTGTGGTTCTGCTGTGCCTGGTCATCTTCCTGATC
TGCACCGCCAAGCGGATGAGAGTGAAGGCCGCAAGAGTGGACAAGTGA
SEQ ID NO: 18 
AGCGTGCTGAGATACGACGATTTCCACATCGACGAGGACAAGCTGGACACCAACAGCGTGTACGAG
CCCTACTACCACAGCGATCACGCCGAGTCTAGCTGGGTCAACAGAGGCGAAAGCAGCAGAAAGGCC
TACGACCACAACAGCCCCTACATCTGGCCCAGAAACGACTACGACGGCTTCCTGGAAAATGCCCAC
GAACACCACGGCGTGTACAATCAAGGCAGAGGCATCGATAGCGGCGAGAGACTGATGCAGCCTACA
CAGATGAGCGCCCAAGAGGATCTGGGCGACGATACAGGCATCCACGTGATCCCTACACTGAACGGC
GACGACCGGCACAAGATCGTGAATGTGGATCAGAGACAGTACGGCGACGTGTTCAAGGGCGATCTG
AACCCTAAGCCTCAGGGCCAGCGCCTGATCGAAGTGTCCGTGGAAGAAAATCACCCCTTCACACTG
AGAGCCCCTATCCAGAGAATCTACGGCGTGCGCTATACCGAGACATGGTCCTTTCTGCCTAGCCTG
ACATGTACTGGCGACGCCGCTCCTGCCATCCAGCACATTTGTCTGAAGCACACCACCTGTTTCCAG
GACGTGGTGGTGGATGTGGACTGCGCCGAGAACACCAAAGAGGATCAGCTGGCCGAGATCAGCTAC
CGGTTCCAGGGAAAGAAAGAGGCCGACCAGCCTTGGATCGTGGTCAACACCAGCACACTGTTCGAC
 
GAGCTGGAACTGGACCCTCCTGAGATTGAGCCTGGGGTGCTGAAGGTGCTGAGAACCGAGAAGCAG
TACCTGGGAGTGTACATCTGGAACATGAGAGGCTCCGACGGCACCAGCACCTACGCCACATTTCTG
GTTACCTGGAAGGGCGACGAAAAGACCAGAAATCCCACACCAGCCGTGACACCTCAACCTAGAGGC
GCCGAGTTTCACATGTGGAATTACCACAGCCACGTGTTCAGCGTGGGCGATACCTTTAGCCTGGCC
ATGCATCTGCAGTACAAGATCCACGAGGCCCCTTTCGACCTGCTGCTGGAATGGCTGTACGTGCCC
ATCGATCCTACCTGCCAGCCTATGAGACTGTACAGCACCTGTCTGTATCACCCCAACGCTCCTCAG
TGCCTGAGCCACATGAATAGCGGCTGCACCTTCACAAGCCCTCACCTGGCTCAGAGAGTGGCCAGC
ACAGTCTACCAGAATTGCGAGCACGCCGACAATTACACCGCCTACTGTCTGGGCATCAGCCACATG
GAACCTAGCTTCGGCCTGATCCTGCACGATGGCGGCACCACACTGAAGTTCGTGGATACACCTGAG
AGCCTGAGCGGCCTGTATGTGTTTGTGGTGTACTTCAACGGCCACGTGGAAGCCGTGGCCTACACT
GTGGTGTCCACCGTGGATCACTTCGTGAACGCCATCGAGGAACGGGGATTTCCACCAACTGCTGGA
CAGCCTCCTGCCACCACCAAGCCTAAAGAAATCACACCCGTGAATCCCGGCACCTCTCCACTGCTT
AGATATGCCGCTTGGACAGGCTGA
SEQ ID NO: 19 
ATGGAGACAGACACACTCCTGCTATGGGTGCTGCTGCTCTGGGTTCCAGGTTCCACAGGTATGGGC
ACAGTGAACAAGCCTGTTGTGGGCGTGCTGATGGGCTTCGGCATCATCACAGGCACCCTGAGAATC
ACCAATCCTGTGCGGGCCAGCGTGCTGAGATACGACGATTTCCACATCGACGAGGACAAGCTGGAC
ACCAACAGCGTGTACGAGCCCTACTACCACAGCGATCACGCCGAGTCTAGCTGGGTCAACAGAGGC
GAAAGCAGCAGAAAGGCCTACGACCACAACAGCCCCTACATCTGGCCCAGAAACGACTACGACGGC
TTCCTGGAAAATGCCCACGAACACCACGGCGTGTACAATCAAGGCAGAGGCATCGATAGCGGCGAG
AGACTGATGCAGCCTACACAGATGAGCGCCCAAGAGGATCTGGGCGACGATACAGGCATCCACGTG
ATCCCTACACTGAACGGCGACGACCGGCACAAGATCGTGAATGTGGATCAGAGACAGTACGGCGAC
GTGTTCAAGGGCGATCTGAACCCTAAGCCTCAGGGCCAGCGCCTGATCGAAGTGTCCGTGGAAGAA
AATCACCCCTTCACACTGAGAGCCCCTATCCAGAGAATCTACGGCGTGCGCTATACCGAGACATGG
TCCTTTCTGCCTAGCCTGACATGTACTGGCGACGCCGCTCCTGCCATCCAGCACATTTGTCTGAAG
CACACCACCTGTTTCCAGGACGTGGTGGTGGATGTGGACTGCGCCGAGAACACCAAAGAGGATCAG
CTGGCCGAGATCAGCTACCGGTTCCAGGGAAAGAAAGAGGCCGACCAGCCTTGGATCGTGGTCAAC
ACCAGCACACTGTTCGACGAGCTGGAACTGGACCCTCCTGAGATTGAGCCTGGGGTGCTGAAGGTG
CTGAGAACCGAGAAGCAGTACCTGGGAGTGTACATCTGGAACATGAGAGGCTCCGACGGCACCAGC
ACCTACGCCACATTTCTGGTTACCTGGAAGGGCGACGAAAAGACCAGAAATCCCACACCAGCCGTG
ACACCTCAACCTAGAGGCGCCGAGTTTCACATGTGGAATTACCACAGCCACGTGTTCAGCGTGGGC
GATACCTTTAGCCTGGCCATGCATCTGCAGTACAAGATCCACGAGGCCCCTTTCGACCTGCTGCTG
GAATGGCTGTACGTGCCCATCGATCCTACCTGCCAGCCTATGAGACTGTACAGCACCTGTCTGTAT
CACCCCAACGCTCCTCAGTGCCTGAGCCACATGAATAGCGGCTGCACCTTCACAAGCCCTCACCTG
 
GCTCAGAGAGTGGCCAGCACAGTCTACCAGAATTGCGAGCACGCCGACAATTACACCGCCTACTGT
CTGGGCATCAGCCACATGGAACCTAGCTTCGGCCTGATCCTGCACGATGGCGGCACCACACTGAAG
TTCGTGGATACACCTGAGAGCCTGAGCGGCCTGTATGTGTTTGTGGTGTACTTCAACGGCCACGTG
GAAGCCGTGGCCTACACTGTGGTGTCCACCGTGGATCACTTCGTGAACGCCATCGAGGAACGGGGA
TTTCCACCAACTGCTGGACAGCCTCCTGCCACCACCAAGCCTAAAGAAATCACACCCGTGAATCCC
GGCACCTCTCCACTGCTTAGATATGCCGCTTGGACAGGCGGACTGGCTGCTGTGGTTCTGCTGTGC
CTGGTCATCTTCCTGATCTGCACCGCCAAGCGGATGAGAGTGAAGGCCTACAGAGTGGACAAGAGC
CCTTACAACCAGAGCATGTACTACGCCGGCCTGCCTGTGGACGATTTCGAGGATAGCGAGAGCACC
GACACCGAGGAAGAGTTTGGCAACGCCATTGGAGGCTCTCACGGCGGCAGCAGCTACACCGTGTAC
ATCGACAAGACCCGGTGA
SEQ ID NO: 20 
ATGGAGACAGACACACTCCTGCTATGGGTGCTGCTGCTCTGGGTTCCAGGTTCCACAGGTAGAATC
ACCAATCCTGTGCGGGCCAGCGTGCTGAGATACGACGATTTCCACATCGACGAGGACAAGCTGGAC
ACCAACAGCGTGTACGAGCCCTACTACCACAGCGATCACGCCGAGTCTAGCTGGGTCAACAGAGGC
GAAAGCAGCAGAAAGGCCTACGACCACAACAGCCCCTACATCTGGCCCAGAAACGACTACGACGGC
TTCCTGGAAAATGCCCACGAACACCACGGCGTGTACAATCAAGGCAGAGGCATCGATAGCGGCGAG
AGACTGATGCAGCCTACACAGATGAGCGCCCAAGAGGATCTGGGCGACGATACAGGCATCCACGTG
ATCCCTACACTGAACGGCGACGACCGGCACAAGATCGTGAATGTGGATCAGAGACAGTACGGCGAC
GTGTTCAAGGGCGATCTGAACCCTAAGCCTCAGGGCCAGCGCCTGATCGAAGTGTCCGTGGAAGAA
AATCACCCCTTCACACTGAGAGCCCCTATCCAGAGAATCTACGGCGTGCGCTATACCGAGACATGG
TCCTTTCTGCCTAGCCTGACATGTACTGGCGACGCCGCTCCTGCCATCCAGCACATTTGTCTGAAG
CACACCACCTGTTTCCAGGACGTGGTGGTGGATGTGGACTGCGCCGAGAACACCAAAGAGGATCAG
CTGGCCGAGATCAGCTACCGGTTCCAGGGAAAGAAAGAGGCCGACCAGCCTTGGATCGTGGTCAAC
ACCAGCACACTGTTCGACGAGCTGGAACTGGACCCTCCTGAGATTGAGCCTGGGGTGCTGAAGGTG
CTGAGAACCGAGAAGCAGTACCTGGGAGTGTACATCTGGAACATGAGAGGCTCCGACGGCACCAGC
ACCTACGCCACATTTCTGGTTACCTGGAAGGGCGACGAAAAGACCAGAAATCCCACACCAGCCGTG
ACACCTCAACCTAGAGGCGCCGAGTTTCACATGTGGAATTACCACAGCCACGTGTTCAGCGTGGGC
GATACCTTTAGCCTGGCCATGCATCTGCAGTACAAGATCCACGAGGCCCCTTTCGACCTGCTGCTG
GAATGGCTGTACGTGCCCATCGATCCTACCTGCCAGCCTATGAGACTGTACAGCACCTGTCTGTAT
CACCCCAACGCTCCTCAGTGCCTGAGCCACATGAATAGCGGCTGCACCTTCACAAGCCCTCACCTG
GCTCAGAGAGTGGCCAGCACAGTCTACCAGAATTGCGAGCACGCCGACAATTACACCGCCTACTGT
CTGGGCATCAGCCACATGGAACCTAGCTTCGGCCTGATCCTGCACGATGGCGGCACCACACTGAAG
TTCGTGGATACACCTGAGAGCCTGAGCGGCCTGTATGTGTTTGTGGTGTACTTCAACGGCCACGTG
GAAGCCGTGGCCTACACTGTGGTGTCCACCGTGGATCACTTCGTGAACGCCATCGAGGAACGGGGA
 
TTTCCACCAACTGCTGGACAGCCTCCTGCCACCACCAAGCCTAAAGAAATCACACCCGTGAATCCC
GGCACCTCTCCACTGCTTAGATATGCCGCTTGGACAGGCGGACTGGCTGCTGTGGTTCTGCTGTGC
CTGGTCATCTTCCTGATCTGCACCGCCAAGCGGATGAGAGTGAAGGCCTACAGAGTGGACAAGAGC
CCTTACAACCAGAGCATGTACTACGCCGGCCTGCCTGTGGACGATTTCGAGGATAGCGAGAGCACC
GACACCGAGGAAGAGTTTGGCAACGCCATTGGAGGCTCTCACGGCGGCAGCAGCTACACCGTGTAC
ATCGACAAGACCCGGTGA
SEQ ID NO: 21 
ATGGAGACAGACACACTCCTGCTATGGGTGCTGCTGCTCTGGGTTCCAGGTTCCACAGGTAGCGTG
CTGAGATACGACGATTTCCACATCGACGAGGACAAGCTGGACACCAACAGCGTGTACGAGCCCTAC
TACCACAGCGATCACGCCGAGTCTAGCTGGGTCAACAGAGGCGAAAGCAGCAGAAAGGCCTACGAC
CACAACAGCCCCTACATCTGGCCCAGAAACGACTACGACGGCTTCCTGGAAAATGCCCACGAACAC
CACGGCGTGTACAATCAAGGCAGAGGCATCGATAGCGGCGAGAGACTGATGCAGCCTACACAGATG
AGCGCCCAAGAGGATCTGGGCGACGATACAGGCATCCACGTGATCCCTACACTGAACGGCGACGAC
CGGCACAAGATCGTGAATGTGGATCAGAGACAGTACGGCGACGTGTTCAAGGGCGATCTGAACCCT
AAGCCTCAGGGCCAGCGCCTGATCGAAGTGTCCGTGGAAGAAAATCACCCCTTCACACTGAGAGCC
CCTATCCAGAGAATCTACGGCGTGCGCTATACCGAGACATGGTCCTTTCTGCCTAGCCTGACATGT
ACTGGCGACGCCGCTCCTGCCATCCAGCACATTTGTCTGAAGCACACCACCTGTTTCCAGGACGTG
GTGGTGGATGTGGACTGCGCCGAGAACACCAAAGAGGATCAGCTGGCCGAGATCAGCTACCGGTTC
CAGGGAAAGAAAGAGGCCGACCAGCCTTGGATCGTGGTCAACACCAGCACACTGTTCGACGAGCTG
GAACTGGACCCTCCTGAGATTGAGCCTGGGGTGCTGAAGGTGCTGAGAACCGAGAAGCAGTACCTG
GGAGTGTACATCTGGAACATGAGAGGCTCCGACGGCACCAGCACCTACGCCACATTTCTGGTTACC
TGGAAGGGCGACGAAAAGACCAGAAATCCCACACCAGCCGTGACACCTCAACCTAGAGGCGCCGAG
TTTCACATGTGGAATTACCACAGCCACGTGTTCAGCGTGGGCGATACCTTTAGCCTGGCCATGCAT
CTGCAGTACAAGATCCACGAGGCCCCTTTCGACCTGCTGCTGGAATGGCTGTACGTGCCCATCGAT
CCTACCTGCCAGCCTATGAGACTGTACAGCACCTGTCTGTATCACCCCAACGCTCCTCAGTGCCTG
AGCCACATGAATAGCGGCTGCACCTTCACAAGCCCTCACCTGGCTCAGAGAGTGGCCAGCACAGTC
TACCAGAATTGCGAGCACGCCGACAATTACACCGCCTACTGTCTGGGCATCAGCCACATGGAACCT
AGCTTCGGCCTGATCCTGCACGATGGCGGCACCACACTGAAGTTCGTGGATACACCTGAGAGCCTG
AGCGGCCTGTATGTGTTTGTGGTGTACTTCAACGGCCACGTGGAAGCCGTGGCCTACACTGTGGTG
TCCACCGTGGATCACTTCGTGAACGCCATCGAGGAACGGGGATTTCCACCAACTGCTGGACAGCCT
CCTGCCACCACCAAGCCTAAAGAAATCACACCCGTGAATCCCGGCACCTCTCCACTGCTTAGATAT
GCCGCTTGGACAGGCGGACTGGCTGCTGTGGTTCTGCTGTGCCTGGTCATCTTCCTGATCTGCACC
GCCAAGCGGATGAGAGTGAAGGCCTACAGAGTGGACAAGAGCCCTTACAACCAGAGCATGTACTAC
GCCGGCCTGCCTGTGGACGATTTCGAGGATAGCGAGAGCACCGACACCGAGGAAGAGTTTGGCAAC
 GCCATTGGAGGCTCTCACGGCGGCAGCAGCTACACCGTGTACATCGACAAGACCCGGTGA
SEQ ID NO: 22 
ATGGGCACAGTGAACAAGCCTGTTGTGGGCGTGCTGATGGGCTTCGGCATCATCACAGGCACCCTG
AGAATCACCAATCCTGTGCGGGCCAGCGTGCTGAGATACGACGATTTCCACATCGACGAGGACAAG
CTGGACACCAACAGCGTGTACGAGCCCTACTACCACAGCGATCACGCCGAGTCTAGCTGGGTCAAC
AGAGGCGAAAGCAGCAGAAAGGCCTACGACCACAACAGCCCCTACATCTGGCCCAGAAACGACTAC
GACGGCTTCCTGGAAAATGCCCACGAACACCACGGCGTGTACAATCAAGGCAGAGGCATCGATAGC
GGCGAGAGACTGATGCAGCCTACACAGATGAGCGCCCAAGAGGATCTGGGCGACGATACAGGCATC
CACGTGATCCCTACACTGAACGGCGACGACCGGCACAAGATCGTGAATGTGGATCAGAGACAGTAC
GGCGACGTGTTCAAGGGCGATCTGAACCCTAAGCCTCAGGGCCAGCGCCTGATCGAAGTGTCCGTG
GAAGAAAATCACCCCTTCACACTGAGAGCCCCTATCCAGAGAATCTACGGCGTGCGCTATACCGAG
ACATGGTCCTTTCTGCCTAGCCTGACATGTACTGGCGACGCCGCTCCTGCCATCCAGCACATTTGT
CTGAAGCACACCACCTGTTTCCAGGACGTGGTGGTGGATGTGGACTGCGCCGAGAACACCAAAGAG
GATCAGCTGGCCGAGATCAGCTACCGGTTCCAGGGAAAGAAAGAGGCCGACCAGCCTTGGATCGTG
GTCAACACCAGCACACTGTTCGACGAGCTGGAACTGGACCCTCCTGAGATTGAGCCTGGGGTGCTG
AAGGTGCTGAGAACCGAGAAGCAGTACCTGGGAGTGTACATCTGGAACATGAGAGGCTCCGACGGC
ACCAGCACCTACGCCACATTTCTGGTTACCTGGAAGGGCGACGAAAAGACCAGAAATCCCACACCA
GCCGTGACACCTCAACCTAGAGGCGCCGAGTTTCACATGTGGAATTACCACAGCCACGTGTTCAGC
GTGGGCGATACCTTTAGCCTGGCCATGCATCTGCAGTACAAGATCCACGAGGCCCCTTTCGACCTG
CTGCTGGAATGGCTGTACGTGCCCATCGATCCTACCTGCCAGCCTATGAGACTGTACAGCACCTGT
CTGTATCACCCCAACGCTCCTCAGTGCCTGAGCCACATGAATAGCGGCTGCACCTTCACAAGCCCT
CACCTGGCTCAGAGAGTGGCCAGCACAGTCTACCAGAATTGCGAGCACGCCGACAATTACACCGCC
TACTGTCTGGGCATCAGCCACATGGAACCTAGCTTCGGCCTGATCCTGCACGATGGCGGCACCACA
CTGAAGTTCGTGGATACACCTGAGAGCCTGAGCGGCCTGTATGTGTTTGTGGTGTACTTCAACGGC
CACGTGGAAGCCGTGGCCTACACTGTGGTGTCCACCGTGGATCACTTCGTGAACGCCATCGAGGAA
CGGGGATTTCCACCAACTGCTGGACAGCCTCCTGCCACCACCAAGCCTAAAGAAATCACACCCGTG
AATCCCGGCACCTCTCCACTGCTTAGATATGCCGCTTGGACAGGCAGCGCCATCGGCATGGAATCT
CAAGTGCGGCAGCAGTTCAGCAAGGACATCGAGAAGCTGCTGAACGAGCAAGTGAACAAAGAGATG
AACAGCAGCAACCTGTACATGAGCATGAGCAGCTGGTGCTACACCCACAGTCTGGATGGCGCCGGA
CTGTTCCTGTTTGATCACGCCGCCGAGGAATACGAGCACGCCAAGAAGCTGATCATCTTCCTGAAC
GAGAACAACGTGCCCGTGCAGCTGACATCTATCAGCGCCCCTGAGCACAAGTTCGAGGGCCTGACA
CAGATCTTCCAGAAGGCCTACGAACACGAGCAGCACATCAGCGAGAGCATCAACAACATCGTGGAC
CACGCCATCAAGAGCAAGGATCACGCCACCTTCAACTTTCTGCAGTGGTACGTGGCCGAACAGCAC
GAGGAAGAGGTGCTGTTTAAGGACATCCTGGACAAGATCGAGCTGATCGGCAACGAGAACCACGGC
 CTGTACCTGGCCGATCAGTATGTGAAGGGAATCGCCAAGAGCCGGAAGTCCTGATGA
SEQ ID NO: 23 
ATGGAGACAGACACACTCCTGCTATGGGTGCTGCTGCTCTGGGTTCCAGGTTCCACAGGTAGCGTG
CTGAGATACGACGATTTCCACATCGACGAGGACAAGCTGGACACCAACAGCGTGTACGAGCCCTAC
TACCACAGCGATCACGCCGAGTCTAGCTGGGTCAACAGAGGCGAAAGCAGCAGAAAGGCCTACGAC
CACAACAGCCCCTACATCTGGCCCAGAAACGACTACGACGGCTTCCTGGAAAATGCCCACGAACAC
CACGGCGTGTACAATCAAGGCAGAGGCATCGATAGCGGCGAGAGACTGATGCAGCCTACACAGATG
AGCGCCCAAGAGGATCTGGGCGACGATACAGGCATCCACGTGATCCCTACACTGAACGGCGACGAC
CGGCACAAGATCGTGAATGTGGATCAGAGACAGTACGGCGACGTGTTCAAGGGCGATCTGAACCCT
AAGCCTCAGGGCCAGCGCCTGATCGAAGTGTCCGTGGAAGAAAATCACCCCTTCACACTGAGAGCC
CCTATCCAGAGAATCTACGGCGTGCGCTATACCGAGACATGGTCCTTTCTGCCTAGCCTGACATGT
ACTGGCGACGCCGCTCCTGCCATCCAGCACATTTGTCTGAAGCACACCACCTGTTTCCAGGACGTG
GTGGTGGATGTGGACTGCGCCGAGAACACCAAAGAGGATCAGCTGGCCGAGATCAGCTACCGGTTC
CAGGGAAAGAAAGAGGCCGACCAGCCTTGGATCGTGGTCAACACCAGCACACTGTTCGACGAGCTG
GAACTGGACCCTCCTGAGATTGAGCCTGGGGTGCTGAAGGTGCTGAGAACCGAGAAGCAGTACCTG
GGAGTGTACATCTGGAACATGAGAGGCTCCGACGGCACCAGCACCTACGCCACATTTCTGGTTACC
TGGAAGGGCGACGAAAAGACCAGAAATCCCACACCAGCCGTGACACCTCAACCTAGAGGCGCCGAG
TTTCACATGTGGAATTACCACAGCCACGTGTTCAGCGTGGGCGATACCTTTAGCCTGGCCATGCAT
CTGCAGTACAAGATCCACGAGGCCCCTTTCGACCTGCTGCTGGAATGGCTGTACGTGCCCATCGAT
CCTACCTGCCAGCCTATGAGACTGTACAGCACCTGTCTGTATCACCCCAACGCTCCTCAGTGCCTG
AGCCACATGAATAGCGGCTGCACCTTCACAAGCCCTCACCTGGCTCAGAGAGTGGCCAGCACAGTC
TACCAGAATTGCGAGCACGCCGACAATTACACCGCCTACTGTCTGGGCATCAGCCACATGGAACCT
AGCTTCGGCCTGATCCTGCACGATGGCGGCACCACACTGAAGTTCGTGGATACACCTGAGAGCCTG
AGCGGCCTGTATGTGTTTGTGGTGTACTTCAACGGCCACGTGGAAGCCGTGGCCTACACTGTGGTG
TCCACCGTGGATCACTTCGTGAACGCCATCGAGGAACGGGGATTTCCACCAACTGCTGGACAGCCT
CCTGCCACCACCAAGCCTAAAGAAATCACACCCGTGAATCCCGGCACCTCTCCACTGCTTAGATAT
GCCGCTTGGACAGGCGGACTGGCTGCTGTGGTTCTGCTGTGCCTGGTCATCTTCCTGATCTGCACC
GCCAAGCGGATGAGAGTGAAGGCCGCAAGAGTGGACAAGTGA
SEQ ID NO: 24 
ATGGAGACAGACACACTCCTGCTATGGGTGCTGCTGCTCTGGGTTCCAGGTTCCACAGGTAGCGTG
CTGAGATACGACGATTTCCACATCGACGAGGACAAGCTGGACACCAACAGCGTGTACGAGCCCTAC
TACCACAGCGATCACGCCGAGTCTAGCTGGGTCAACAGAGGCGAAAGCAGCAGAAAGGCCTACGAC
 
CACAACAGCCCCTACATCTGGCCCAGAAACGACTACGACGGCTTCCTGGAAAATGCCCACGAACAC
CACGGCGTGTACAATCAAGGCAGAGGCATCGATAGCGGCGAGAGACTGATGCAGCCTACACAGATG
AGCGCCCAAGAGGATCTGGGCGACGATACAGGCATCCACGTGATCCCTACACTGAACGGCGACGAC
CGGCACAAGATCGTGAATGTGGATCAGAGACAGTACGGCGACGTGTTCAAGGGCGATCTGAACCCT
AAGCCTCAGGGCCAGCGCCTGATCGAAGTGTCCGTGGAAGAAAATCACCCCTTCACACTGAGAGCC
CCTATCCAGAGAATCTACGGCGTGCGCTATACCGAGACATGGTCCTTTCTGCCTAGCCTGACATGT
ACTGGCGACGCCGCTCCTGCCATCCAGCACATTTGTCTGAAGCACACCACCTGTTTCCAGGACGTG
GTGGTGGATGTGGACTGCGCCGAGAACACCAAAGAGGATCAGCTGGCCGAGATCAGCTACCGGTTC
CAGGGAAAGAAAGAGGCCGACCAGCCTTGGATCGTGGTCAACACCAGCACACTGTTCGACGAGCTG
GAACTGGACCCTCCTGAGATTGAGCCTGGGGTGCTGAAGGTGCTGAGAACCGAGAAGCAGTACCTG
GGAGTGTACATCTGGAACATGAGAGGCTCCGACGGCACCAGCACCTACGCCACATTTCTGGTTACC
TGGAAGGGCGACGAAAAGACCAGAAATCCCACACCAGCCGTGACACCTCAACCTAGAGGCGCCGAG
TTTCACATGTGGAATTACCACAGCCACGTGTTCAGCGTGGGCGATACCTTTAGCCTGGCCATGCAT
CTGCAGTACAAGATCCACGAGGCCCCTTTCGACCTGCTGCTGGAATGGCTGTACGTGCCCATCGAT
CCTACCTGCCAGCCTATGAGACTGTACAGCACCTGTCTGTATCACCCCAACGCTCCTCAGTGCCTG
AGCCACATGAATAGCGGCTGCACCTTCACAAGCCCTCACCTGGCTCAGAGAGTGGCCAGCACAGTC
TACCAGAATTGCGAGCACGCCGACAATTACACCGCCTACTGTCTGGGCATCAGCCACATGGAACCT
AGCTTCGGCCTGATCCTGCACGATGGCGGCACCACACTGAAGTTCGTGGATACACCTGAGAGCCTG
AGCGGCCTGTATGTGTTTGTGGTGTACTTCAACGGCCACGTGGAAGCCGTGGCCTACACTGTGGTG
TCCACCGTGGATCACTTCGTGAACGCCATCGAGGAACGGGGATTTCCACCAACTGCTGGACAGCCT
CCTGCCACCACCAAGCCTAAAGAAATCACACCCGTGAATCCCGGCACCTCTCCACTGCTTAGATAT
GCCGCTTGGACAGGCAGCGCCATCGGCATGGAATCTCAAGTGCGGCAGCAGTTCAGCAAGGACATC
GAGAAGCTGCTGAACGAGCAAGTGAACAAAGAGATGAACAGCAGCAACCTGTACATGAGCATGAGC
AGCTGGTGCTACACCCACAGTCTGGATGGCGCCGGACTGTTCCTGTTTGATCACGCCGCCGAGGAA
TACGAGCACGCCAAGAAGCTGATCATCTTCCTGAACGAGAACAACGTGCCCGTGCAGCTGACATCT
ATCAGCGCCCCTGAGCACAAGTTCGAGGGCCTGACACAGATCTTCCAGAAGGCCTACGAACACGAG
CAGCACATCAGCGAGAGCATCAACAACATCGTGGACCACGCCATCAAGAGCAAGGATCACGCCACC
TTCAACTTTCTGCAGTGGTACGTGGCCGAACAGCACGAGGAAGAGGTGCTGTTTAAGGACATCCTG
GACAAGATCGAGCTGATCGGCAACGAGAACCACGGCCTGTACCTGGCCGATCAGTATGTGAAGGGA
ATCGCCAAGAGCCGGAAGTCCTGATGA
SEQ ID NO: 25 
TAATACGACTCACTATAGGACAGATCGCCTGGAGACGCCATCCACGCTGTTTTGACCTCCATAGAA
GACACCGGGACCGATCCAGCCTCCGCGGCCGGGAACGGTGCATTGGAACGCGGATTCCCCGTGCCA
AGAGTGACGTAAGTACCGCAGTCACCGTCCTTGACACG
SEQ ID NO: 26 
TCTTCACCCAGTCTGTCTCTTAGAATTTATTTCCCACTGACACAAACAAGCCATTTTGTCTGCTTG
TATAACCC
SEQ ID NO: 27 
AAAAAAAAAAAAGAAAAAAAAAAAAAAAAAAAGAAAGAAAAAAAAAAAAAAAAAAAAAAAAAAAAAAA

AAAAAAAAAAAAAAAAAAAAAAAAAAAAAAAAAAAAAAAAA
SEQ ID NO: 28 
METDTLLLWVLLLWVPGSTGMGTVNKPVVGVLMGFGIITGTLRITNPVRASVLRYDDFHIDEDKLD
TNSVYEPYYHSDHAESSWVNRGESSRKAYDHNSPYIWPRNDYDGFLENAHEHHGVYNQGRGIDSGE
RLMQPTQMSAQEDLGDDTGIHVIPTLNGDDRHKIVNVDQRQYGDVFKGDLNPKPQGQRLIEVSVEE
NHPFTLRAPIQRIYGVRYTETWSFLPSLTCTGDAAPAIQHICLKHTTCFQDVVVDVDCAENTKEDQ
LAEISYRFQGKKEADQPWIVVNTSTLFDELELDPPEIEPGVLKVLRTEKQYLGVYIWNMRGSDGTS
TYATFLVTWKGDEKTRNPTPAVTPQPRGAEFHMWNYHSHVFSVGDTFSLAMHLQYKIHEAPFDLLL
EWLYVPIDPTCQPMRLYSTCLYHPNAPQCLSHMNSGCTFTSPHLAQRVASTVYQNCEHADNYTAYC
LGISHMEPSFGLILHDGGTTLKFVDTPESLSGLYVFVVYFNGHVEAVAYTVVSTVDHFVNAIEERG
FPPTAGQPPATTKPKEITPVNPGTSPLLRYAAWTGGLAAVVLLCLVIFLICTAKRMRVKAYRVDKS
PYNQSMYYAGLPVDDFEDSESTDTEEEFGNAIGGSHGGSSYTVYIDKTR
SEQ ID NO: 29 
METDTLLLWVLLLWVPGSTGRITNPVRASVLRYDDFHIDEDKLDTNSVYEPYYHSDHAESSWVNRG
ESSRKAYDHNSPYIWPRNDYDGFLENAHEHHGVYNQGRGIDSGERLMQPTQMSAQEDLGDDTGIHV
IPTLNGDDRHKIVNVDQRQYGDVFKGDLNPKPQGQRLIEVSVEENHPFTLRAPIQRIYGVRYTETW
SFLPSLTCTGDAAPAIQHICLKHTTCFQDVVVDVDCAENTKEDQLAEISYRFQGKKEADQPWIVVN
TSTLFDELELDPPEIEPGVLKVLRTEKQYLGVYIWNMRGSDGTSTYATFLVTWKGDEKTRNPTPAV
TPQPRGAEFHMWNYHSHVFSVGDTFSLAMHLQYKIHEAPFDLLLEWLYVPIDPTCQPMRLYSTCLY
HPNAPQCLSHMNSGCTFTSPHLAQRVASTVYQNCEHADNYTAYCLGISHMEPSFGLILHDGGTTLK
FVDTPESLSGLYVFVVYFNGHVEAVAYTVVSTVDHFVNAIEERGFPPTAGQPPATTKPKEITPVNP
GTSPLLRYAAWTGGLAAVVLLCLVIFLICTAKRMRVKAYRVDKSPYNQSMYYAGLPVDDFEDSEST
DTEEEFGNAIGGSHGGSSYTVYIDKTR
SEQ ID NO: 30 
METDTLLLWVLLLWVPGSTGSVLRYDDFHIDEDKLDTNSVYEPYYHSDHAESSWVNRGESSRKAYD
HNSPYIWPRNDYDGFLENAHEHHGVYNQGRGIDSGERLMQPTQMSAQEDLGDDTGIHVIPTLNGDD
RHKIVNVDQRQYGDVFKGDLNPKPQGQRLIEVSVEENHPFTLRAPIQRIYGVRYTETWSFLPSLTC
TGDAAPAIQHICLKHTTCFQDVVVDVDCAENTKEDQLAEISYRFQGKKEADQPWIVVNTSTLFDEL
ELDPPEIEPGVLKVLRTEKQYLGVYIWNMRGSDGTSTYATFLVTWKGDEKTRNPTPAVTPQPRGAE
FHMWNYHSHVFSVGDTFSLAMHLQYKIHEAPFDLLLEWLYVPIDPTCQPMRLYSTCLYHPNAPQCL
SHMNSGCTFTSPHLAQRVASTVYQNCEHADNYTAYCLGISHMEPSFGLILHDGGTTLKFVDTPESL
SGLYVFVVYFNGHVEAVAYTVVSTVDHFVNAIEERGFPPTAGQPPATTKPKEITPVNPGTSPLLRY
AAWTGGLAAVVLLCLVIFLICTAKRMRVKAYRVDKSPYNQSMYYAGLPVDDFEDSESTDTEEEFGN
AIGGSHGGSSYTVYIDKTR
SEQ ID NO: 31 
MGTVNKPVVGVLMGFGIITGTLRITNPVRASVLRYDDFHIDEDKLDTNSVYEPYYHSDHAESSWVN
RGESSRKAYDHNSPYIWPRNDYDGFLENAHEHHGVYNQGRGIDSGERLMQPTQMSAQEDLGDDTGI
HVIPTLNGDDRHKIVNVDQRQYGDVFKGDLNPKPQGQRLIEVSVEENHPFTLRAPIQRIYGVRYTE
TWSFLPSLTCTGDAAPAIQHICLKHTTCFQDVVVDVDCAENTKEDQLAEISYRFQGKKEADQPWIV
VNTSTLFDELELDPPEIEPGVLKVLRTEKQYLGVYIWNMRGSDGTSTYATFLVTWKGDEKTRNPTP
AVTPQPRGAEFHMWNYHSHVFSVGDTFSLAMHLQYKIHEAPFDLLLEWLYVPIDPTCQPMRLYSTC
LYHPNAPQCLSHMNSGCTFTSPHLAQRVASTVYQNCEHADNYTAYCLGISHMEPSFGLILHDGGTT
LKFVDTPESLSGLYVFVVYFNGHVEAVAYTVVSTVDHFVNAIEERGFPPTAGQPPATTKPKEITPV
NPGTSPLLRYAAWTGSAIGMESQVRQQFSKDIEKLLNEQVNKEMNSSNLYMSMSSWCYTHSLDGAG
LFLFDHAAEEYEHAKKLIIFLNENNVPVQLTSISAPEHKFEGLTQIFQKAYEHEQHISESINNIVD
HAIKSKDHATFNFLQWYVAEQHEEEVLFKDILDKIELIGNENHGLYLADQYVKGIAKSRKS
SEQ ID NO: 32 
METDTLLLWVLLLWVPGSTGSVLRYDDFHIDEDKLDTNSVYEPYYHSDHAESSWVNRGESSRKAYD
HNSPYIWPRNDYDGFLENAHEHHGVYNQGRGIDSGERLMQPTQMSAQEDLGDDTGIHVIPTLNGDD
RHKIVNVDQRQYGDVFKGDLNPKPQGQRLIEVSVEENHPFTLRAPIQRIYGVRYTETWSFLPSLTC
TGDAAPAIQHICLKHTTCFQDVVVDVDCAENTKEDQLAEISYRFQGKKEADQPWIVVNTSTLFDEL
ELDPPEIEPGVLKVLRTEKQYLGVYIWNMRGSDGTSTYATFLVTWKGDEKTRNPTPAVTPQPRGAE
FHMWNYHSHVFSVGDTFSLAMHLQYKIHEAPFDLLLEWLYVPIDPTCQPMRLYSTCLYHPNAPQCL
SHMNSGCTFTSPHLAQRVASTVYQNCEHADNYTAYCLGISHMEPSFGLILHDGGTTLKFVDTPESL
SGLYVFVVYFNGHVEAVAYTVVSTVDHFVNAIEERGFPPTAGQPPATTKPKEITPVNPGTSPLLRY
AAWTGGLAAVVLLCLVIFLICTAKRMRVKAARVDK
SEQ ID NO: 33 
METDTLLLWVLLLWVPGSTGSVLRYDDFHIDEDKLDTNSVYEPYYHSDHAESSWVNRGESSRKAYD
HNSPYIWPRNDYDGFLENAHEHHGVYNQGRGIDSGERLMQPTQMSAQEDLGDDTGIHVIPTLNGDD
RHKIVNVDQRQYGDVFKGDLNPKPQGQRLIEVSVEENHPFTLRAPIQRIYGVRYTETWSFLPSLTC
TGDAAPAIQHICLKHTTCFQDVVVDVDCAENTKEDQLAEISYRFQGKKEADQPWIVVNTSTLFDEL
ELDPPEIEPGVLKVLRTEKQYLGVYIWNMRGSDGTSTYATFLVTWKGDEKTRNPTPAVTPQPRGAE
FHMWNYHSHVFSVGDTFSLAMHLQYKIHEAPFDLLLEWLYVPIDPTCQPMRLYSTCLYHPNAPQCL
SHMNSGCTFTSPHLAQRVASTVYQNCEHADNYTAYCLGISHMEPSFGLILHDGGTTLKFVDTPESL
SGLYVFVVYFNGHVEAVAYTVVSTVDHFVNAIEERGFPPTAGQPPATTKPKEITPVNPGTSPLLRY
AAWTGSAIGMESQVRQQFSKDIEKLLNEQVNKEMNSSNLYMSMSSWCYTHSLDGAGLFLFDHAAEE
YEHAKKLIIFLNENNVPVQLTSISAPEHKFEGLTQIFQKAYEHEQHISESINNIVDHAIKSKDHAT
FNFLQWYVAEQHEEEVLFKDILDKIELIGNENHGLYLADQYVKGIAKSRKS
SEQ ID NO: 34
   ATGGAGACAGACACACTCCTGCTATGGGTGCTGCTGCTCTGGGTTCCAGGTTCCACAGGT
SEQ ID NO: 35

ATGGAATCTCAAGTGCGGCAGCAGTTCAGCAAGGACATCGAGAAGCTGCTGAACGAGCAAGTGAAC
AAAGAGATGAACAGCAGCAACCTGTACATGAGCATGAGCAGCTGGTGCTACACCCACAGTCTGGAT
GGCGCCGGACTGTTCCTGTTTGATCACGCCGCCGAGGAATACGAGCACGCCAAGAAGCTGATCATC
TTCCTGAACGAGAACAACGTGCCCGTGCAGCTGACATCTATCAGCGCCCCTGAGCACAAGTTCGAG
GGCCTGACACAGATCTTCCAGAAGGCCTACGAACACGAGCAGCACATCAGCGAGAGCATCAACAAC
ATCGTGGACCACGCCATCAAGAGCAAGGATCACGCCACCTTCAACTTTCTGCAGTGGTACGTGGCC
GAACAGCACGAGGAAGAGGTGCTGTTTAAGGACATCCTGGACAAGATCGAGCTGATCGGCAACGAG
AACCACGGCCTGTACCTGGCCGATCAGTATGTGAAGGGAATCGCCAAGAGCCGGAAGTCCTGATGA

## Claims

1. A non-natural nucleic acid comprising a polynucleotide encoding a VZV gE protein or a fragment thereof.

2. The nucleic acid according to claim 1, wherein the amino acid sequence of the VZV gE protein comprises or is an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 1.

3. The nucleic acid according to claim 1, wherein the VZV gE protein is a full-length gE protein.

4. The nucleic acid according to claim 1, wherein the fragment of the VZV gE protein is one of the follows:
(1) a truncated polypeptide of positions 23 to 623, positions 31 to 623, positions 1 to 561, positions 1 to 573, positions 1 to 543, positions 31 to 573 or positions 31 to 543 of the VZVgE protein; and
(2) a polypeptide having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence of the truncated polypeptide of (1).

5. The nucleic acid according to any one of claims 1-4, wherein the VZV gE protein or a fragment thereof further has a mutation; preferably, the mutation is one or both of the mutations: Y569A and Y582G.

6. The nucleic acid according to any one of claims 1-5, wherein the VZV gE protein or a fragment thereof comprises or is one of the following polypeptides or proteins:
(1) a polypeptide or protein having an amino acid sequence as set forth in one of SEQ ID NOs: 1-8; and
(2) a polypeptide or protein comprising an amino acid sequence having at least 90%, 91%, 92%, 93%, 94%, 95%, 96%, 97%, 98% or 99% identity to one of the sequences SEQ ID NOs: 1-8.

7. The nucleic acid according to any one of claims 1-6, wherein the VZV gE protein or a fragment thereof is further linked to a heterologous signal peptide and/or ferritin.

8. The nucleic acid according to claim 7, wherein the heterologous signal peptide comprises one or more of: an IgE signal peptide and an IgGκ signal peptide;
preferably, the amino acid sequence of the IgGκ signal peptide is set forth in SEQ ID NO: 9;
and/or, the amino acid sequence of ferritin is set forth in SEQ ID NO: 10.

9. The nucleic acid according to any one of claims 1-6, wherein the nucleic acid is RNA;
preferably, the polynucleotide encoding the VZV gE protein or a fragment thereof is codon optimized;
preferably, the polynucleotide encoding the VZV gE protein or a fragment thereof comprises or is one of the follows:
(1) an RNA corresponding to a polynucleotide having a nucleotide sequence set forth in one of SEQ ID NOs: 11-18; and
(2) an RNA corresponding to a polynucleotide comprising a nucleotide sequence having at least 50%, 55%, 60%, 65%, 70%, 75%, 80%, 85%, 90%, 91%, 92%, 93%, 94%, 95%, 96%, 97%, 98% or 99% identity to the nucleotide sequence set forth in one of SEQ ID NOs: 11-18.

10. The nucleic acid according to any one of claims 1-9, wherein the nucleic acid is RNA, and the nucleic acid comprises or is one of the following RNAs:
(1) an RNA corresponding to a polynucleotide having a nucleotide sequence set forth in one of SEQ ID NOs: 19-24; and
(2) an RNA corresponding to a polynucleotide comprising a nucleotide sequence having at least 50%, 55%, 60%, 65%, 70%, 75%, 80%, 85%, 90%, 91%, 92%, 93%, 94%, 95%, 96%, 97%, 98% or 99% identity to the nucleotide sequence set forth in one of SEQ ID NOs: 19-24.

11. The nucleic acid according to claim 8-10, wherein the nucleic acid is mRNA; preferably, the mRNA comprises at least one of a 5'-cap structure, a 5'-UTR, a 3'-UTR and a poly(A) tail.

12. The nucleic acid according to claim 11, wherein the DNA sequence corresponding to the 5'-UTR is set forth in SEQ ID NO: 25;
and/or, the DNA sequence corresponding to the 3'-UTR is set forth in SEQ ID NO: 26;
and/or, the nucleotides constituting the poly(A) tail comprises at least 20, at least 40, at least 80, at least 100 or at least 120 A nucleotides; preferably, the nucleotides constituting the poly(A) tail comprises at least 20, at least 40, at least 80, at least 100 or at least 120 continuous A nucleotides; preferably, the nucleotides constituting the poly(A) tail comprises one or more nucleotides other than the A nucleotide; more preferably, the DNA sequence corresponding to the poly(A) tail is set forth in SEQ ID NO: 27.

13. The nucleic acid according to any one of claims 1-8, wherein the nucleic acid is DNA; preferably, the DNA can be transcribed into RNA,
preferably, the polynucleotide encoding the VZV gE protein or a fragment thereof is codon optimized;
preferably, the polynucleotide encoding the VZV gE protein or a fragment thereof comprises or is one of the follows:
(1) a polynucleotide having a nucleotide sequence set forth in one of SEQ ID NOs: 11-18; and
(2) a polynucleotide comprising a nucleotide sequence having at least 50%, 55%, 60%, 65%, 70%, 75%, 80%, 85%, 90%, 91%, 92%, 93%, 94%, 95%, 96%, 97%, 98% or 99% identity to the nucleotide sequence set forth in one of SEQ ID NOs: 11-18.

14. The nucleic acid according to claim 13, comprising or being one of the following polynucleotides:
(1) a polynucleotide having a nucleotide sequence set forth in one of SEQ ID NOs: 19-24; and
(2) a polynucleotide comprising a nucleotide sequence having at least 50%, 55%, 60%, 65%, 70%, 75%, 80%, 85%, 90%, 91%, 92%, 93%, 94%, 95%, 96%, 97%, 98% or 99% identity to the nucleotide sequence set forth in one of SEQ ID NOs: 19-24.

15. The nucleic acid according to any one of claims 1-14, comprising a modified nucleotide;
preferably, the nucleic acid comprises a modified nucleoside;
preferably, the modified nucleoside comprises at least one of a modified uridine, a modified cytidine, a modified adenosine, and a modified guanosine.

16. A genetic engineering vector, comprising the nucleic acid according to any one of claims 1-15, or comprising a polynucleotide that can be transcribed into the nucleic acid according to any one of claims 1-15.

17. A host cell comprising the genetic engineering vector according to claim 16.

18. A protein or polypeptide encoded by the nucleic acid according to any one of claims 1-15.

19. A delivery carrier comprising the nucleic acid according to any one of claims 1-15, or the genetic engineering vector according to claim 16.

20. The delivery carrier according to claim 19, wherein the delivery carrier is a lipid nanoparticle (LNP), a cationic liposome, a cationic protein or a lipopolyplex (LPP);
preferably, the delivery carrier is a lipid nanoparticle comprising a cationic lipid comprising the following compound (IV), or a pharmaceutically acceptable salt or stereoisomer thereof: 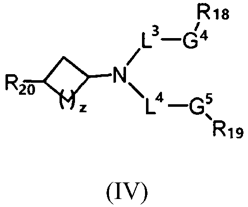 where:
L³ and L⁴ are the same or different, and are each independently C1-C12 alkylene, C2-C12 alkenylene or C2-C12 alkynylene; preferably L³ and L⁴ are the same or different, and are each independently C3-C10 alkylene, C3-C10 alkenylene or C3-C10 alkynylene; further preferably, L³ and L⁴ are the same or different, and are each independently C3-C10 alkylene; most preferably, L³ and L⁴ are the same or different, and are each independently C5-C8 alkylene;
G⁴ and G⁵ are the same or different, and are each independently -O-(C=O)-, -(C=O)-O-, -C(=O)-, -O-, -C(=O)-S- or -S-C(=O)-; preferably, G⁴ and G⁵ are the same or different, and are each independently -O-(C=O)-, -(C=O)-O-, -C(=O)- or -O-; most preferably, G⁴ and G⁵ are the same or different, and are each independently selected from -O-(C=O)- or -(C=O)-O-;
R₁₈ and R₁₉ are the same or different, and are each independently C5-C27 alkyl or C5-C27 alkenyl containing one or more double bonds; preferably, R₁₈ and R₁₉ are the same or different, and are each independently C8-C20 alkyl or C8-C20 alkenyl containing one or more double bonds; further preferably, R₁₈ and R₁₉ are the same or different, and are each independently C9-C17 alkyl or C9-C18 alkenyl containing one or two double bonds; most preferably, R₁₈ and R₁₉ are the same or different, and are each independently 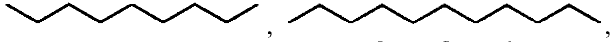 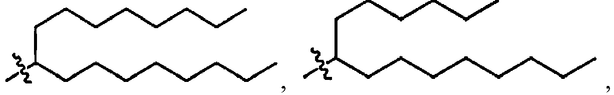 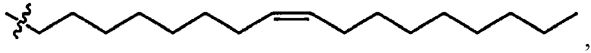
or 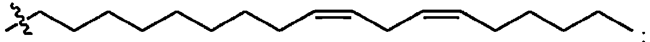
R₂₀ is halogen, hydroxyl, cyano, C1-C6 alkyl, nitro, C1-C6 alkoxy, C1-C6 alkylcarbonyloxy, C1-C6 alkoxycarbonyl, C1-C6 alkylaminocarbonyl or C1-C6 alkylcarbonylamino; preferably, R₂₀ is halogen, hydroxyl, cyano, C1-C6 alkoxy, C1-C6 alkylcarbonyloxy, C1-C6 alkoxycarbonyl, C1-C6 alkylaminocarbonyl or C1-C6 alkylcarbonylamino; further preferably, R₂₀ is halogen, hydroxyl, cyano, C1-C4 alkoxy, C1-C4 alkylcarbonyloxy, C1-C4 alkoxycarbonyl, C1-C4 alkylaminocarbonyl or C1-C4 alkylcarbonylamino; most preferably, R₂₀ is fluorine, hydroxyl, cyano, methoxy, acetoxy, methoxycarbonyl, butylaminocarbonyl or acetamido;
z is 1, 2 or 3.

21. The delivery carrier according to claim 20, wherein the cationic lipid is the following compound (IV-1), or a pharmaceutically acceptable salt or stereoisomer thereof: 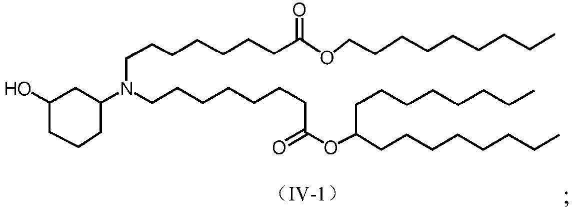 or the cationic lipid is the following compound, or a pharmaceutically acceptable salt thereof:

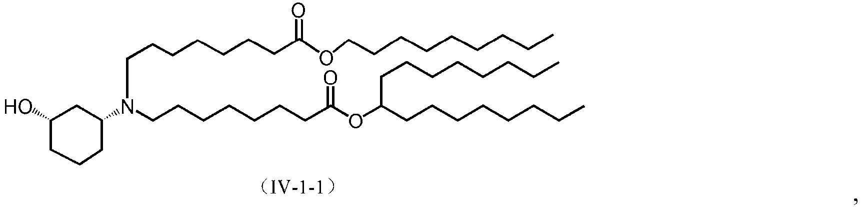

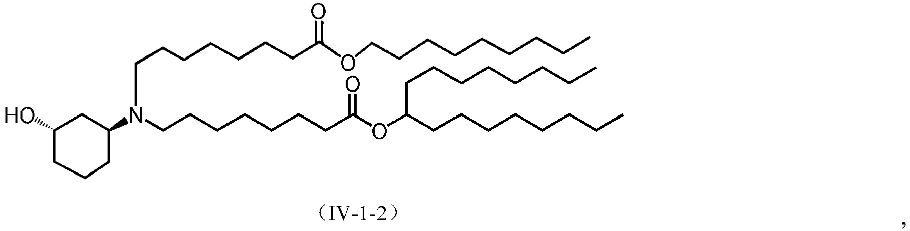

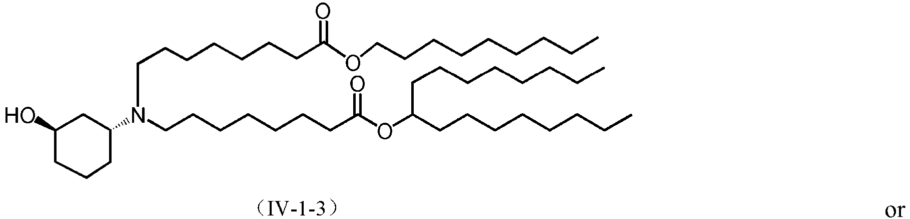

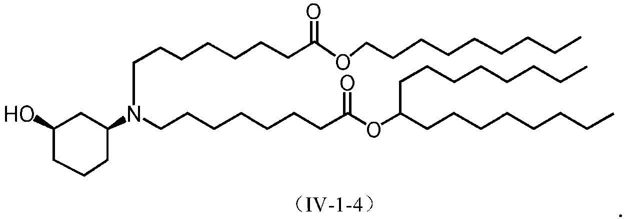

22. A pharmaceutical composition comprising the nucleic acid according to any one of claims 1-15, the genetic engineering vector according to claim 16, the host cell according to claim 17, the protein or polypeptide according to claim 18, or the delivery carrier according to any one of claims 19-21, and a pharmaceutically acceptable carrier.

23. Use of the nucleic acid according to any one of claims 1-15, the genetic engineering vector according to claim 16, the host cell according to claim 17, the protein or polypeptide according to claim 18, or the delivery carrier according to any one of claims 19-21 or the pharmaceutical composition according to claim 22 in the preparation of a medicament;
preferably, the medicament is used for preventing or treating the infection of VZV or a disease caused by the infection of VZV;
preferably, the medicament is used for preventing, treating or ameliorating neuralgia caused by VZV infection;
preferably, the medicament is used for preventing, treating or ameliorating post-herpes zoster neuralgia;
preferably, the medicament is a vaccine; preferably, the medicament is an mRNA vaccine;
preferably, the medicament is used for preventing or treating varicella or herpes zoster.

24. A vaccine comprising the nucleic acid of any one of claims 1-15, the genetic engineering vector of claim 16, the protein or polypeptide of claim 18, or the delivery carrier of any one of claims 19-21.
